# EUROPEAN PATENT APPLICATION

(11) **EP 4 796 122 A2**
(43) Date of publication of application: **26.08.2026**
(21) Application number: 26190850.3
(22) Date of filing: 10.11.2020
(51) Int. Cl.: A61K 39/00

(54) **METHODS OF DETECTING A FUSION GENE ENCODING A NEOANTIGEN**

(30) Priority: 12.11.2019 US 201962934445 P; 12.08.2020 US 202063064728 P
(62) Document number(s) of the earlier application(s) in accordance with Art. 76 EPC: 20886856.2 / 4 058 593
(71) Applicant: Foundation Medicine, Inc., Boston, MA 02210 (US)
(72) Inventor: JIN, Dexter X., Cambridge, 02141 (US); SOKOL, Ethan, Cambridge, 02141 (US); TRABUCCO, Sally, Cambridge, 02141 (US); PAVLICK, Dean, Cambridge, 02141 (US); ALI, Siraj, Cambridge, 02138 (US)
(74) Representative: CMS Cameron McKenna Nabarro Olswang LLP

(57) **Abstract**

Provided herein are methods for detecting a Ewing sarcoma breakpoint region 1 ( *EWSR1 )* fusion gene encoding a neoantigen (e.g., in a patient sample), as well as methods of prognosis and treatment related thereto, in some embodiments, the methods further comprise administering a cancer immunotherapy. In some embodiments, the *EWSR1* fusion gene is a fusion gene between *EWSR1 and WT1, FLI1, ERG, FEV, NR4A3, ATF1, CREB1, CREM, CREB3LI CREB3L2, PATZ1, NFATC2, KLF15, C11orf93, ZNF444, PBX1, DD1T3, or TFCP2* that encodes a neoantigen.

## Description

### CROSS REFERENCE TO RELATED APPLICATIONS

This application claims the priority benefit of U.S. Provisional Application Nos. 62/934,445, filed November 12, 2019, and 63/064,728, filed August 12, 2020, each of which is hereby incorporated by reference in its entirety.

### SUBMISSION OF SEQUENCE LISTING ON ASCII TEXT FILE

The content of the following submission on ASCII text file is incorporated herein by reference in its entirety: a computer readable form (CRF) of the Sequence Listing (file name: 197102000240SEQLIST.TXT, date recorded: November 3, 2020, size: 59 KB).

### FIELD

Provided herein are methods for detecting a Ewing sarcoma breakpoint region 1 (*EWSR1*) fusion gene encoding a neoantigen (*e.g*., in a patient sample), as well as methods of prognosis and treatment related thereto. In some embodiments, the methods further comprise administering a cancer immunotherapy. In some embodiments, the *EWSR1* fusion gene is a fusion gene between *EWSR1* and *WT1, FLI1, ERG, FEV, NR4A3, ATF1, CREB1, CREM, CREB3L1, CREB3L2, PATZ1, NFATC2, KLF15, C11orf93, ZNF444, PBX1, DDIT3,* or *TFCP2* that encodes a neoantigen.

### BACKGROUND

Chromosomal rearrangements resulting in gene fusions are a class of mutation found in many cancers. Starting with the discovery of the Philadelphia chromosome in various leukemias, a number of chromosomal translocations resulting in gene fusions have been described. *EWSR1-WT1* gene fusions are the result of a translocation between parts of chromosomes 11 and 22 (Sawyer, J.R. et al. (1992) Am. J. Surg. Pathol. 16:411-416). In particular, desmoplastic small round cell tumor (DSRCT) is a rare and aggressive class of sarcomas characterized by an *EWSR1-WT1* gene fusion. Treatment of patients with DSRCT is limited to chemo-radiotherapy and surgical resection due, in part, to a paucity of actionable genomic alterations (GAs). Despite this aggressive therapy, these pts have a 5-year survival rate of 15-25%.

Recently, Yang et al. (Nat. Med. 25:767-775 (2019)) have highlighted that gene fusions can be a source of immunogenic neoantigens and identified an exceptional responder to immune checkpoint inhibition (ICI), despite having a low tumor mutational burden (TMB). However, it is an outstanding question whether cancers bearing an *EWSR1-WT1* gene fusion are characterized by an actionable genomic alterations, such as immunogenic neoantigens.

Therefore, there remains a need for effective treatments for cancers characterized by *EWSR1-WT1* gene fusions *(e.g.,* DSRCT), as well as the identification of cancer patients that will respond to a particular therapeutic approach.

All references cited herein, including patent applications and publications, are incorporated by reference in their entirety.

### SUMMARY

To meet these and other needs, provided herein are methods for detecting a Ewing sarcoma breakpoint region 1 *(EWSR1)* fusion gene encoding a neoantigen *(e.g.,* in a patient sample), as well as methods of prognosis and treatment related thereto. In some embodiments, the *EWSR1* fusion gene is a fusion gene between *EWSR1* and *WT1, FLI1, ERG, FEV, NR4A3, ATF1, CREBI, CRIM, CREB3L1, CREB3L2, PATZ1, NFATC2, KLF15, C11orf93, ZNF444, PBX1, DDIT3,* or *TFCP2* that encodes a neoantigen.

In certain aspects, provided herein is a method of detecting a fusion gene encoding a neoantigen, the method comprising detecting presence of a Ewing sarcoma breakpoint region 1 - Wilms tumor protein (*EWSR1-WT1*) fusion gene encoding a neoantigen in a sample from an individual. In certain aspects, provided herein is a method of detecting a fusion gene encoding a neoantigen, the method comprising detecting presence of a Ewing sarcoma breakpoint region 1 - Wilms tumor protein (*EWSR1-WT1*) fusion gene encoding a neoantigen from tumor nucleic acid obtained from an individual.

In certain aspects, provided herein is a method of identifying an individual having a disorder who may benefit from a treatment comprising an immunotherapy, the method comprising detecting presence of a Ewing sarcoma breakpoint region 1 - Wilms tumor protein *(EWSR1-WT1)* fusion gene encoding a neoantigen in a sample from the individual, wherein the presence of an *EWSR1-WT1* fusion gene encoding a neoantigen in the sample identifies the individual as one who may benefit from a treatment comprising an immunotherapy. In certain aspects, provided herein is a method of identifying an individual having cancer who may benefit from a treatment comprising a cancer immunotherapy, the method comprising detecting presence of a Ewing sarcoma breakpoint region 1 - Wilms tumor protein *(EWSR1-WT1)* fusion gene encoding a neoantigen in a sample from the individual, wherein the presence of an *EWSR1-WT1* fusion gene encoding a neoantigen in the sample identifies the individual as one who may benefit from a treatment comprising a cancer immunotherapy.

In certain aspects, provided herein is a method of selecting a therapy for an individual having a disorder, the method comprising detecting presence of a Ewing sarcoma breakpoint region 1 - Wilms tumor protein (*EWSR1-WT1*) fusion gene encoding a neoantigen in a sample from the individual, wherein the presence of an *EWSR1-WT1* fusion gene encoding a neoantigen in the sample identifies the individual as one who may benefit from a treatment comprising an immunotherapy. In certain aspects, provided herein is a method of selecting a therapy for an individual having cancer, the method comprising detecting presence of a Ewing sarcoma breakpoint region 1 - Wilms tumor protein (*EWSR1-WT1*) fusion gene encoding a neoantigen in a sample from the individual, wherein the presence of an *EWSR1-WT1* fusion gene encoding a neoantigen in the sample identifies the individual as one who may benefit from a treatment comprising a cancer immunotherapy.

In certain aspects, provided herein is a method of identifying one or more treatment options for an individual having cancer, the method comprising: (a) detecting presence of a Ewing sarcoma breakpoint region 1 - Wilms tumor protein (*EWSR1-WT1*) fusion gene encoding a neoantigen in a sample from the individual; and (b) generating a report comprising one or more treatment options identified for the individual based at least in part on the presence of the *EWSR1-WT1* fusion gene encoding a neoantigen in the sample, wherein the one or more treatment options comprise a treatment comprising a cancer immunotherapy. In certain aspects, provided herein is a method of identifying one or more treatment options for an individual having cancer, the method comprising: (a) acquiring knowledge of a Ewing sarcoma breakpoint region 1 - Wilms tumor protein *(EWSR1-WT1)* fusion gene encoding a neoantigen in a sample from the individual; and (b) generating a report comprising one or more treatment options identified for the individual based at least in part on said knowledge, wherein the one or more treatment options comprise a treatment comprising a cancer immunotherapy. In some embodiments, the report further comprises a score that associates the one or more treatment options with a predicted outcome and/or response.

In certain aspects, provided herein is a method of selecting treatment for an individual having cancer, comprising acquiring knowledge of a Ewing sarcoma breakpoint region 1 - Wilms tumor protein *(EWSR1-WT1)* fusion gene encoding a neoantigen in a sample from the individual (*e.g.,* by sequencing, such as next-generation sequencing or sequencing by mass spectrometry), wherein responsive to the acquisition of said knowledge: (i) the individual is classified as a candidate to receive a treatment comprising a cancer immunotherapy, and/or (ii) the individual is identified as likely to respond to a treatment that comprises a cancer immunotherapy. In some embodiments, the method optionally further comprises providing a report to another party, wherein said report comprises: information on the role of the *EWSR1-WT1* fusion gene encoding a neoantigen, or wildtype sequence, in disease; information on prognosis, resistance, or potential or suggested therapeutic options for the individual; information on the likely effectiveness of a therapeutic option, the acceptability of a therapeutic option, or the advisability of applying a therapeutic option to the individual; or information, or a recommendation on, the administration of a drug to the individual.

In certain aspects, provided herein is a method of treating or delaying progression of a disorder, comprising administering to the individual an effective amount of a treatment comprising an immunotherapy, wherein the a sample obtained from the individual comprises a Ewing sarcoma breakpoint region 1 - Wilms tumor protein (*EWSR1-WT1*) fusion gene encoding a neoantigen. In certain aspects, provided herein is a method of treating or delaying progression of cancer, comprising, responsive to knowledge of a Ewing sarcoma breakpoint region 1 - Wilms tumor protein (*EWSR1-WT1*) fusion gene encoding a neoantigen in a sample from an individual, administering to the individual an effective amount of a treatment comprising a cancer immunotherapy. In certain aspects, provided herein is a method of treating or delaying progression of cancer, comprising administering to the individual an effective amount of a treatment comprising a cancer immunotherapy, wherein the cancer comprises a Ewing sarcoma breakpoint region 1 - Wilms tumor protein (*EWSR1-WT1*) fusion gene encoding a neoantigen. In some embodiments, an *EWSR1-WT1* fusion gene encoding a neoantigen has been detected in a sample from the individual prior to administration of the treatment. In some embodiments, an *EWSR1-WT1* fusion gene encoding a neoantigen has been detected in a sample from the cancer of the individual prior to administration of the treatment. In some embodiments, the method further comprises detecting an *EWSR1-WT1* fusion gene encoding a neoantigen in a sample from the individual (*e.g*., prior to treatment). In some embodiments, the method further comprises detecting an *EWSR1-WT1* fusion gene encoding a neoantigen in a sample from the cancer of the individual (*e.g*., prior to treatment).

In some embodiments according to any of the embodiments described herein, the methods further comprise *(e.g.,* prior to detecting the presence of an *EWSR1-WT1* fusion gene encoding a neoantigen in the sample) obtaining the sample from the individual. In some embodiments, the sample is a blood, bone marrow, tumor, or tissue sample. In some embodiments, the sample is from amniotic fluid, blood, plasma, serum, semen, lymphatic fluid, cerebral spinal fluid, ocular fluid, urine, saliva, stool, mucus, sweat, blood, skin, hair, hair follicles, saliva, oral mucous, vaginal mucus, sweat, tears, epithelial tissues, urine, semen, seminal fluid, seminal plasma, prostatic fluid, Cowper's fluid, excreta, biopsy, ascites, cerebrospinal fluid, or lymph. In some embodiments, the sample is a biopsy or formalin-fixed paraffin-embedded (FFPE) sample. In some embodiments, the presence of an *EWSR1-WT1* fusion gene is detected in DNA or RNA from the sample. In some embodiments, the presence of an *EWSR1-WT1* fusion gene is detected in cell-free DNA (cfDNA) or cell-free RNA (cfRNA). In some embodiments, the presence of an *EWSR1-WT1* fusion gene is detected in DNA by next-generation sequencing (NGS). In some embodiments, the presence of an *EWSR1-WT1* fusion gene is detected in DNA by polymerase chain reaction (PCR), Sanger sequencing, or fluorescence *in situ* hybridization (FISH). In some embodiments, the presence of an *EWSR1-WT1* fusion gene is detected in RNA by RNA-sequencing (RNA-seq). In some embodiments, the presence of an *EWSR1-W1* fusion gene is detected in RNA by polymerase chain reaction (PCR), Sanger sequencing, or fluorescence *in situ* hybridization (FISH).

In some embodiments according to any of the embodiments described herein, the neoantigen binds, or is predicted to bind, a major histocompatibility complex (MHC) class 1 molecule of the individual. In some embodiments, the neoantigen binds, or is predicted to bind, an MHC class I molecule of the individual with a dissociation constant (K_{D}) of 50 nM or less. In some embodiments, the method further comprises genotyping one or more human leukocyte antigen (HLA)-A, HLA-B, and HLA-C alleles from the sample from the individual. In some embodiments, the cancer does not comprise a loss-of-heterozygosity at an HLA-A, HLA-B, or HLA-C allele that encodes an MHC class 1 molecule to which the neoantigen binds, or is predicted to bind. In some embodiments, the cancer comprises a loss-of-heterozygosity at one or more, but not all, of HLA-A, HLA-B, or HLA-C allele(s) that encode an MHC class 1 molecule to which the neoantigen binds, or is predicted to bind. In some embodiments, the cancer does not comprise a loss-of-function mutation in a *CIITA* or *B2M* gene. In some embodiments, the method further comprises genotyping a *CIITA* and/or *B2M* gene from the sample from the individual. In some embodiments, the method further comprises determining a tumor mutational burden (TMB) from the sample from the individual. In some embodiments, the cancer is not characterized by a high tumor mutational burden (TMB). In some embodiments, the cancer is characterized by a high tumor mutational burden (TMB). In some embodiments, the cancer is not characterized by microsatellite instability (MSI). In some embodiments, the cancer is characterized by microsatellite instability (MSI). In some embodiments, the method further comprises determining microsatellite instability (MSI) from the sample from the individual. In some embodiments, the cancer is a desmoplastic small round cell tumor (DSRCT). In some embodiments, the DSRCT is characterized by a low TMB. In some embodiments, the DSRCT has a TMB of about 20 mutations/Mb or less. In some embodiments, the DSRCT has a TMB of about 10 mutations/Mb or less. In some embodiments, the DSRCT is not characterized by microsatellite instability (MSI). In some embodiments, the cancer is a sarcoma. In some embodiments, the sarcoma is selected from the group consisting of synovial sarcomas, ewing sarcomas, clear cell sarcomas, solitary fibrous tumors, myxoid liposarcomas, mesenchymal chondrosarcomas, uterine endometrial stromal sarcomas, undifferentiated round cell/ewing-like sarcomas, alveolar soft part sarcomas, alveolar rhabdomyosarcomas, inflammatory myofibroblastic tumors, extraskeletal myxoid chondrosarcomas, epithelioid hemangioendotheliomas, low grade fibromyxoid sarcomas, sclerosing epithelioid fibrosarcomas, dermatofibrosarcoma protuberans, and ossifying fibromyxoid tumors. In some embodiments, the cancer is a carcinoma. In some embodiments, the cancer is a NUT midline carcinoma. In some embodiments, the cancer is selected from the group consisting of lung cancer, kidney cancer, a bladder cancer, breast cancer, colorectal cancer, ovarian cancer, pancreatic cancer, gastric carcinoma, esophageal cancer, mesothelioma, melanoma, head and neck cancer, thyroid cancer, , prostate cancer, glioblastoma, cervical cancer, thymic carcinoma, leukemia, lymphoma, myeloma, mycoses fungoides, merkel cell cancer, hematologic malignancy, cancer of hematological tissues, B cell cancer, bronchus cancer, stomach cancer, brain or central nervous system cancer, peripheral nervous system cancer, uterine or endometrial cancer, cancer of the oral cavity or pharynx, liver cancer, testicular cancer, biliary tract cancer, small bowel or appendix cancer, salivary gland cancer, adrenal gland cancer, adenocarcinoma, inflammatory myofibroblastic tumor, gastrointestinal stromal tumor (GIST), colon cancer, myelodysplastic syndrome (MDS), myeloproliferative disorder (MPD), polycythemia Vera, chordoma, synovioma. Ewing's tumor, squamous cell carcinoma, basal cell carcinoma, adenocarcinoma, sweat gland carcinoma, sebaceous gland carcinoma, papillary carcinoma, papillary adenocarcinoma, medullary carcinoma, bronchogenic carcinoma, renal cell carcinoma, hepatoma, bile duct carcinoma, choriocarcinoma, seminoma, embryonal carcinoma, Wilms' tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, follicular lymphoma, diffuse large B-cell lymphoma, mantle cell lymphoma, hepatocellular carcinoma, thyroid cancer, small cell cancer, essential thrombocythemia, agnogenic myeloid metaplasia, hypereosinophilic syndrome, systemic mastocytosis, familiar hypereosinophilia, neuroendocrine cancer, and carcinoid tumor

In some embodiments according to any of the embodiments described herein, the *EWSR1-WT1* fusion gene comprises a coding sequence of *EWSR1* at the 5' end and a coding sequence of *WT1* at the 3' end. In some embodiments, the *EWSR1-WT1* fusion gene is formed via a breakpoint in intron 7, intron 8, intron 9, or exon 7 of *EWSR1.* In some embodiments, the *EWSR1-WT1* fusion gene is formed via breakpoints in intron 7 of *EWSR1* and intron 7 of *WT1.* In some embodiments, the *EWSR1-WT1* fusion gene encodes exons 1-7 of *EWSR1* and exons 8-10 of *WT1.* In some embodiments, the *EWSR1-WT1* fusion gene encodes exons 1-7 of *EWSR1* and exons 7-9 of *WT1.* In some embodiments, the neoantigen comprises at least 5 contiguous amino acids from the sequence SSYGQQSEK (SEQ ID NO:1). In some embodiments, the neoantigen comprises 6, 7, 8, or 9 contiguous amino acids from the sequence SSYGQQSEK (SEQ ID NO: 1). In some embodiments, the neoantigen comprises 7, 8, or 9 contiguous amino acids from the sequence SSYGQQSEK (SEQ ID NO: 1). In some embodiments, the neoantigen comprises 8 or 9 contiguous amino acids from the sequence SSYGQQSEK (SEQ ID NO:1). In some embodiments, the neoantigen comprises the sequence SSYGQQSEK (SEQ ID NO: 1). In some embodiments, the neoantigen comprises at least 5 contiguous amino acids from the sequence YGQQSEKPY (SEQ ID NO:2). In some embodiments, the neoantigen comprises 6, 7, 8, or 9 contiguous amino acids from the sequence YGQQSEKPY (SEQ ID NO:2). In some embodiments, the neoantigen comprises 7, 8, or 9 contiguous amino acids from the sequence YGQQSEKPY (SEQ ID NO:2). In some embodiments, the neoantigen comprises 8 or 9 contiguous amino acids from the sequence YGQQSEKPY (SEQ ID NO:2). In some embodiments, the neoantigen comprises the sequence YGQQSEKPY (SEQ ID NO:2). In some embodiments, the *EWSR1-WT1* fusion gene encodes a sequence that has at least 80%, at least 85%, at least 90%, at least 91%, at least 92%, at least 93%, at least 94%, at least 95%, at least 96%, at least 97%, at least 98%, or at least 99% identity to the sequence 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:4),
 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:5),
 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGEKPFSCRWPSCQKKFARSDELVRH
HNMHQRNMTKLQLAL (SEQ ID NO:6),
 
MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGEKPFSCRWPSCQKKFARSDEL
VRHHNMHQRNMTKLQLAL (SEQ ID NO:7),
 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:8),
 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGKTSEKPFSCRWPSCQKKFARSDELV
RHHNMHQRNMTKLQLAL (SEQ ID NO:9),
 
MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGKTSEKPFSCRWPSCQKKFARS
DELVRHHNMHQRNMTKLQLAL (SEQ ID NO:10), and/or
 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
 
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:11). In some embodiments, the EWSR1-WT1 fusion gene
 encodes the sequence SSSYGQQSEKPYQCDF (SEQ ID NO:3),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:4),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:5),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGEKPFSCRWPSCQKKFARSDELVRH
HNMHQRNMTKLQLAL (SEQ ID NO:6),

MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGEKPFSCRWPSCQKKFARSDEL
VRHHNMHQRNMTKLQLAL (SEQ ID NO:7),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:8),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGKTSEKPFSCRWPSCQKKFARSDELV
RHHNMHQRNMTKLQLAL (SEQ ID NO:9),

MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGKTSEKPFSCRWPSCQKKFARS
DELVRHHNMHQRNMTKLQLAL (SEQ ID NO:10), and/or

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:11).

In some embodiments according to any of the embodiments described herein, the method further comprises detecting one or more additional mutations in the cancer or sample. In some embodiments, the one or more additional mutations are in one or more genes other than *EWSR1* and *WT1.* In some embodiments, the one or more additional mutations are in one or more genes selected from the group consisting of ABL1, BRAF, CDKN1A, EPHA3, FGFR4, IKZF1, MCL1, NKX2-1, PMS2, RNF43, TET2, ACVR1B, BRCA1, CDKN1B, EPHB1, FH, INPP4B, MDM2, NOTCH1, POLD1, ROS1, TGFBR2, AKT1, BRCA2, CDKN2A, EPHB4, FLCN, IRF2, MDM4, NOTCH2, POLE, RPTOR, TIPARP, AKT2, BRD4, CDKN2B, ERBB2, FLT1, IRF4, MED12, NOTCH3, PPARG, SDHA, TNFAIP3, AKT3, BRIP1, CDKN2C, ERBB3, FLT3, IRS2, MEF2B, NPM1, PPP2R1A, SDHB, TNFRSF14, ALK, BTG1, CEBPA, ERBB4, FOXL2, JAK1, MEN1, NRAS, PPP2R2A, SDHC, TP53, ALOX12B, BTG2, CHEK1, ERCC4, FLBP1, JAK2, MERTK, NT5C2, PRDM1, SDHD, TSC1, AMER1, BTK, CHEK2, ERG, GABRA6, JAK3, MET, NTRK1, PRKAR1A, SETD2, TSC2, APC, C11orf30, CIC, ERRFI1, GATA3, JUN, MITF, NTRK2, PRKCI, SF3B1, TYRO3, AR, CALR, CREBBP, ESR1, GATA4, KDM5A, MKNK1, NTRK3, PTCH1, SGK1, U2AF1, ARAF, CARD11, CRKL, EZH2, GATA6, KDM5C, MLH1, P2RY8, PTEN, SMAD2, VEGFA, ARFRP1, CASP8, CSF1R, FAM46C, GID4, (C17orf39), KDM6A, MPL, PALB2, PTPN11, SMAD4, VHL, ARID1A, CBFB, CSF3R, FANCA, GNA11, KDR, MRE11A, PARK2, PTPRO, SMARCA4, WHSC1, ASXL1, CBL, CTCF, FANCC, GNA13, KEAP1, MSH2, PARP1, QKI, SMARCBI, WHSC1L1, ATM, CCND1, CTNNA1, FANCG, GNAQ, KEL, MSH3, PARP2, RAC1, SMO, WT1, ATR, CCND2, CTNNB1, FANCL, GNAS, KIT, MSH6, PARP3, RAD21, SNCAIP, XPO1, ATRX, CCND3, CUL3, FAS, GRM3, KLHL6, MST1R, PAX5, RAD51, SOCS1, XRCC2, AURKA, CCNE1, CUL4A, FBXW7, GSK3B, KMT2A, (MLL), MTAP, PBRM1, RAD51B, SOX2, ZNF217, AURKB, CD22, CXCR4, FGF10, H3F3A, KMT2D, (MLL2), MTOR, PDCD1, RAD51C, SOX9, ZNF703, AXIN1, CD274, CYP17A1, FGF12, HDAC1, KRAS, MUTYH, PDCD1LG2, RAD51D, SPEN, AXL, CD70, DAXX, FGF14, HGF, LTK, MYC, PDGFRA, RAD52, SPOP, BAP1, CD79A, DDR1, FGF19, HNF1A, LYN, MYCL, PDGFRB, RAD54L, SRC, BARD1, CD79B, DDR2, FGF23, HRAS, MAF, MYCN, PDK1, RAF1, STAG2, BCL2, CDC73, DIS3, FGF3, HSD3B1, MAP2K1, MYD88, PIK3C2B, RARA, STAT3, BCL2L1, CDH1, DNMT3A, FGF4, ID3, MAP2K2, NBN, PIK3C2G, RB1, STK11, BCL2L2, CDK12, DOT1L, FGF6, IDH1, MAP2K4, NF1, PIK3CA, RBM10, SUFU, BCL6, CDK4, EED, FGFR1, IDH2, MAP3K1, NF2, PIK3CB, REL, SYK, BCOR, CDK6, EGFR, FGFR2, IGF1R, MAP3K13, NFE2L2, PIK3R1, RET, TBX3, BCORL1, CDK8, EP300, FGFR3, IKBKE, MAPK1, NFKBIA, PIM1, RICTOR, TEK, BCR, CD74, ETV4, ETV5, ETV6, EWSR1, EZR, MYB, NUTM1, RSPO2, SDC4, SLC34A2, TERC, TERT, and TMPRSS2.

In some embodiments according to any of the embodiments described herein, the cancer immunotherapy comprises one or more of: a checkpoint inhibitor, cancer vaccine, cell-based therapy, T cell receptor (TCR)-based therapy, adjuvant immunotherapy, cytokine immunotherapy, and oncolytic virus therapy. In some embodiments, the cancer immunotherapy comprises small molecule, nucleic acid, polypeptide, carbohydrate, toxin, cell-based, or binding agent therapeutic agent. In some embodiments, the cancer immunotherapy comprises a checkpoint inhibitor. In some embodiments, the checkpoint inhibitor targets PD-L1, PD-1, CTLA-4, CEACAM, LAIR1, CD160, 2B4, CD80, CD86, CD276, VTCN1, HVEM, KIR, A2AR, MHC class I, MHC class II, GALS, adenosine, TGFR, OX40, CD137, CD40, IDO, CSF1R, TIM-3, BTLA, VISTA, LAG-3, TIGIT, IDO, MICA/B, and/or arginase. In some embodiments, the checkpoint inhibitor is an agent that inhibits PD-1. In some embodiments, the agent that inhibits PD-1 is a small molecule, a nucleic acid, a polypeptide, carbohydrate, a lipid, a metal, or a toxin. In some embodiments, the agent that inhibits PD-1 is a PD-1 binding antagonist. In some embodiments, the PD-1 binding antagonist is an antibody, antibody-drug conjugate, antibody fragment, or immunoadhesin. In some embodiments, the PD-1 binding antagonist is selected from the group consisting of pembrolizumab, nivolumab, cemiplimab, spartalizumab, genolimzumab, SHR1210, JS001, BGB-108, BGB-A317, IBI308, GLS-010, BMS-936558, BCD-100, REGN2810, MGA-012, BI 754091, STI-A1110, INCSHR-1210, PF-06801591, TSR-042, AM0001, JNJ-63723283, and ENUM 244C8. In some embodiments, the checkpoint inhibitor is an agent that inhibits PD-L1 and/or PD-L2. In some embodiments, the agent that inhibits PD-L1 and/or PD-L2 is a small molecule, a nucleic acid, a polypeptide, carbohydrate, a lipid, a metal, or a toxin. In some embodiments, the agent that inhibits PD-L1 is a PD-L1 binding antagonist. In some embodiments, the PD-L1 binding antagonist is an antibody, antibody-drug conjugate, antibody fragment, or immunoadhesin. In some embodiments, the PD-L1 binding antagonist is selected from the group consisting of atezolizumab, avelumab, durvalumab, KN035, CS1001, MDX-1105, LY3300054, STI-A1014, FAZ053, and CX-072. In some embodiments, the checkpoint inhibitor is an agent that inhibits CTLA4. In some embodiments, the agent that inhibits CTLA4 is a small molecule, a nucleic acid, a polypeptide, carbohydrate, a lipid, a metal, or a toxin. In some embodiments, the agent that inhibits CTLA4 is an antibody, antibody-drug conjugate, antibody fragment, or immunoadhesin. In some embodiments, the agent that inhibits CTLA4 is selected from the group consisting of ipilimumab, APL-509, AGEN1884, and CS1002. In some embodiments, the methods further comprise administering an additional anti-cancer therapy to the individual. In some embodiments, the additional anti-cancer therapy comprises one or more of surgery, radiotherapy, chemotherapy, anti-angiogenic therapy, anti-DNA repair therapy, and antiinflammatory therapy. In some embodiments, the additional anti-cancer therapy comprises a chemotherapeutic agent. In some embodiments, the chemotherapeutic agent is a platinum-based chemotherapeutic agent. In some embodiments, the methods further comprise administering an additional cancer immunotherapy to the individual.

In some embodiments according to any of the embodiments described herein, the *EWSR1-WT1* fusion gene encoding the neoantigen is detected using one or more oligonucleotides. In some embodiments, the *EWSR1-WT1* fusion gene encoding the neoantigen is detected by PCR amplification of a DNA or RNA sequence encoding the amino acid sequence of any one of SEQ ID Nos:1-11. In some embodiments, the *EWSR1-WT1* fusion gene encoding the neoantigen is detected by sequencing a DNA or RNA sequence encoding the amino acid sequence of any one of SEQ ID Nos:1-11. In some embodiments, the *EWSR1-WT1* fusion gene encoding the neoantigen is detected by *in situ* hybridization of a DNA or RNA oligonucleotide with a sequence encoding the amino acid sequence of any one of SEQ ID Nos: 1-11. In some embodiments, the methods further comprise administering a cancer immunotherapy to the individual.

Further provided herein are kits or articles of manufacture comprising one or more oligonucleotides, wherein the one or more oligonucleotides hybridize with at least a portion of a DNA or RNA sequence encoding the amino acid sequence of any one of SEQ ID Nos:1-11.

Further provided herein is the use of a cancer immunotherapy in a method of treating or delaying progression of cancer according to any one of the above embodiments. Further provided herein is the use of a cancer immunotherapy for the manufacture of a medicament for treating or delaying progression of cancer according to any one of the above embodiments, e.g., wherein the medicament is to be administered to an individual, and wherein a Ewing sarcoma breakpoint region 1 - Wilms tumor protein (*EWSR1-WT1*) fusion gene encoding a neoantigen has been detected in a sample obtained from the individual.

Further provided herein are isolated polypeptides comprising an amino acid sequence that is at least 80% identical, at least 85% identical, at least 90% identical, at least 91% identical, at least 92% identical, at least 93% identical, at least 94% identical, at least 95% identical, at least 96% identical, at least 97% identical, at least 98% identical, at least 99% identical, or 100% identical to a sequence selected from the group consisting of SEQ ID Nos:1-11. Also provided herein are isolated polynucleotides encoding an amino acid sequence that is at least 80% identical, at least 85% identical, at least 90% identical, at least 91% identical, at least 92% identical, at least 93% identical, at least 94% identical, at least 95% identical, at least 96% identical, at least 97% identical, at least 98% identical, at least 99% identical, or 100% identical to a sequence selected from the group consisting of SEQ ID Nos:1-11. Also provided herein are vectors (e.g., expression vectors) comprising a polynucleotide according to any one of the above embodiments. Also provided herein are compositions comprising the isolated polypeptide, polynucleotide, or vector according to any one of the above embodiments.

In certain aspects, provided herein is a method of detecting a fusion gene encoding a neoantigen, the method comprising detecting presence of a Ewing sarcoma breakpoint region 1 (*EWSR1*) fusion gene encoding a neoantigen in a sample from an individual. In certain aspects, provided herein is a method of detecting a fusion gene encoding a neoantigen, the method comprising detecting presence of a Ewing sarcoma breakpoint region 1 (*EWSR1*) fusion gene encoding a neoantigen from tumor nucleic acid obtained from an individual.

In certain aspects, provided herein is a method of identifying an individual having a disorder who may benefit from a treatment comprising an immunotherapy, the method comprising detecting presence of a Ewing sarcoma breakpoint region 1 *(EWSR1)* fusion gene encoding a neoantigen in a sample from the individual, wherein the presence of an *EWSR1* fusion gene encoding a neoantigen in the sample identifies the individual as one who may benefit from a treatment comprising an immunotherapy. In certain aspects, provided herein is a method of identifying an individual having cancer who may benefit from a treatment comprising a cancer immunotherapy, the method comprising detecting presence of a Ewing sarcoma breakpoint region 1 (*EWSR1*) fusion gene encoding a neoantigen in a sample from the individual, wherein the presence of an *EWSR1* fusion gene encoding a neoantigen in the sample identifies the individual as one who may benefit from a treatment comprising a cancer immunotherapy.

In certain aspects, provided herein is a method of selecting a therapy for an individual having a disorder, the method comprising detecting presence of a Ewing sarcoma breakpoint region 1 (*EWSR1*) fusion gene encoding a neoantigen in a sample from the individual, wherein the presence of an *EWSR1* fusion gene encoding a neoantigen in the sample identifies the individual as one who may benefit from a treatment comprising an immunotherapy. In certain aspects, provided herein is a method of selecting a therapy for an individual having cancer, the method comprising detecting presence of a Ewing sarcoma breakpoint region 1 (*EWSR1*) fusion gene encoding a neoantigen in a sample from the individual, wherein the presence of an *EWSR1* fusion gene encoding a neoantigen in the sample identifies the individual as one who may benefit from a treatment comprising a cancer immunotherapy.

In certain aspects, provided herein is a method of identifying one or more treatment options for an individual having cancer, the method comprising: (a) detecting presence of a Ewing sarcoma breakpoint region 1 *(EWSR1)* fusion gene encoding a neoantigen in a sample from the individual; and (b) generating a report comprising one or more treatment options identified for the individual based at least in part on the presence of the *EWSR1* fusion gene encoding a neoantigen in the sample, wherein the one or more treatment options comprise a treatment comprising a cancer immunotherapy. In certain aspects, provided herein is a method of identifying one or more treatment options for an individual having cancer, the method comprising: (a) acquiring knowledge of a Ewing sarcoma breakpoint region 1 *(EWSR1)* fusion gene encoding a neoantigen in a sample from the individual; and (b) generating a report comprising one or more treatment options identified for the individual based at least in part on said knowledge, wherein the one or more treatment options comprise a treatment comprising a cancer immunotherapy. In some embodiments, the report further comprises a score that associates the one or more treatment options with a predicted outcome and/or response.

In certain aspects, provided herein is a method of selecting treatment for an individual having cancer, comprising acquiring knowledge of a Ewing sarcoma breakpoint region 1 (*EWSR1*) fusion gene encoding a neoantigen in a sample from the individual *(e.g.,* by sequencing, such as next-generation sequencing or sequencing by mass spectrometry), wherein responsive to the acquisition of said knowledge: (i) the individual is classified as a candidate to receive a treatment comprising a cancer immunotherapy; and/or (ii) the individual is identified as likely to respond to a treatment that comprises a cancer immunotherapy. In some embodiments, the method optionally further comprises providing a report to another party, wherein said report comprises: information on the role of the *EWSR1* fusion gene encoding a neoantigen, or wildtype sequence, in disease; information on prognosis, resistance, or potential or suggested therapeutic options for the individual; information on the likely effectiveness of a therapeutic option, the acceptability of a therapeutic option, or the advisability of applying a therapeutic option to the individual; or information, or a recommendation on, the administration of a drug to the individual.

In certain aspects, provided herein is a method of treating or delaying progression of a disorder, comprising administering to the individual an effective amount of a treatment comprising an immunotherapy, wherein the a sample obtained from the individual comprises a Ewing sarcoma breakpoint region 1 (*EWSR1*) fusion gene encoding a neoantigen. In certain aspects, provided herein is a method of treating or delaying progression of cancer, comprising, responsive to knowledge of a Ewing sarcoma breakpoint region 1 *(EWSR1)* fusion gene encoding a neoantigen in a sample from an individual, administering to the individual an effective amount of a treatment comprising a cancer immunotherapy. In certain aspects, provided herein is a method of treating or delaying progression of cancer, comprising administering to the individual an effective amount of a treatment comprising a cancer immunotherapy, wherein the cancer comprises a Ewing sarcoma breakpoint region 1 (*EWSR1*) fusion gene encoding a neoantigen. In some embodiments, an *EWSR1* fusion gene encoding a neoantigen has been detected in a sample from the individual prior to administration of the treatment. In some embodiments, an *EWSR1* fusion gene encoding a neoantigen has been detected in a sample from the cancer of the individual prior to administration of the treatment. In some embodiments, the method further comprises detecting an *EWSR1* fusion gene encoding a neoantigen in a sample from the individual (*e.g*., prior to treatment). In some embodiments, the method further comprises detecting an *EWSR1* fusion gene encoding a neoantigen in a sample from the cancer of the individual (e.g., prior to treatment).

In certain aspects, provided herein is a method of treating or delaying progression of cancer, comprising: (a) detecting a Ewing sarcoma breakpoint region 1 (*EWSR1*) fusion gene encoding a neoantigen in a sample from an individual; and (b) administering to the individual an effective amount of a treatment comprising a cancer immunotherapy. In certain aspects, provided herein is a method of treating or delaying progression of cancer, comprising: (a) acquiring knowledge of a Ewing sarcoma breakpoint region 1 (*EWSR1*) fusion gene encoding a neoantigen in a sample from an individual; and (b) responsive to said knowledge, administering to the individual an effective amount of a treatment comprising a cancer immunotherapy.

In some embodiments according to any of the embodiments described herein, the *EWSR1* fusion gene is a fusion gene between *EWSR1* and one of: *FLI1, ERG, FEV, NR4A3, ATF1, CREB1, CREM, CREB3L1, CREB3L2, PATZ1, NFATC2, KLF15, C11orf93, ZNF444, PBX1, DDIT3,* and *TFCP2.* In some embodiments, the methods further comprise *(e.g.,* prior to detecting the presence of an *EWSR1* fusion gene encoding a neoantigen in the sample) obtaining the sample from the individual. In some embodiments, the sample is a blood, bone marrow, tumor, or tissue sample. In some embodiments, the sample is from amniotic fluid, blood, plasma, serum, semen, lymphatic fluid, cerebral spinal fluid, ocular fluid, urine, saliva, stool, mucus, sweat, blood, skin, hair, hair follicles, saliva, oral mucous, vaginal mucus, sweat, tears, epithelial tissues, urine, semen, seminal fluid, seminal plasma, prostatic fluid, Cowper's fluid, excreta, biopsy, ascites, cerebrospinal fluid, or lymph. In some embodiments, the sample is a biopsy or formalin-fixed paraffin-embedded (FFPE) sample. In some embodiments, the presence of an *EWSR1* fusion gene is detected in DNA or RNA from the sample. In some embodiments, the presence of an *EWSR1* fusion gene is detected in cell-free DNA (cfDNA) or cell-free RNA (cfRNA). In some embodiments, the presence of an *EWSR1* fusion gene is detected in DNA by next-generation sequencing (NGS). In some embodiments, the presence of an *EWSR1* fusion gene is detected in DNA by polymerase chain reaction (PCR), Sanger sequencing, or fluorescence *in situ* hybridization (FISH). In some embodiments, the presence of an *EWSR1* fusion gene is detected in RNA by RNA-sequencing (RNA-seq). In some embodiments, the presence of an *EWSR1* fusion gene is detected in RNA by polymerase chain reaction (PCR), Sanger sequencing, or fluorescence *in situ* hybridization (FISH).

In some embodiments according to any of the embodiments described herein, the cancer is Ewing sarcoma, extraskeletal myxoid chondrosarcoma, clear cell sarcoma, low-grade fibromyxoid sarcoma/sclerosing epithelioid fibrosarcoma, undifferentiated round cell sarcoma/Ewing-like, sarcoma not otherwise specified (NOS), rare sarcoma, myxoid liposarcoma, or rhabdomyosarcoma NOS.

In some embodiments according to any of the embodiments described herein, the neoantigen binds, or is predicted to bind, a major histocompatibility complex (MHC) class I molecule of the individual. In some embodiments, the neoantigen binds, or is predicted to bind, an MHC class 1 molecule of the individual with a dissociation constant (K_{D}) of 50 nM or less. In some embodiments, the method further comprises genotyping one or more human leukocyte antigen (HLA)-A, HLA-B, and HLA-C alleles from the sample from the individual. In some embodiments, the cancer does not comprise a loss-of-heterozygosity at an HLA-A, HLA-B, or HLA-C allele that encodes an MHC class I molecule to which the neoantigen binds, or is predicted to bind. In some embodiments, the cancer comprises a loss-of-heterozygosity at one or more, but not all, of HLA-A, HLA-B, or HLA-C allele(s) that encode an MHC class I molecule to which the neoantigen binds, or is predicted to bind.

In some embodiments according to any of the embodiments described herein, the cancer immunotherapy comprises one or more of: a checkpoint inhibitor, cancer vaccine, cell-based therapy, T cell receptor (TCR)-based therapy, adjuvant immunotherapy, cytokine immunotherapy, and oncolytic virus therapy. In some embodiments, the cancer immunotherapy comprises small molecule, nucleic acid, polypeptide, carbohydrate, toxin, cell-based, or binding agent therapeutic agent. In some embodiments, the cancer immunotherapy comprises a checkpoint inhibitor. In some embodiments, the checkpoint inhibitor targets PD-L1, PD-1, CTLA-4, CEACAM, LAIR1, CD160, 2B4, CD80, CD86, CD276, VTCN1, HVEM, KIR, A2AR, MHC class I, MHC class II, GALS, adenosine, TGFR, OX40, CD137, CD40, IDO, CSF1R, TIM-3, BTLA, VISTA, LAG-3, TIGIT, IDO, MICA/B, and/or arginase. In some embodiments, the checkpoint inhibitor is an agent that inhibits PD-1. In some embodiments, the agent that inhibits PD-1 is a small molecule, a nucleic acid, a polypeptide, carbohydrate, a lipid, a metal, or a toxin. In some embodiments, the agent that inhibits PD-1 is a PD-1 binding antagonist. In some embodiments, the PD-1 binding antagonist is an antibody, antibody-drug conjugate, antibody fragment, or immunoadhesin. In some embodiments, the PD-1 binding antagonist is selected from the group consisting of pembrolizumab, nivolumab, cemiplimab, spartalizumab, genolimzumab, SHR1210, JS001, BGB-108, BGB-A317, IBI308, GLS-010, BMS-936558, BCD-100, REGN2810, MGA-012, BI 754091, STI-A1110, INCSHR-1210, PF-06801591, TSR-042, AM0001, JNJ-63723283, and ENUM 244C8. In some embodiments, the checkpoint inhibitor is an agent that inhibits PD-L1 and/or PD-L2. In some embodiments, the agent that inhibits PD-L1 and/or PD-L2 is a small molecule, a nucleic acid, a polypeptide, carbohydrate, a lipid, a metal, or a toxin. In some embodiments, the agent that inhibits PD-L1 is a PD-L1 binding antagonist. In some embodiments, the PD-L1 binding antagonist is an antibody, antibody-drug conjugate, antibody fragment, or immunoadhesin. In some embodiments, the PD-L1 binding antagonist is selected from the group consisting of atezolizumab, avelumab, durvalumab, KN035, CS1001, MDX-1105, LY3300054, STI-A1014, FAZ053, and CX-072. In some embodiments, the checkpoint inhibitor is an agent that inhibits CTLA4. In some embodiments, the agent that inhibits CTLA4 is a small molecule, a nucleic acid, a polypeptide, carbohydrate, a lipid, a metal, or a toxin. In some embodiments, the agent that inhibits CTLA4 is an antibody, antibody-drug conjugate, antibody fragment, or immunoadhesin. In some embodiments, the agent that inhibits CTLA4 is selected from the group consisting of ipilimumab, APL-509, AGEN1884, and CS1002. In some embodiments, the methods further comprise administering an additional anti-cancer therapy to the individual. In some embodiments, the additional anti-cancer therapy comprises one or more of surgery, radiotherapy, chemotherapy, anti-angiogenic therapy, anti-DNA repair therapy, and antiinflammatory therapy. In some embodiments, the additional anti-cancer therapy comprises a chemotherapeutic agent. In some embodiments, the chemotherapeutic agent is a platinum-based chemotherapeutic agent. In some embodiments, the methods further comprise administering an additional cancer immunotherapy to the individual.

Further provided herein is the use of a cancer immunotherapy in a method of treating or delaying progression of cancer according to any one of the above embodiments. Further provided herein is the use of a cancer immunotherapy for the manufacture of a medicament for treating or delaying progression of cancer according to any one of the above embodiments, e.g., wherein the medicament is to be administered to an individual, and wherein a Ewing sarcoma breakpoint region 1 (*EWSR1*) fusion gene encoding a neoantigen has been detected in a sample obtained from the individual.

It is to be understood that one, some, or all of the properties of the various embodiments described herein may be combined to form other embodiments of the present invention. These and other aspects of the invention will become apparent to one of skill in the art. These and other embodiments of the invention are further described by the detailed description that follows.

### BRIEF DESCRIPTION OF THE DRAWINGS

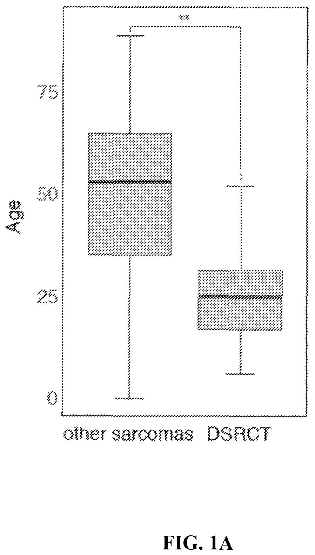

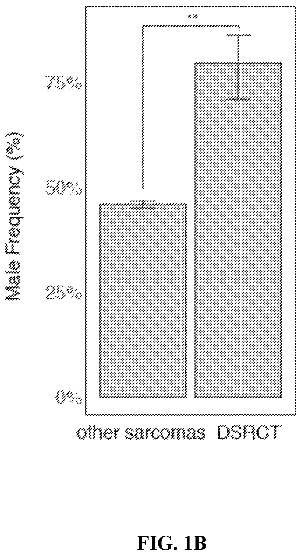

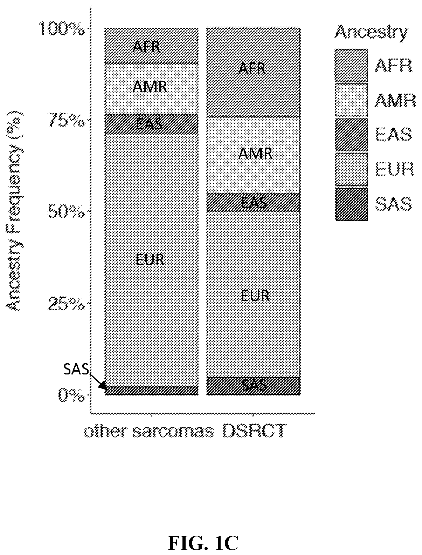

**FIGS. 1A-1C** show DSRCT patient characteristics. **FIG. 1A**: distribution of patient ages for the DSRCT and other sarcomas cohorts. **FIG. 1B****:** frequency of male patients in the DSRCT cohort and the cohort of other sarcomas. Error bars denote 95% confidence interval. **FIG. 1C****:** Ancestry frequencies in patients with DSRCT and in patients with other sarcomas.

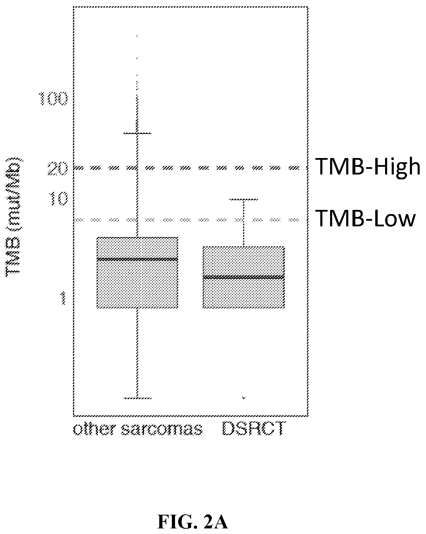

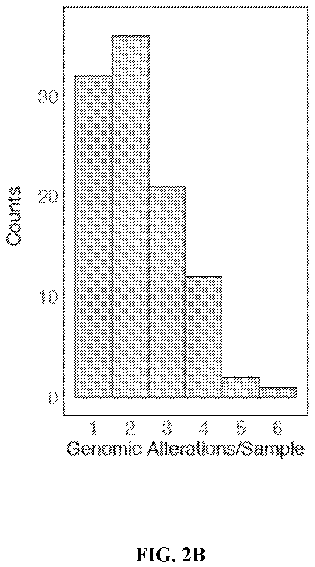

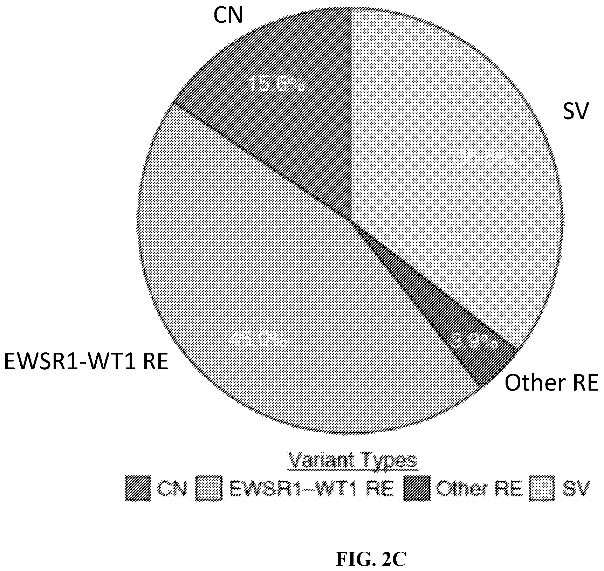

**FIGS. 2A-2C** show genomic alterations in DSRCT. **FIG. 2A****:** distribution of tumor mutational burden (TMB; mut/Mb) in DSRCT and other sarcoma samples. Dotted lines denote TMB-High (>20 mut/Mb) and TMB-low (<6 mut/Mb) cutoffs, as indicated. **FIG. 2B****:** histogram of the number of known or likely pathogenic genomic alterations per sample, inclusive of the requisite *EWSR1-WT1* rearrangement. **FIG. 2C****:** frequency of variant types. CN: copy number changes: RE: rearrangements; SV: short variants (insertions, deletions, and base substitutions).

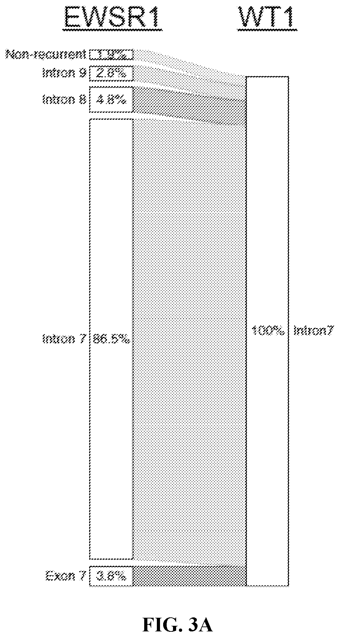

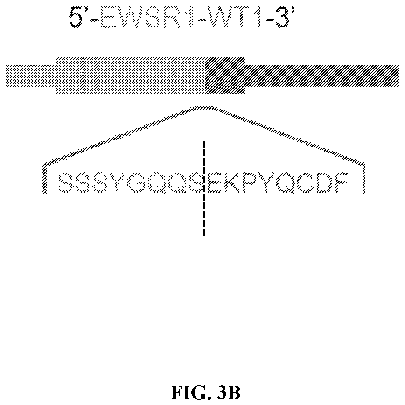

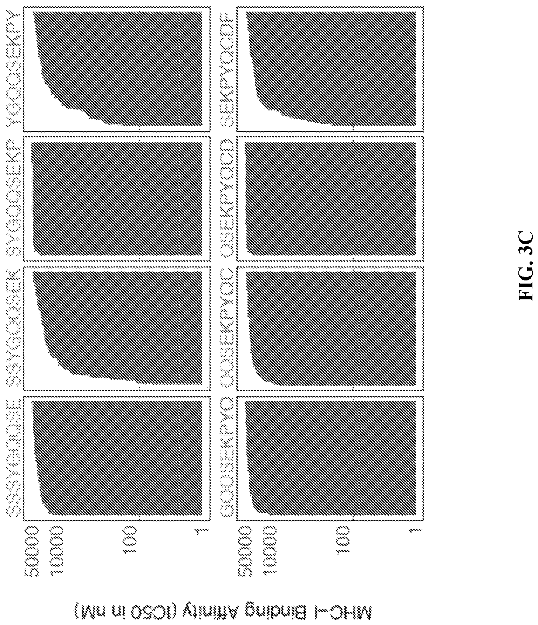

**FIGS. 3A-3C** show that pathogenic alterations are highly neoantigenic. **FIG. 3A****:** frequencies of breakpoints in *EWSR1* and their associated breakpoint in *WT1.* **FIG. 3B****:** schematic of the *EWSR1-WT1* fusion product. Boxes denote exons. Narrow regions denote noncoding regions of exons. Magnified sequence is the 16 amino acids around the fusion (indicated by dotted line). **FIG. 3C****:** waterfall plots of the IC₅₀ of combinations of all novel 9-mer amino acid sequences with all HLA-A, HLA-B, or HLA-C types identified in the DSRCT cohort. Dotted line indicates threshold used for strong binding (IC₅₀ < 50 nM).

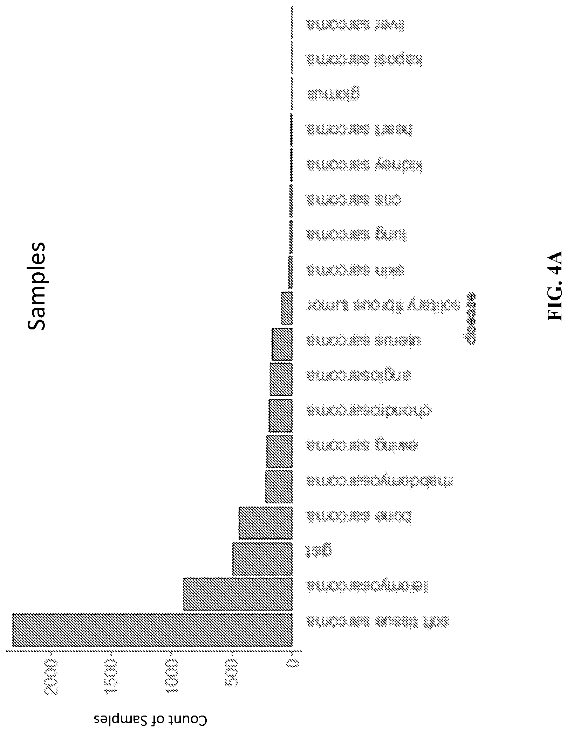

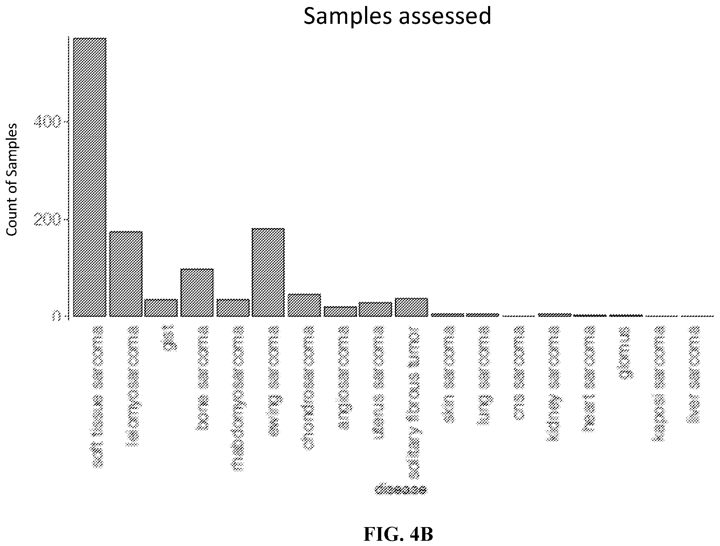

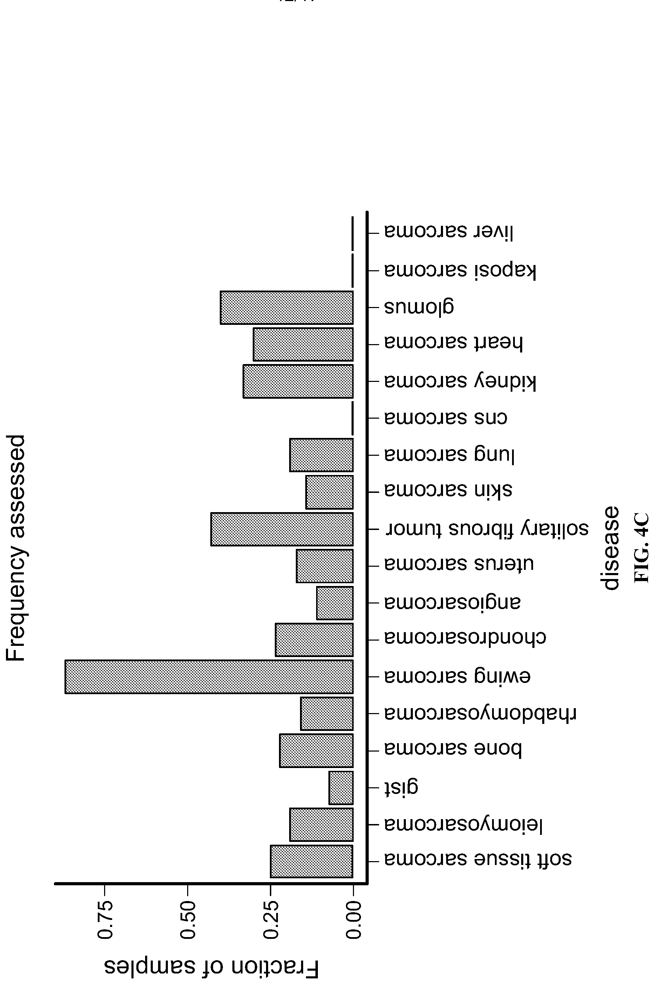

**FIGS. 4A-4C** show a count of total samples **(****FIG. 4A****),** a count of samples assessed **(****FIG. 4B****),** and the fraction of samples assessed **(****FIG. 4C****)** from the indicated sarcomas in which fusion neoantigens were identified.

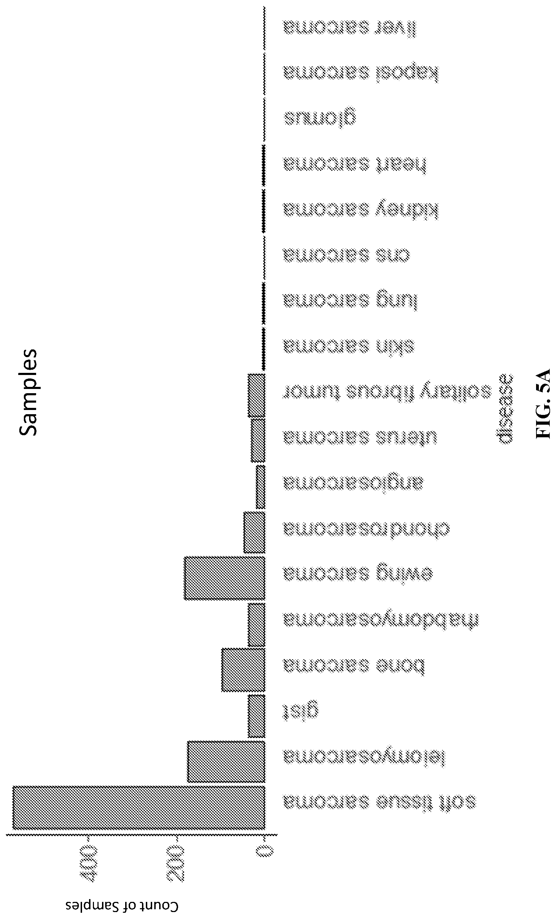

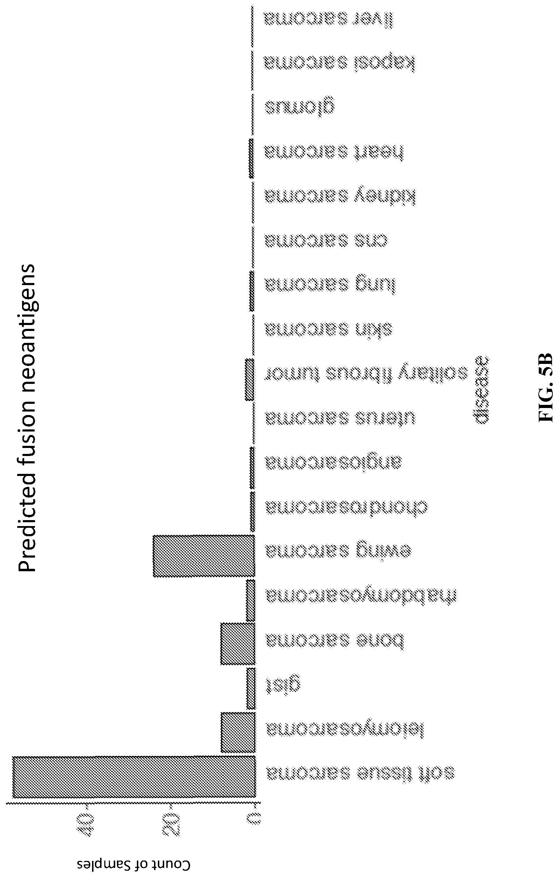

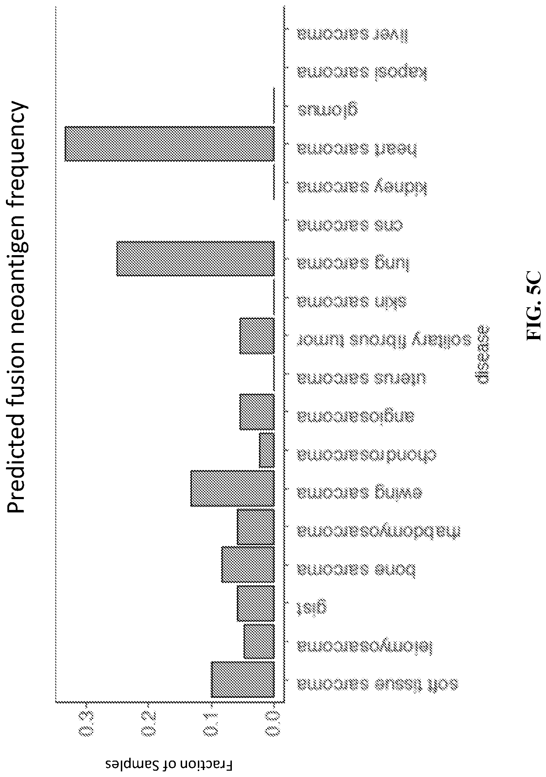

**FIGS. 5A-5C** show a count of samples assessed **(****FIG. 5A****),** a count of samples with predicted fusion neoantigens **(****FIG. 5B****),** and the fraction of samples with predicted fusion neoantigens **(****FIG. 5C**) from the indicated sarcomas in which fusion neoantigens were identified.

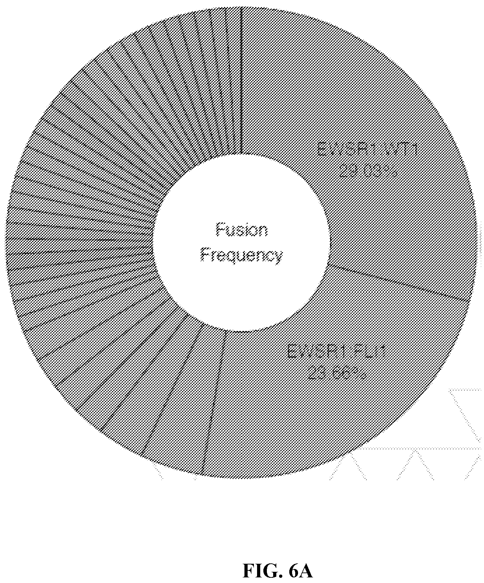

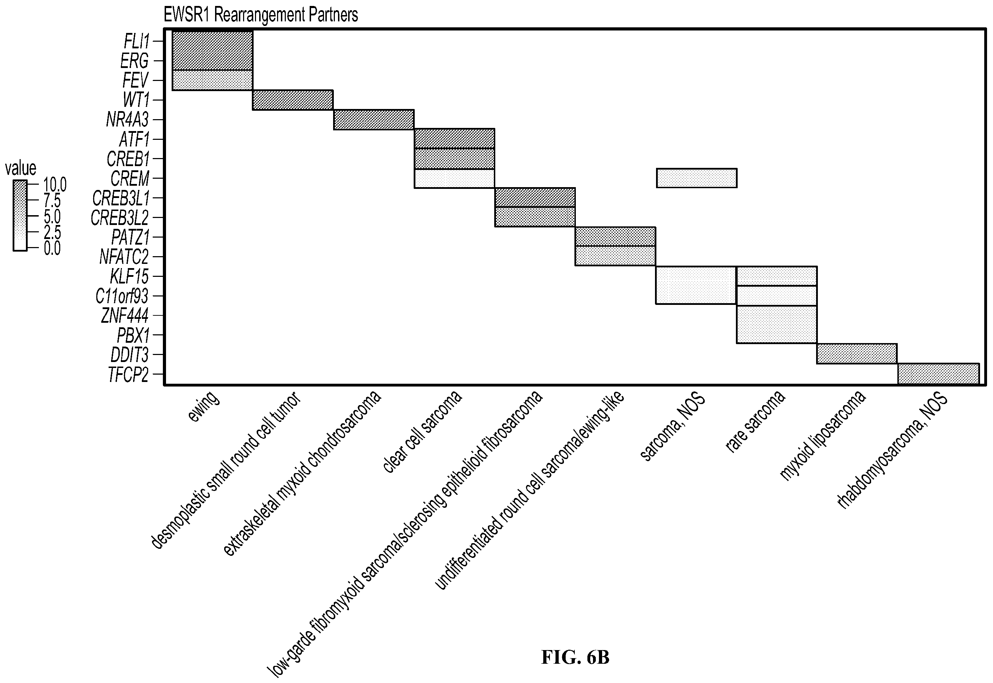

**FIGS. 6A** **&** **6B** show the *EWSR1* gene rearrangements leading to predicted fusion neoantigens that were detected, along with the frequency of *EWSR1-WT1* and *EWSR1-FLI1* gene fusions.

### DETAILED DESCRIPTION

The present disclosure relates generally to the detection of an *EWSR1-WT1* fusion gene encoding a neoantigen, as well as therapeutic methods related thereto. The present disclosure demonstrates that *EWSR1-WT1* fusion genes result in a consistent novel fusion peptide sequence, creating a neoantigen that binds strongly to MHC class I. Without wishing to be bound to theory, it is thought that the neoantigen(s) resulting from this gene fusion can serve as a target of the immune system according to various immunotherapeutic approaches, thereby providing a rationale for therapeutic treatment.

### I. General Techniques

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press, Cell Biology: A Laboratory Notebook (J.E. Cellis, ed., 1998) Academic Press, Animal Cell Culture (RI. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; *Handbook of Experimental Immunology* (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

### II. Definitions

As used in this specification and the appended claims, the singular forms "a", "an" and "the" include plural referents unless the content clearly dictates otherwise. Thus, for example, reference to "a molecule" optionally includes a combination of two or more such molecules, and the like.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of" aspects and embodiments.

The terms "cancer" and "cancerous" refer to or describe the physiological condition in mammals that is typically characterized by unregulated cell growth. Included in this definition are benign and malignant cancers. By "early stage cancer" or "early stage tumor" is meant a cancer that is not invasive or metastatic or is classified as a Stage 0, 1 , or 2 cancer. Examples of a cancer include, but are not limited to, a lung cancer (e.g.. a non-small cell lung cancer (NSCLC)), a kidney cancer (e.g, a kidney urothelial carcinoma), a bladder cancer (e g., a bladder urothelial (transitional cell) carcinoma), a breast cancer, a colorectal cancer (e.g., a colon adenocarcinoma), an ovarian cancer, a pancreatic cancer, a gastric carcinoma, an esophageal cancer, a mesothelioma, a melanoma (e.g., a skin melanoma), a head and neck cancer (e.g., a head and neck squamous cell carcinoma (HNSCC)), a thyroid cancer, a sarcoma (e.g., a soft-tissue sarcoma, a fibrosarcoma, a myxosarcoma, a liposarcoma, an osteogenic sarcoma, an osteosarcoma, a chondrosarcoma, an angiosarcoma, an endotheliosarcoma, a lymphangiosarcoma, a lymphangioendotheliosarcoma, a leiomyosarcoma, or a rhabdomyosarcoma), a prostate cancer, a glioblastoma, a cervical cancer, a thymic carcinoma, a leukemia (e.g., an acute lymphocytic leukemia (ALL), an acute myelocytic leukemia (AML), a chronic myelocytic leukemia (CML), a chronic eosinophilic leukemia, or a chronic lymphocytic leukemia (CLL)), a lymphoma (e.g., a Hodgkin lymphoma or a non-Hodgkin lymphoma (NHL)), a myeloma (e.g., a multiple myeloma (MM)), a mycoses fungoides, a merkel cell cancer, a hematologic malignancy, a cancer of hematological tissues, a B cell cancer, a bronchus cancer, a stomach cancer, a brain or central nervous system cancer, a peripheral nervous system cancer, a uterine or endometrial cancer, a cancer of the oral cavity or pharynx, a liver cancer, a testicular cancer, a biliary tract cancer, a small bowel or appendix cancer, a salivary gland cancer, an adrenal gland cancer, an adenocarcinoma, an inflammatory myofibroblastic tumor, a gastrointestinal stromal tumor (GIST), a colon cancer, a myelodysplastic syndrome (MDS), a myeloproliferative disorder (MPD), a polycythemia Vera, a chordoma, a synovioma, an Ewing's tumor, a squamous cell carcinoma, a basal cell carcinoma, an adenocarcinoma, a sweat gland carcinoma, a sebaceous gland carcinoma, a papillary carcinoma, a papillary adenocarcinoma, a medullary carcinoma, a bronchogenic carcinoma, a renal cell carcinoma, a hepatoma, a bile duct carcinoma, a choriocarcinoma, a seminoma, an embryonal carcinoma, a Wilms' tumor, a bladder carcinoma, an epithelial carcinoma, a glioma, an astrocytoma, a medulloblastoma, a craniopharyngioma, an ependymoma, a pinealoma, a hemangioblastoma, an acoustic neuroma, an oligodendroglioma, a meningioma, a neuroblastoma, a retinoblastoma, a follicular lymphoma, a diffuse large B-cell lymphoma, a mantle cell lymphoma, a hepatocellular carcinoma, a thyroid cancer, a small cell cancer, an essential thrombocythemia, an agnogenic myeloid metaplasia, a hypereosinophilic syndrome, a systemic mastocytosis, a familiar hypereosinophilia, a neuroendocrine cancer, or a carcinoid tumor.

The term "tumor," as used herein, refers to all neoplastic cell growth and proliferation, whether malignant or benign, and all pre-cancerous and cancerous cells and tissues. The terms "cancer," "cancerous." and "tumor" are not mutually exclusive as referred to herein.

As used herein, the term "checkpoint inhibitor" (the term "immune checkpoint inhibitor" may be used interchangeably herein) refers to a therapeutic agent that targets at least one immune checkpoint protein to alter the regulation of an immune response, e.g., down-modulating or inhibiting an immune response. Immune checkpoint proteins are known in the art and include, without limitation, cytotoxic T-lymphocyte antigen 4 (CTLA-4), programmed cell death 1 (PD-1), programmed cell death ligand 1 (PD-L1), programmed cell death ligand 2 (PD-L2), V-domain Ig suppressor of T cell activation (VISTA), B7-H2, B7-H3, B7-H4, B7-H6, 2B4, ICOS, HVEM, CD160, gp49B, PIR-B, KIR family receptors. TIM-1 , TIM-3. TIM-4, LAG-3, BTLA, SIRPalpha (CD47), CD48, 2B4 (CD244), B7.1 , B7 2, ILT-2, ILT-4, TIGIT, LAG-3, BTLA, IDO, OX40, and A2aR. In some instances, an immune checkpoint protein may be expressed on the surface of an activated T cell. Therapeutic agents that can act as immune checkpoint inhibitors useful in the methods of the present invention, include, but are not limited to, therapeutic agents that target one or more of CTLA-4, PD-1, PD-L1 , PD-L2, VISTA, B7-H2, B7-H3. B7-H4, B7-H6, 2B4, ICOS, HVEM, CD160, gp49B. PIR-B, KIR family receptors, TIM-1 , TIM-3, TIM-4, LAG- 3, BTLA, SIRPalpha (CD47), CD48, 2B4 (CD244), B7.1 , B7.2, ILT-2, ILT-4, TIGIT, LAG-3, BTLA, IDO, OX40, and A2aR. In some instances, an immune checkpoint inhibitor enhances or suppresses the function of one or more targeted immune checkpoint proteins. In some instances, the immune checkpoint inhibitor is a PD-L1 axis binding antagonists as described herein.

As used herein, a "PD-L1 binding antagonist" is a molecule that decreases, blocks, inhibits, abrogates or interferes with signal transduction resulting from the interaction of PD-L1 with either one or more of its binding partners, such as PD-1 and/or B7-1 . In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1 . In some embodiments, PD-L1 binding antagonists include anti-PD-L1 antibodies and antigen-binding fragments thereof, immunoadhesins, fusion proteins, oligopeptides, small molecule antagonists, polynucleotide antagonists, and other molecules that decrease, block, inhibit, abrogate or interfere with signal transduction resulting from the interaction of PD-L1 with one or more of its binding partners, such as PD-1 and/or B7-1 . In one embodiment, a PD-L1 binding antagonist reduces the negative signal mediated by or through cell surface proteins expressed on T lymphocytes and other cells through PD-L1 or PD-1 so as to render a dysfunctional T-cell less dysfunctional (e.g , enhancing effector responses to antigen recognition) In some embodiments, a PD-L1 binding antagonist is an anti-PD-L1 antibody. In a specific aspect, an anti-PD-L1 antibody is YW243.55 S70 described herein. In another specific aspect, an anti-PD-L1 antibody is MDX-1105 described herein. In still another specific aspect, an anti-PD-L1 antibody is atezolizumab (CAS Registry Number 1422185-06-5), also known as MPDL3280A, described herein. In still another specific aspect, an anti-PD-L1 antibody is MEDI4736 (durvalumab) described herein. In still another specific aspect, an anti-PD-L1 antibody is MSB0010718C (avelumab) described herein.

As used herein, a "PD-1 binding antagonist" is a molecule that decreases, blocks, inhibits, abrogates or interferes with signal transduction resulting from the interaction of PD-1 with one or more of its binding partners, such as PD-L1 and/or PD-L2. In some embodiments, the PD-1 binding antagonist is a molecule that inhibits the binding of PD-1 to its binding partners. In a specific aspect, the PD-1 binding antagonist inhibits the binding of PD-1 to PD-L1 and/or PD-L2. For example, PD-1 binding antagonists include anti-PD-1 antibodies and antigen-binding fragments thereof, immunoadhesins, fusion proteins, oligopeptides, small molecule antagonists, polynucleotide antagonists, and other molecules that decrease, block, inhibit, abrogate or interfere with signal transduction resulting from the interaction of PD-1 with PD-L1 and/or PD-L2. In one embodiment, a PD-1 binding antagonist reduces the negative signal mediated by or through cell surface proteins expressed on T lymphocytes and other cells through PD-1 or PD-L1 so as to render a dysfunctional T-cell less dysfunctional. In some embodiments, the PD-1 binding antagonist is an anti-PD-1 antibody. In a specific aspect, a PD-1 binding antagonist is MDX-1106 (nivolumab) described herein. In another specific aspect, a PD-1 binding antagonist is MK-3475 (pembrolizumab) described herein. In another specific aspect, a PD-1 binding antagonist is MED1-0680 (AMP-514). In another specific aspect, a PD-1 binding antagonist is PDR001 . In another specific aspect, a PD-1 binding antagonist is REGN2810. In another specific aspect, a PD-1 binding antagonist is BGB-108. In another specific aspect, a PD-1 binding antagonist is AMP-224 described herein.

The terms "Programmed Death Ligand 1 " and "PD-L1 " refer herein to a native sequence PD-L1 polypeptide, polypeptide variants (i.e., PD-L1 polypeptide variants), and fragments of a native sequence polypeptide and polypeptide variants (which are further defined herein). The PD-L1 polypeptide described herein may be that which is isolated from a variety of sources, such as from human tissue types or from another source, or prepared by recombinant or synthetic methods.

As used herein, a "CTLA4 antagonist" is a molecule that decreases, blocks, inhibits, abrogates or interferes with signal transduction resulting from the interaction of CTLA4 with one or more of its binding partners, such as CD80 and/or CD86. CTLA4 is also known as CD152, ALPSS. CD, CELIAC3, CTLA-4, GRD4, GSE, and IDDM12. For exemplary human CTLA4 sequence, *see* NCBI RefSeq NP_005205.2. In some embodiments, the CTLA4 antagonist is a molecule that inhibits the binding of CTLA4 to its binding partners. In a specific aspect, the CTLA4 antagonist inhibits the binding of CTLA4 to CD80 and/or CD86. For example, CTLA4 antagonists include anti-CTLA4 antibodies and antigen-binding fragments thereof, immunoadhesins, fusion proteins, oligopeptides, small molecule antagonists, polynucleotide antagonists, and other molecules that decrease, block, inhibit, abrogate or interfere with signal transduction resulting from the interaction of CTLA4 with CD80 and/or CD86. Exemplary CTLA4 antagonists are described herein.

The term "CTLA4" refers herein to a native sequence CTLA4 polypeptide, polypeptide variants (i.e., CTLA4 polypeptide variants), and fragments of a native sequence polypeptide and polypeptide variants. The CTLA4 polypeptide described herein may be that which is isolated from a variety of sources, such as from human tissue types or from another source, or prepared by recombinant or synthetic methods.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA The strands in such regions may be from the same molecule or from different molecules. The regions may include all of one or more of the molecules, but more typically involve only a region of some of the molecules One of the molecules of a triple-helical region often is an oligonucleotide The term "polynucleotide" specifically includes cDNAs.

A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally-occurring nucleotides with an analog, internucleotide modifications such as, for example, those with uncharged linkages (e.g., methyl phosphonates, phosphotriesters, phosphoamidates, carbamates, and the like) and with charged linkages (e.g., phosphorothioates, phosphorodithioates, and the like), those containing pendant moieties, such as, for example, proteins (e.g., nucleases, toxins, antibodies, signal peptides, poly-L-lysine, and the like), those with intercalators (e.g., acridine, psoralen, and the like), those containing chelators (e.g., metals, radioactive metals, boron, oxidative metals, and the like), those containing alkylators, those with modified linkages (e.g., alpha anomeric nucleic acids), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-0-methyl-, 2'-0-allyl-, 2'-fluoro-, or 2'-azido-ribose, carbocyclic sugar analogs, a-anomeric sugars, epimeric sugars such as arabinose, xyloses or lyxoses, pyranose sugars, furanose sugars, sedoheptuloses, acyclic analogs, and abasic nucleoside analogs such as methyl riboside. One or more phosphodiester linkages may be replaced by alternative linking groups. These alternative linking groups include, but are not limited to, embodiments wherein phosphate is replaced by P(0)S ("thioate"). P(S)S ("dithioate"). "(0)NR₂ ("amidate"), P(0)R, P(0)OR', CO or CH₂ ("formacetal"), in which each R or R' is independently H or substituted or unsubstituted alkyl (1 -20 C) optionally containing an ether (-0-) linkage, aryl, alkenyl, cycloalkyl, cycloalkenyl or araldyl. Not all linkages in a polynucleotide need be identical A polynucleotide can contain one or more different types of modifications as described herein and/or multiple modifications of the same type. The preceding description applies to all polynucleotides referred to herein, including RNA and DNA.

"Oligonucleotide," as used herein, generally refers to short, single stranded, polynucleotides that are, but not necessarily, less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "antibody" herein is used in the broadest sense and encompasses various antibody structures, including but not limited to monoclonal antibodies, polyclonal antibodies, multispecific antibodies (e.g.. bispecific antibodies), and antibody fragments so long as they exhibit the desired antigen-binding activity.

An "isolated" antibody is one which has been identified and separated and/or recovered from a component of its natural environment. Contaminant components of its natural environment are materials which would interfere with research, diagnostic, and/or therapeutic uses for the antibody, and may include enzymes, hormones, and other proteinaceous or nonproteinaceous solutes. In some embodiments, an antibody is purified (1 ) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. An isolated antibody includes the antibody in situ within recombinant cells since at least one component of the antibody's natural environment will not be present. Ordinarily, however, an isolated antibody will be prepared by at least one purification step.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

The "light chains" of antibodies (immunoglobulins) from any mammalian species can be assigned to one of two clearly distinct types, called kappa ("κ") and lambda ("A"), based on the amino acid sequences of their constant domains.

The term "constant domain" refers to the portion of an immunoglobulin molecule having a more conserved amino acid sequence relative to the other portion of the immunoglobulin, the variable domain, which contains the antigen binding site. The constant domain contains the CH1 , CH2, and CH3 domains (collectively, CH) of the heavy chain and the CHL (or CL) domain of the light chain.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat et al., Sequences of Proteins of Immunological Interest, Fifth Edition, National Institute of Health, Bethesda, Md. (1991 )). The constant domains are not involved directly in the binding of an antibody to an antigen, but exhibit various effector functions, such as participation of the antibody in antibody-dependent cellular toxicity.

The term "hypervariable region," "HVR," or "HV," as used herein, refers to the regions of an antibody variable domain which are hypervariable in sequence and/or form structurally defined loops. Generally, antibodies comprise six HVRs, three in the VH (H1 , H2, H3), and three in the VL (L1 , L2, L3). In native antibodies, H3 and L3 display the most diversity of the six HVRs, and H3 in particular is believed to play a unique role in conferring fine specificity to antibodies. See, for example, Xu et al , Immunity 13:37-45 (2000); Johnson and Wu, in Methods in Molecular Biology 248:1 -25 (Lo, ed., Human Press, Totowa, N.J., 2003). Indeed, naturally occurring camelid antibodies consisting of a heavy chain only are functional and stable in the absence of light chain. See, for example, Hamers-Casterman et al., Nature 363:446-448 (1 993). Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1 991 )). Chothia refers instead to the location of the structural loops (Chothia and Lesk J. Mol. Biol. 196:901 -917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat numbering) |
| H1 | H31-H35 | H26-H35 | H26-H32 | H30-H35 (Chothia numbering) |
| H2 | H50-H65 | H50-H58 | H53-H55 | H47-H58 |
| H3 | H95-H102 | H95-H 102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat *et al., supra,* for each of these definitions.

"Framework" or "FR" residues are those variable domain residues other than the HVR residues as herein defined.

The term "variable domain residue numbering as in Kabat" or "amino acid position numbering as m Kabat," and variations thereof, refers to the numbering system used for heavy chain variable domains or light chain variable domains of the compilation of antibodies in Kabat et al., supra. Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g., residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1 -107 of the light chain and residues 1 -1 13 of the heavy chain) (e.g., Kabat et al., Sequences of Immunological Interest. 5th Ed. Public Health Service, National Institutes of Health, Bethesda, Md. (1991 )). The "EU numbering system" or "EU index" is generally used when referring to a residue in an immunoglobulin heavy chain constant region (e.g., the EU index reported in Kabat et al., supra). The "EU index as in Kabat" refers to the residue numbering of the human IgG1 EU antibody.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

"Antibody fragments" comprise a portion of an intact antibody, in some embodiments comprising the antigen-binding region thereof. In some embodiments, the antibody fragment described herein is an antigen-binding fragment. Examples of antibody fragments include Fab, Fab', F(ab')2, and Fv fragments: diabodies; linear antibodies; single-chain antibody molecules; and multispecific antibodies formed from antibody fragments

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen. "Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site. The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cystemes between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv, see, e.g., Pluckthun, in The Pharmacology of Monoclonal Antibodies,vol. 1 13, Rosenburg and Moore eds., (Springer-Verlag. New York, 1994), pp. 269-315.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO 1993/01 161 : Hudson et al , Nat. Med. 9:129-134 (2003); and Hollinger et al., Proc. Natl Acad. Sci. USA 90: 6444-6448 (1993). Triabodies and tetrabodies are also described in Hudson et al., Nat Med 9:129-134 (2003).

The "class" of an antibody refers to the type of constant domain or constant region possessed by its heavy chain. There are five major classes of antibodies: IgA, IgD. IgE, IgG, and IgM. and several of these may be further divided into subclasses (isotypes), e.g , lgG1 , lgG2, lgG3, lgG4, lgA1 , and IgA2. The heavy chain constant domains that correspond to the different classes of antibodies are called α, δ, ε, γ, and µ, respectively.

The term "monoclonal antibody" as used herein refers to an antibody obtained from a population of substantially homogeneous antibodies. e.g., the individual antibodies comprising the population are identical except for possible mutations, e.g.. naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target-binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target-binding sequence can be further altered, for example, to improve affinity for the target, to humanize the target-binding sequence, to improve its production in cell culture, to reduce its immunogenicity in vivo, to create a multispecific antibody, etc., and that an antibody comprising the altered target-binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the invention may be made by a variety of techniques, including, for example, the hybridoma method (e.g., Kohler and Milstein, Nature 256:495-97 (1975); Hongo et al., Hybridoma 14 (3): 253-260 (1995), Harlow et al., Antibodies. A Laboratory Manual (Cold Spring Harbor Laboratory Press, 2nd ed. 1988); Hammerling et al., in: Monoclonal Antibodies and T-Cell Hybridomas 563-681 (Elsevier, N.Y , 1991 )), recombinant DNA methods (see, e.g., U.S Pat. No. 4,816,567), phage-display technologies (see, e g., Clackson et al., Nature, 352. 624-628 (1991 ); Marks et al.. J. Mol. Biol. 222. 581 -597 (1992); Sidhu et al., J. Mol. Biol. 338(2): 299-31 0 (2004); Lee et al., J. Mol. Biol. 340(5): 1073-1093 (2004); Fellouse, Proc. Natl. Acad. Sci. USA 101 (34): 12467-12472 (2004); and Lee et al., J. Immunol. Methods 284(1 -2): 1 1 9-132 (2004)), and technologies for producing human or human-like antibodies in animals that have parts or all of the human immunoglobulin loci or genes encoding human immunoglobulin sequences (see, e.g.. WO 1998/24893; WO 1996/34096; WO 1996/33735; WO 1991 /10741 ; Jakobovits et al., Proc. Natl. Acad. Sci. USA 90: 2551 (1993), Jakobovits et al., Nature 362. 255-258 (1993), Bruggemann et al.. Year in Immunol. 7.33 (1 993); U.S. Pat. Nos. 5,545,807; 5,545,806; 5,569,825; 5,625,126; 5,633,425; and 5,661 ,016; Marks et al., Bio/Technology 10. 779-783 (1992); Lonberg et al., Nature 368: 856-859 (1994); Morrison, Nature 368: 812-813 (1994); Fishwild et al., Nature Biotechnol. 14. 845-851 (1996); Neuberger, Nature Biotechnol. 14: 826 (1996); and Lonberg et al.. Intern. Rev. Immunol. 13: 65-93 (1995)).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see, e.g., U.S. Pat. No. 4,816,567; and Morrison et al., Proc. Natl. Acad Sci USA 81 :6851 -6855 (1984)). Chimeric antibodies include PRIMATIZED^{®} antibodies wherein the antigen-binding region of the antibody is derived from an antibody produced by, e.g., immunizing macaque monkeys with the antigen of interest.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human framework regions (FRs). In certain embodiments, a humanized antibody will comprise substantially all of at least one, and typically two, variable domains, in which all or substantially all of the HVRs (e g., CDRs) correspond to those of a non-human antibody, and all or substantially all of the FRs correspond to those of a human antibody. A humanized antibody optionally may comprise at least a portion of an antibody constant region derived from a human antibody.

A "humanized form" of an antibody, e.g., a non-human antibody, refers to an antibody that has undergone humanization.

The terms "anti-PD-L1 antibody" and "an antibody that binds to PD-L1" refer to an antibody that is capable of binding PD-L1 with sufficient affinity such that the antibody is useful as a diagnostic and/or therapeutic agent in targeting PD-L1. In one embodiment, the extent of binding of an anti-PD-L1 antibody to an unrelated, non-PD-L1 protein is less than about 10% of the binding of the antibody to PD-L1 as measured, for example, by a radioimmunoassay (RIA). In certain embodiments, an anti-PD-L1 antibody binds to an epitope of PD-L1 that is conserved among PD-L1 from different species.

The terms "anti-PD-1 antibody" and "an antibody that binds to PD-1" refer to an antibody that is capable of binding PD-1 with sufficient affinity such that the antibody is useful as a diagnostic and/or therapeutic agent in targeting PD-1. In one embodiment, the extent of binding of an anti-PD-1 antibody to an unrelated, non-PD-1 protein is less than about 10% of the binding of the antibody to PD-1 as measured, for example, by a radioimmunoassay (RIA). In certain embodiments, an anti-PD-1 antibody binds to an epitope of PD-1 that is conserved among PD-1 from different species.

The terms "anti-CTLA4 antibody" and "an antibody that binds to CTLA4" refer to an antibody that is capable of binding CTLA4 with sufficient affinity such that the antibody is useful as a diagnostic and/or therapeutic agent in targeting CTLA4. In one embodiment, the extent of binding of an anti-CTLA4 antibody to an unrelated, non-CTLA4 protein is less than about 10% of the binding of the antibody to CTLA4 as measured, for example, by a radioimmunoassay (RIA). In certain embodiments, an anti-CTLA4 antibody binds to an epitope of CTLA4 that is conserved among CTLA4 from different species.

A "blocking" antibody or an "antagonist" antibody is one which inhibits or reduces biological activity of the antigen it binds For example, blocking antibodies or antagonist antibodies substantially or completely inhibit the biological activity of the antigen
"Affinity" refers to the strength of the sum total of noncovalent interactions between a single binding site of a molecule (e.g., an antibody) and its binding partner (e.g, an antigen). Unless indicated otherwise, as used herein, "binding affinity" refers to intrinsic binding affinity which reflects a 1 .1 interaction between members of a binding pair (e.g.. antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

As used herein, the term "binds", "specifically binds to" or is "specific for" refers to measurable and reproducible interactions such as binding between a target and an antibody, which is determinative of the presence of the target in the presence of a heterogeneous population of molecules including biological molecules. For example, an antibody that binds to or specifically binds to a target (which can be an epitope) is an antibody that binds this target with greater affinity, avidity, more readily, and/or with greater duration than it binds to other targets. In one embodiment, the extent of binding of an antibody to an unrelated target is less than about 1 0% of the binding of the antibody to the target as measured, e.g., by a radioimmunoassay (RIA). In certain embodiments, an antibody that specifically binds to a target has a dissociation constant (Kd) of < 1 µM, < 100 nM, < 10 nM, < 1 nM, or < 0.1 nM. In certain embodiments, an antibody specifically binds to an epitope on a protein that is conserved among the protein from different species. In another embodiment, specific binding can include, but does not require exclusive binding.

An "affinity matured" antibody refers to an antibody with one or more alterations in one or more hypervariable regions (HVRs), compared to a parent antibody which does not possess such alterations, such alterations resulting in an improvement in the affinity of the antibody for antigen

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more

An "immunoconjugate" is an antibody conjugated to one or more heterologous molecule(s), including but not limited to a cytotoxic agent

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as lgG1 , lgG2 (including lgG2A and lgG2B), lgG3, or lgG4 subtypes, IgA (including lgA1 and lgA2), IgE. IgD or IgM. The Ig fusions include the substitution of a domain of a polypeptide or antibody described herein in the place of at least one variable region within an Ig molecule. In some embodiments, the immunoglobulin fusion includes the hinge, CH2 and CH3, or the hinge, CH1 , CH2 and CH3 regions of an IgG1 molecule. For the production of immunoglobulin fusions see also US Patent No. 5,428,130. For example, useful immunoadhesins as medicaments useful for therapy herein include polypeptides that comprise the extracellular domain (ECD) or PD-1 -binding portions of PD-L1 or PD-L2, or the extracellular or PD-L1 - or PD-L2-binding portions of PD-1 , fused to a constant domain of an immunoglobulin sequence, such as a PD-L1 ECD-Fc, a PD-L2 ECD-Fc, and a PD-1 ECD-Fc, respectively. Immunoadhesin combinations of Ig Fc and ECD of cell surface receptors are sometimes termed soluble receptors.

A "fusion protein" and a "fusion polypeptide" refer to a polypeptide having two portions covalently linked together, where each of the portions is a polypeptide having a different property. The property may be a biological property, such as activity in vitro or in vivo. The property may also be a simple chemical or physical property, such as binding to a target molecule, catalysis of a reaction, and the like. The two portions may be linked directly by a single peptide bond or through a peptide linker but are in reading frame with each other
"Percent (%) amino acid sequence identity" with respect to the polypeptide sequences identified herein is defined as the percentage of amino acid residues in a candidate sequence that are identical with the amino acid residues in the polypeptide being compared, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program ALIGN-2. The ALIGN-2 sequence comparison computer program was authored by Genentech, Inc. and the source code has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, for example, digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: $100 times the fraction X / Y$ where X is the number of amino acid residues scored as identical matches by the sequence alignment program ALIGN-2 in that program's alignment of A and B, and where Y is the total number of amino acid residues in B It will be appreciated that where the length of amino acid sequence A is not equal to the length of amino acid sequence B, the % amino acid sequence identity of A to B will not equal the % amino acid sequence identity of B to A Unless specifically stated otherwise, all % amino acid sequence identity values used herein are obtained as described in the immediately preceding paragraph using the ALIGN-2 computer program.

The term "detection" includes any means of detecting, including direct and indirect detection. The term "biomarker" as used herein refers to an indicator, e.g., predictive, diagnostic, and/or prognostic, which can be detected in a sample. The biomarker may serve as an indicator of a particular subtype of a disease or disorder (e g., cancer) characterized by certain, molecular, pathological, histological, and/or clinical features (e.g., responsiveness to therapy including a checkpoint inhibitor). In some embodiments, a biomarker is a collection of genes or a collective number of mutations/alterations (e.g., somatic mutations) in a collection of genes. Biomarkers include, but are not limited to, polynucleotides (e.g., DNA and/or RNA), polynucleotide alterations (e.g., polynucleotide copy number alterations, e.g., DNA copy number alterations), polypeptides, polypeptide and polynucleotide modifications (e.g., post-translational modifications), carbohydrates, and/or glycolipid-based molecular markers.

The "amount" or "number" of somatic mutations associated with an increased clinical benefit to an individual is a detectable level in a biological sample. These can be measured by methods known to one skilled in the art and also disclosed herein. The amount of a somatic mutation assessed can be used to determine the response to the treatment.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The technique of "polymerase chain reaction" or "PCR" as used herein generally refers to a procedure wherein minute amounts of a specific piece of nucleic acid, RNA and/or DNA, are amplified as described, for example, in U.S. Pat. No. 4,683,195. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage, or plasmid sequences, etc. See generally Mullis et al., Cold Spring Harbor Symp. Quant. Biol. 51 :263 (1987) and Erlich, ed., PCR Technology (Stockton Press, NY, 1989). As used herein, PCR is considered to be one, but not the only, example of a nucleic acid polymerase reaction method for amplifying a nucleic acid test sample, comprising the use of a known nucleic acid (DNA or RNA) as a primer and utilizes a nucleic acid polymerase to amplify or generate a specific piece of nucleic acid or to amplify or generate a specific piece of nucleic acid which is complementary to a particular nucleic acid.

The term "diagnosis" is used herein to refer to the identification or classification of a molecular or pathological state, disease or condition (e.g., cancer). For example, "diagnosis" may refer to identification of a particular type of cancer. "Diagnosis" may also refer to the classification of a particular subtype of cancer, for instance, by histopathological criteria, or by molecular features (e.g., a subtype characterized by expression of one or a combination of biomarkers (e.g., particular genes or proteins encoded by said genes)).

The term "aiding diagnosis" is used herein to refer to methods that assist in making a clinical determination regarding the presence, or nature, of a particular type of symptom or condition of a disease or disorder (e.g., cancer). For example, a method of aiding diagnosis of a disease or condition (e.g., cancer) can comprise measuring certain somatic mutations in a biological sample from an individual.

"Acquire" or "acquiring" as the terms are used herein, refer to obtaining possession of a physical entity, a value *(e.g.,* a quantitative or qualitative value), a sequence, or knowledge thereof by directly acquiring or indirectly acquiring the physical entity, value, or sequence. Directly acquiring means performing a process (e.g., performing a synthetic or analytical method, an *in vitro* method, etc.) to obtain the physical entity, value, or sequence, e.g., using any methodology known in the art. In some embodiments, directly acquiring a physical entity may include performing a process that causes a physical change in a physical substance, *e.g*., a starting material. Exemplary changes include, but are not limited to, reacting two or more starting materials (e.g., to make a substance), shearing or fragmenting a substance, separating or purifying a substance, combining two or more separate entities into a mixture, performing a chemical reaction that includes breaking or forming a covalent or non-covalent bond. Directly acquiring a value may include, but is not limited to, performing a process that includes a physical change in a sample or another substance, e.g., performing an analytical process which includes a physical change in a substance, e.g., a sample, analyte, or reagent, performing an analytical method, e.g., a method which includes one or more of the following: separating, purifying, or characterizing a substance, e.g., an analyte, or a fragment or other derivative thereof, from another substance; combining an analyte, or fragment or other derivative thereof, with another substance, e.g., a buffer, solvent, or reactant; or changing the structure of an analyte, or a fragment or other derivative thereof, *e.g.,* by breaking or forming a covalent or non-covalent bond, between a first and a second atom of the analyte, or by changing the structure of another substance, or a fragment or other derivative thereof, *e.g*., by breaking or forming a covalent or non-covalent bond, between a first and a second atom of the other substance (*e.g*., a reagent). For example, directly acquiring a mutation or fusion gene can include performing a process to obtain the sequence, such as performing a sequencing method (*e.g*., a DNA sequencing method, an RNA sequencing method, a methylation sequencing method, etc.). The sequence acquired or obtained need not be a full sequence, *e.g*., sequencing of at least one nucleotide, a region, a fragment etc.. Indirectly acquiring refers to receiving the physical entity, value, or sequence from another source, including another party *(e.g.,* a third-party laboratory that directly acquired the physical entity or value), or receiving from the source confirmation that a particular sequence or fusion gene is present, without necessarily receiving the sequence *per se.* "Acquiring knowledge" of a physical entity *(e.g.,* a mutation or fusion gene) or a value can encompass directly or indirectly acquiring the entity or value.

The term "sample," as used herein, refers to a composition that is obtained or derived from a subject and/or individual of interest that contains a cellular and/or other molecular entity that is to be characterized and/or identified, for example, based on physical, biochemical, chemical, and/or physiological characteristics. For example, the phrase "disease sample" and variations thereof refers to any sample obtained from a subject of interest that would be expected or is known to contain the cellular and/or molecular entity that is to be characterized. Samples include, but are not limited to, tissue samples, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, plasma, serum, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof. In some instances, the sample is a whole blood sample, a plasma sample, a serum sample, or a combination thereof. In some embodiments, the sample is from a tumor (e.g., a "tumor sample"), such as from a biopsy. In some embodiments, the sample is a formalin-fixed paraffin-embedded (FFPE) sample.

A "tumor cell" as used herein, refers to any tumor cell present in a tumor or a sample thereof. Tumor cells may be distinguished from other cells that may be present in a tumor sample, for example, stromal cells and tumor-infiltrating immune cells, using methods known in the art and/or described herein.

A "reference sample," "reference cell," "reference tissue," "control sample," "control cell," or "control tissue," as used herein, refers to a sample, cell, tissue, standard, or level that is used for comparison purposes.

By "correlate" or "correlating" is meant comparing, in any way, the performance and/or results of a first analysis or protocol with the performance and/or results of a second analysis or protocol. For example, one may use the results of a first analysis or protocol in carrying out a second protocol and/or one may use the results of a first analysis or protocol to determine whether a second analysis or protocol should be performed. With respect to the embodiment of polypeptide analysis or protocol, one may use the results of the polypeptide expression analysis or protocol to determine whether a specific therapeutic regimen should be performed. With respect to the embodiment of polynucleotide analysis or protocol, one may use the results of the polynucleotide expression analysis or protocol to determine whether a specific therapeutic regimen should be performed.

"Individual response" or "response" can be assessed using any endpoint indicating a benefit to the individual, including, without limitation, (1 ) inhibition, to some extent, of disease progression (e.g., cancer progression), including slowing down or complete arrest; (2) a reduction in tumor size; (3) inhibition (i.e., reduction, slowing down, or complete stopping) of cancer cell infiltration into adjacent peripheral organs and/or tissues; (4) inhibition (i.e. reduction, slowing down, or complete stopping) of metastasis; (5) relief, to some extent, of one or more symptoms associated with the disease or disorder (e.g., cancer); (6) increase or extension in the length of survival, including overall survival and progression free survival; and/or (7) decreased mortality at a given point of time following treatment.

An "effective response" of a patient or a patient's "responsiveness" to treatment with a medicament and similar wording refers to the clinical or therapeutic benefit imparted to a patient at risk for, or suffering from, a disease or disorder, such as cancer. In one embodiment, such benefit includes any one or more of: extending survival (including overall survival and/or progression-free survival); resulting in an objective response (including a complete response or a partial response); or improving signs or symptoms of cancer.

An "objective response" refers to a measurable response, including complete response (CR) or partial response (PR). In some embodiments, the "objective response rate (ORR)" refers to the sum of complete response (CR) rate and partial response (PR) rate.

By "complete response" or "CR" is intended the disappearance of all signs of cancer (e.g., disappearance of all target lesions) in response to treatment. This does not always mean the cancer has been cured.

"Sustained response" refers to the sustained effect on reducing tumor growth after cessation of a treatment. For example, the tumor size may be the same size or smaller as compared to the size at the beginning of the medicament administration phase. In some embodiments, the sustained response has a duration at least the same as the treatment duration, at least 1 .5x, 2.0x, 2.5x, or 3.0x length of the treatment duration, or longer.

As used herein, "reducing or inhibiting cancer relapse" means to reduce or inhibit tumor or cancer relapse or tumor or cancer progression. As disclosed herein, cancer relapse and/or cancer progression include, without limitation, cancer metastasis.

As used herein, "partial response" or "PR" refers to a decrease in the size of one or more tumors or lesions, or in the extent of cancer in the body, in response to treatment. For example, in some embodiments, PR refers to at least a 30% decrease in the sum of the longest diameters (SLD) of target lesions, taking as reference the baseline SLD.

As used herein, "stable disease" or "SD" refers to neither sufficient shrinkage of target lesions to qualify for PR, nor sufficient increase to qualify for PD, taking as reference the smallest SLD since the treatment started.

As used herein, "progressive disease" or "PD" refers to at least a 20% increase in the SLD of target lesions, taking as reference the smallest SLD recorded since the treatment started or the presence of one or more new lesions

The term "survival" refers to the patient remaining alive, and includes overall survival as well as progression-free survival.

As used herein, "progression-free survival" or "PFS" refers to the length of time during and after treatment during which the disease being treated (e.g., cancer) does not get worse. Progression-free survival may include the amount of time patients have experienced a complete response or a partial response, as well as the amount of time patients have experienced stable disease.

As used herein, "overall survival" or "OS" refers to the percentage of individuals in a group who are likely to be alive after a particular duration of time.

By "extending survival" is meant increasing overall or progression-free survival in a treated patient relative to an untreated patient (i.e. relative to a patient not treated with the medicament), or relative to a patient who does not have somatic mutations at the designated level, and/or relative to a patient treated with an anti-tumor agent.

An "effective amount" refers to an amount of a therapeutic agent to treat or prevent a disease or disorder in a mammal. In the case of cancers, the therapeutically effective amount of the therapeutic agent may reduce the number of cancer cells; reduce the primary tumor size; inhibit (i.e., slow to some extent and in some embodiments stop) cancer cell infiltration into peripheral organs; inhibit (i.e., slow to some extent and in some embodiments stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), response rates (e.g., CR and PR), duration of response, and/or quality of life.

The term "tumor," as used herein, refers to all neoplastic cell growth and proliferation, whether malignant or benign, and all pre-cancerous and cancerous cells and tissues. The terms "cancer," "cancerous," and "tumor" are not mutually exclusive as referred to herein.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies (e.g., antibody-based checkpoint inhibitors) are used to delay development of a disease or to slow the progression of a disease.

As used herein, the terms "individual," "patient," or "subject" are used interchangeably and refer to any single animal, e.g., a mammal (including such non-human animals as, for example, dogs, cats, horses, rabbits, zoo animals, cows, pigs, sheep, and non-human primates) for which treatment is desired. In particular embodiments, the patient herein is a human.

As used herein, "administering" is meant a method of giving a dosage of a compound (e.g., an antagonist) or a pharmaceutical composition (e.g., a pharmaceutical composition including an antagonist) to a subject (e.g., a patient). Administering can be by any suitable means, including parenteral, intrapulmonary, and intranasal, and, if desired for local treatment, intralesional administration. Parenteral infusions include, for example, intramuscular, intravenous, intraarterial, intraperitoneal, or subcutaneous administration. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "reduce or inhibit" is meant the ability to cause an overall decrease of 20%, 30%, 40%, 50%, 60%, 70%, 75%, 80%, 85%, 90%, 95%, or greater. Reduce or inhibit can refer, for example, to the symptoms of the disorder being treated, the presence or size of metastases, or the size of the primary tumor.

The term "package insert" is used to refer to instructions customarily included in commercial packages of therapeutic products, that contain information about the indications, usage, dosage, administration, combination therapy, contraindications, and/or warnings concerning the use of such therapeutic products.

An "article of manufacture" is any manufacture (e.g., a package or container) or kit comprising at least one reagent, e.g., a medicament for treatment of a disease or disorder (e.g., cancer), or a probe for specifically detecting a biomarker (e.g., a neoantigen or *EWSR1-WT1* gene fusion) described herein. In certain embodiments, the manufacture or kit is promoted, distributed, or sold as a unit for performing the methods described herein.

The phrase "based on" when used herein means that the information about one or more biomarkers is used to inform a treatment decision, information provided on a package insert, or marketing/promotional guidance, etc.

### III. Methods

In one aspect, provided herein are methods of detecting a fusion gene encoding a neoantigen. In another aspect, provided herein are methods of identifying an individual who may benefit from a treatment comprising a cancer immunotherapy. In another aspect, provided herein are methods of selecting a therapy for an individual. In another aspect, provided herein are methods of identifying one or more treatment options for an individual. In another aspect, provided herein are methods of treating or delaying progression of cancer. In some embodiments, the methods comprise detecting presence of an *EWSR1* or *EWSR1-WT1* fusion gene encoding a neoantigen, *e.g.,* in a patient sample. In some embodiments, the presence of an *EWSR1* or *EWSRI-WT1* fusion gene encoding a neoantigen is detected *in vitro.*

The present disclosure provides, *inter alia,* prognostic and pharmacodynamic methods. In some instances, the methods may involve providing a prognosis for an individual having a cancer. In other instances, the methods may involve monitoring a response of a patient to treatment with a cancer immunotherapy. In particular instances, the methods and assays provided herein may be used to determine whether an individual having a cancer is likely to respond to treatment with a cancer immunotherapy, the method including detecting presence of an *EWSR1* or *EWSR1-WT1* fusion gene encoding a neoantigen *(e.g.,* in a patient sample), wherein the presence of an *EWSR1* or *EWSR1-WT1* fusion gene encoding a neoantigen identifies the individual as one who is likely to respond to treatment comprising a cancer immunotherapy.

Any of the methods of the present disclosure may include selecting a cancer immunotherapy for the individual. In some embodiments, the method further comprises administering a cancer immunotherapy to the individual. In some embodiments, the method may further include selecting and/or administering a cancer immunotherapy. In some embodiments, the cancer immunotherapy is selected and/or administered to the individual as soon as possible.

In some embodiments, the methods further comprise generating a report comprising one or more treatment options identified for an individual based at least in part on the presence of an *EWSR1* or *EWSR1-WT1* fusion gene encoding a neoantigen, *e.g.,* in a sample obtained from the patient. In some embodiments, the report further comprises a score that associates the one or more treatment options with a predicted outcome and/or response.

In some embodiments, the methods further comprise administering to an individual an effective amount of a treatment comprising a cancer immunotherapy. Exemplary cancer immunotherapies and their administration are described *infra.* In some embodiments, an *EWSR1* or *EWSR1-WT1* fusion gene encoding a neoantigen has been detected in a sample from the cancer of the individual prior to administration of a treatment of the present disclosure. In some embodiments, the methods further comprise detecting an *EWSR1* or *EWSR1-WT1* fusion gene encoding a neoantigen in a sample from an individual prior to administration of a treatment of the present disclosure. In some embodiments, the methods further comprise obtaining a sample from an individual prior to administration of a treatment of the present disclosure.

Certain aspects of the present disclosure relate to cancer, *e.g*., a tumor or cancer characterized by an *EWSR1* or *EWSR1-WT1* fusion gene encoding a neoantigen.

In certain embodiments, the cancer is a desmoplastic small round cell tumor (DSRCT). In some embodiments, the DSRCT is characterized by a low TMB. In some embodiments, the DSRCT is characterized by a TMB of less than about 20 mutations/Mb, less than about 15 mutations/Mb, less than about 10 mutations/Mb, less than about 6 mutations/Mb, less than about 5 mutations/Mb, less than about 3 mutations/Mb, or about 1 mutation/Mb. In some embodiments, the DSRCT is not characterized by MSI.

In some embodiments, the cancer is a sarcoma. In some embodiments, the sarcoma is selected from the group consisting of synovial sarcomas, ewing sarcomas, clear cell sarcomas, solitary fibrous tumors, myxoid liposarcomas, mesenchymal chondrosarcomas, uterine endometrial stromal sarcomas, undifferentiated round cell/ewing-like sarcomas, alveolar soft part sarcomas, alveolar rhabdomyosarcomas, inflammatory myofibroblastic tumors, extraskeletal myxoid chondrosarcomas, epithelioid hemangioendotheliomas, low grade fibromyxoid sarcomas, sclerosing epithelioid fibrosarcomas, dermatofibrosarcoma protuberans, and ossifying fibromyxoid tumors.

In some embodiments, the cancer is a carcinoma. In some embodiments, the carcinoma is a NUT midline carcinoma.

In some embodiments, the cancer is selected from the group consisting of a lung cancer (e g., a non-small cell lung cancer (NSCLC)), a kidney cancer (e.g. a kidney orothelial carcinoma), a bladder cancer (e.g., a bladder urothelial (transitional cell) carcinoma), a breast cancer, a colorectal cancer (e.g., a colon adenocarcinoma), an ovarian cancer, a pancreatic cancer, a gastric carcinoma, an esophageal cancer, a mesothelioma, a melanoma (e.g., a skin melanoma), a head and neck cancer (e.g., a head and neck squamous cell carcinoma (HNSCC)), a thyroid cancer, a sarcoma (e.g., a soft-tissue sarcoma, a fibrosarcoma, a myxosarcoma, a liposarcoma, an osteogenic sarcoma, an osteosarcoma, a chondrosarcoma, an angiosarcoma, an endotheliosarcoma, a lymphangiosarcoma, a lymphangioendotheliosarcoma, a leiomyosarcoma, or a rhabdomyosarcoma), a prostate cancer, a glioblastoma, a cervical cancer, a thymic carcinoma, a leukemia (e.g., an acute lymphocytic leukemia (ALL), an acute myelocytic leukemia (AML), a chronic myelocytic leukemia (CML), a chronic eosinophilic leukemia, or a chronic lymphocytic leukemia (CLL)), a lymphoma (e.g.. a Hodgkin lymphoma or a non-Hodgkin lymphoma (NHL)), a myeloma (e.g., a multiple myeloma (MM)), a mycoses fungoides, a merkel cell cancer, a hematologic malignancy, a cancer of hematological tissues, a B cell cancer, a bronchus cancer, a stomach cancer, a brain or central nervous system cancer, a peripheral nervous system cancer, a uterine or endometrial cancer, a cancer of the oral cavity or pharynx, a liver cancer, a testicular cancer, a biliary tract cancer, a small bowel or appendix cancer, a salivary gland cancer, an adrenal gland cancer, an adenocarcinoma, an inflammatory myofibroblastic tumor, a gastrointestinal stromal tumor (GIST), a colon cancer, a myelodysplastic syndrome (MDS), a myeloproliferative disorder (MPD), a polycythemia Vera, a chordoma, a synovioma, an Ewing's tumor, a squamous cell carcinoma, a basal cell carcinoma, an adenocarcinoma, a sweat gland carcinoma, a sebaceous gland carcinoma, a papillary carcinoma, a papillary adenocarcinoma, a medullary carcinoma, a bronchogenic carcinoma, a renal cell carcinoma, a hepatoma, a bile duct carcinoma, a choriocarcinoma, a seminoma, an embryonal carcinoma, a Wilms' tumor, a bladder carcinoma, an epithelial carcinoma, a glioma, an astrocytoma, a medulloblastoma, a craniopharyngioma, an ependymoma, a pinealoma, a hemangioblastoma, an acoustic neuroma, an oligodendroglioma, a meningioma, a neuroblastoma, a retinoblastoma, a follicular lymphoma, a diffuse large B-cell lymphoma, a mantle cell lymphoma, a hepatocellular carcinoma, a thyroid cancer, a small cell cancer, an essential thrombocythemia, an agnogenic myeloid metaplasia, a hypereosinophilic syndrome, a systemic mastocytosis, a familiar hypereosinophilia, a neuroendocrine cancer, and a carcinoid tumor.

### Fusion genes and Neoantigens

Certain aspects of the present disclosure relate to detection of an *EWSR1-WT1* fusion gene encoding a neoantigen. Further aspects of the present disclosure relate to detection of a fusion gene between *EWSR1* and one of: *FLI1, ERG, FEV, NR4A3, ATF1, CREB1, CREM, CREB3L1, CREB3L2, PATZ1, NFATC2, KLF15, C11orf93, ZNF444, PBX1, DDT73,* and *TFCP2.*

In some embodiments, the *EWSR1* gene is a human *EWSR1* gene, also known as EWS-FLI1 and bK984G1.4. *EWSR1* encodes a multifunctional protein with an N-terminal transcriptional activation domain and a C-terminal RNA-binding domain. An exemplary *EWSR1* gene is represented by NCBI Gene ID No. 2130.

In some embodiments, the *WT1* gene is a human *WT1* gene, also known as GUD, AWT1, WAGR, WT33, NPHS4, and WIT-2. *WT1* encodes a transcription factor with 4 zinc finger motifs at the DNA binding C-terminus and a pro/glu-rich DNA binding domain at the N-terminus. An exemplary *WT1* gene is represented by NCBI Gene ID No. 7490.

In some embodiments, an *EWSR1-WT1* fusion gene of the present disclosure comprises a coding sequence of *EWSR1* at the 5' end and a coding sequence of *WT1* at the 3' end. In some embodiments, an *EWSR1-WT1* fusion gene of the present disclosure encodes the N-terminal activation domain of EWSR1 and the C-terminal DNA binding domain of WT1. In some embodiments, an *EWSR1-WT1* fusion gene of the present disclosure is formed via a breakpoint in intron 7, intron 8, intron 9, or exon 7 of *EWSR1.* In some embodiments, an *EWSR1-WT1* fusion gene of the present disclosure is formed via breakpoints in intron 7 of *EWSR1* and intron 7 of *WT1.* In some embodiments, an *EWSR1-WT1* fusion gene of the present disclosure is formed via breakpoints shown in **FIG. 3A****.** In some embodiments, an *EWSR1-WT1* fusion gene of the present disclosure encodes exons 1-7 of *EWSR1* and exons 8-10 of *WT1.* In some embodiments, an *EWSR1-WT1* fusion gene of the present disclosure encodes exons 1-7 of *EWSR1* and exons 7-9 of *WT1.*

In some embodiments, a neoantigen of the present disclosure comprises at least 5 contiguous amino acids from the sequence SSYGQQSEK (SEQ ID NO:1), *e.g., 5,* 6, 7, 8, or 9 contiguous amino acids from the sequence SSYGQQSEK (SEQ ID NO:1), 6, 7, 8, or 9 contiguous amino acids from the sequence SSYGQQSEK (SEQ ID NO:1), 7, 8, or 9 contiguous amino acids from the sequence SSYGQQSEK (SEQ ID NO:1), or 8 or 9 contiguous amino acids from the sequence SSYGQQSEK (SEQ ID NO:1). In some embodiments, a neoantigen of the present disclosure comprises the sequence SSYGQQSEK (SEQ ID NO:1).

In some embodiments, a neoantigen of the present disclosure comprises at least 5 contiguous amino acids from the sequence YGQQSEKPY (SEQ ID NO:2), *e.g.,* 5, 6, 7, 8, or 9 contiguous amino acids from the sequence YGQQSEKPY (SEQ ID NO:2), 6, 7, 8, or 9 contiguous amino acids from the sequence YGQQSEKPY (SEQ ID NO:2), 7, 8, or 9 contiguous amino acids from the sequence YGQQSEKPY (SEQ ID NO:2), or 8 or 9 contiguous amino acids from the sequence YGQQSEKPY (SEQ ID NO:2). In some embodiments, a neoantigen of the present disclosure comprises the sequence YGQQSEKPY (SEQ ID NO:2).

In some embodiments, a neoantigen of the present disclosure encodes a sequence that has at least 80%, at least 85%, at least 90%, at least 91%, at least 92%, at least 93%, at least 94%, at least 95%, at least 96%, at least 97%, at least 98%, or at least 99% identity to the sequence SSSYGQQSEKPYQCDF (SEQ ID NO:3),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:4),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:5),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGEKPFSCRWPSCQKKFARSDELVRH
HNMHQRNMTKLQLAL (SEQ ID NO:6),

MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG

QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGEKPFSCRWPSCQKKFARSDEL
VRHHNMHQRNMTKLQLAL (SEQ ID NO:7),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:8),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGKTSEKPFSCRWPSCQKKFARSDELV
RHHNMHQRNMTKLQLAL (SEQ ID NO:9),

MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGKTSEKPFSCRWPSCQKKFARS
DELVRHHNMHQRNMTKLQLAL (SEQ ID NO:10), or

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:11)

In some embodiments, a neoantigen of the present disclosure encodes the sequence SSSYGQQSEKPYQCDF (SEQ ID NO:3),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:4),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:5),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGEKPFSCRWPSCQKKFARSDELVRH
HNMHQRNMTKLQLAL (SEQ ID NO:6),

MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGEKPFSCRWPSCQKKFARSDEL
VRHHNMHQRNMTKLQLAL (SEQ ID NO:7),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ

SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:8),

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGKTSEKPFSCRWPSCQKKFARSDELV
RHHNMHQRNMTKLQLAL (SEQ ID NO:9),

MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGKTSEKPFSCRWPSCQKKFARS
DELVRHHNMHQRNMTKLQLAL (SEQ ID NO:10), or

MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:11).

In some embodiments, the methods of the present disclosure further comprise detecting one or more additional mutations in a sample, tumor, or cancer. In some embodiments, the additional mutation(s) are in one or more genes other than *EWSR1* and *WT1.* In some embodiments, the additional mutation(s) are in one or more genes selected from the group consisting of ABL1, BRAF, CDKN1A, EPHA3, FGFR4, IKZF1, MCL1, NKX2-1, PMS2, RNF43, TET2, ACVR1B, BRCA1, CDKN1B, EPHB1, FH, INPP4B, MDM2, NOTCH 1, POLD1, ROS1, TGFBR2, AKT1, BRCA2, CDKN2A, EPHB4, FLCN, IRF2, MDM4, NOTCH2, POLE, RPTOR, TIPARP, AKT2, BRD4, CDKN2B, ERBB2, FLT1, IRF4, MED12, NOTCH3, PPARG, SDHA, TNFAIP3, AKT3, BRIP1, CDKN2C, ERBB3, FLT3, IRS2, MEF2B, NPMI, PPP2R1A, SDHB, TNFRSF14, ALK, BTG1, CEBPA, ERBB4, FOXL2, JAK1, MEN1, NRAS, PPP2R2A, SDHC, TP53, ALOX12B, BTG2, CHEK1, ERCC4, FUBP1, JAK2, MERTK, NT5C2, PRDM1, SDHD, TSC1, AMER1, BTK, CHEK2, ERG, GABRA6, JAK3, MET, NTRK1, PRKAR1A, SETD2, TSC2, APC, C11orf30, CIC, ERRFI1, GATA3, JUN, MITF, NTRK2, PRKCI, SF3B1, TYRO3, AR, CALR, CREBBP, ESR1, GATA4, KDM5A, MKNK1, NTRK3, PTCH1, SGK1, U2AF1, ARAF, CARD11, CRKL, EZH2, GATA6, KDM5C, MLH1, P2RY8, PTEN, SMAD2, VEGFA, ARFRP1, CASP8, CSF1R, FAM46C, GID4, (C17orf39), KDM6A, MPL, PALB2, PTPN11, SMAD4, VHL, ARID1A, CBFB, CSF3R, FANCA, GNA11, KDR, MRE11A, PARK2, PTPRO, SMARCA4, WHSC1, ASXL1, CBL, CTCF, FANCC, GNA13, KEAP1, MSH2, PARP1, QKI, SMARCB1, WHSC1L1, ATM, CCND1, CTNNA1, FANCG, GNAQ, KEL, MSH3, PARP2, RAC1, SMO, WTI, ATR, CCND2, CTNNB1, FANCL, GNAS, KIT, MSH6, PARP3, RAD21, SNCAIP, XPO1, ATRX, CCND3, CUL3, FAS, GRM3, KLHL6, MSTIR, PAX5, RAD51, SOCS1, XRCC2, AURKA, CCNE1, CUL4A, FBXW7, GSK3B, KMT2A, (MLL), MTAP, PBRM1, RAD51B, SOX2, ZNF217, AURKB, CD22, CXCR4, FGF10, H3F3A, KMT2D, (MLL2), MTOR, PDCD1, RAD51C, SOX9, ZNF703, AXIN1, CD274, CYP17A1, FGF12, HDAC1, KRAS, MUTYH, PDCD1LG2, RAD51D, SPEN, AXL, CD70, DAXX, FGF14, HGF, LTK, MYC, PDGFRA, RAD52, SPOP, BAP1, CD79A, DDR1, FGF19, HNF1A, LYN, MYCL, PDGFRB, RAD54L, SRC, BARD1, CD79B, DDR2, FGF23, HRAS, MAF, MYCN, PDK1, RAFI, STAG2, BCL2, CDC73, DIS3, FGF3, HSD3B1, MAP2K1, MYD88, PIK3C2B, RARA, STAT3, BCL2L1, CDH1, DNMT3A, FGF4, ID3, MAP2K2, NBN, PIK3C2G, RB1, STK11, BCL2L2, CDK12, DOT1L, FGF6, IDH1, MAP2K4, NF1, PIK3CA, RBM10, SUFU, BCL6, CDK4, EED, FGFR1, IDH2, MAP3K1, NF2, PIK3CB, REL, SYK, BCOR, CDK6, EGFR, FGFR2, IGF1R, MAP3K13, NFE2L2, PIK3R1, RET, TBX3, BCORL1, CDK8, EP300, FGFR3, IKBKE, MAPK1, NFKBIA, PIM1, RICTOR, TEK, BCR, CD74, ETV4, ETV5, ETV6, EWSR1, EZR, MYB, NUTM1, RSPO2, SDC4, SLC34A2, TERC, TERT, and TMPRSS2.

### Detection

Certain aspects of the present disclosure relate to detection of an *WSR1-WT1* fusion gene encoding a neoantigen in a sample, e.g., a patient sample. In some embodiments, the *EWSR1-WT1* fusion gene is detected *in vitro.*

In some embodiments, an *EWSR1-WT1* fusion gene encoding a neoantigen is detected from DNA, *e.g.,* from a sample of the present disclosure. In some embodiments, an *EWSR1-WT1* fusion gene encoding a neoantigen is detected from RNA, *e.g.,* from a sample of the present disclosure. In some embodiments, an *EWSR1-WT1* fusion gene encoding a neoantigen is detected from DNA and RNA, e.g., from a sample of the present disclosure.

Various methods for detecting DNA and/or RNA, *e.g.,* from an *EWSR1-WT1* fusion gene are known in the art. For example, in some embodiments, an *EWSR1-WT1* fusion gene is detected from DNA using next-generation sequencing (NGS), polymerase chain reaction (PCR), Sanger sequencing, or fluorescence *in situ* hybridization (FISH). In some embodiments, an *EWSR1-WT1* fusion gene is detected from RNA using RNA-sequencing (RNA-seq), polymerase chain reaction (PCR), Sanger sequencing, or fluorescence *in situ* hybridization (FISH). In some embodiments, an *EWSR1-WT1* fusion gene is detected from RNA by first synthesizing cDNA, then using RNA-sequencing (RNA-seq), polymerase chain reaction (PCR), Sanger sequencing, or fluorescence *in situ* hybridization (FISH). In some embodiments, the detection targets a specific or predetermined *EWSR1-WT1* fusion gene or neoantigen thereof. In some embodiments, the detection provides the sequence of an *EWSR1-WT1* fusion gene or neoantigen thereof.

In some embodiments, an *EWSR1-WT1* fusion gene or neoantigen is detected using one or more oligonucleotides. In some embodiments, an *EWSR1-WT1* fusion gene or neoantigen is detected by PCR amplification of a DNA or RNA sequence encoding the amino acid sequence of any one of SEQ ID Nos: 1-11, *e.g.,* using two or more oligonucleotides that hybridize with portions of the DNA or RNA (e.g., cDNA) sequence on opposite strands. In some embodiments, an *EWSR1-WT1* fusion gene or neoantigen is detected by sequencing DNA or RNA sequence encoding the amino acid sequence of any one of SEQ ID Nos: 1-11, *e.g.,* using one or more oligonucleotides that hybridize with portions of the DNA or RNA *(e.g.,* cDNA) sequence. In some embodiments, an *WSR1-WT1* fusion gene or neoantigen is detected by hybridization (*e.g., in situ* hybridization) of a DNA or RNA oligonucleotide with a sequence encoding the amino acid sequence of any one of SEQ ID Nos:1-11.

In some embodiments, the neoantigen binds a major histocompatibility complex (MHC) class I molecule, e.g., of an individual from whom the sample is obtained. In some embodiments, the neoantigen is predicted to bind a major histocompatibility complex (MHC) class I molecule, *e.g.,* of an individual from whom the sample is obtained. In some embodiments, the neoantigen binds, or is predicted to bind, an MHC class I molecule (e.g., of an individual from whom the sample is obtained) with a dissociation constant (K_{D}) of 250 nM or less, 200 nM or less, 150 nM or less, 100 nM or less, or 50 nM or less. Methods for determining and/or predicting binding affinity of an antigen for MHC class I are known in the art. For example, in some embodiments, antigen binding to MHC class I is predicted by the NetMHCpan v4.0 (IEDB) MHC-I binding prediction tool (*see, e.g.,* Hartmaier, R.J. et al. (2017) Genome Med. 9:16).

In some embodiments, the methods of the present disclosure further comprise genotyping one or more human leukocyte antigen (HLA)-A, HLA-B, and HLA-C alleles, *e.g*., from a patient sample. Methods for genotyping HLA alleles are known in the art. For example, in some embodiments, HLA alleles are genotyped using the OptiType HLA genotyping algorithm (*see, e.g.,* Szolek, A. et al. (2014) Bioinformatics 30:3310-3316). In some embodiments, a sample, tumor, or cancer of the present disclosure does not comprise a loss-of-heterozygosity at an HLA-A, HLA-B, or HLA-C allele that encodes an MHC class I molecule to which a neoantigen of the present disclosure binds, or is predicted to bind. In some embodiments, a sample, tumor, or cancer of the present disclosure comprises a loss-of-heterozygosity at one or more, but not all, of HLA-A, HLA-B, or HLA-C allele(s) that encode an MHC class I molecule to which a neoantigen of the present disclosure binds, or is predicted to bind. For example, in some embodiments, a sample, tumor, or cancer of the present disclosure has a LOH at an HLA-A and/or HLA-B allele, but a neoantigen of the present disclosure binds, or is predicted to bind, an MHC-I molecule of an HLA-C allele of the individual. In some embodiments, a sample, tumor, or cancer of the present disclosure has a LOH at an HLA-B and/or HLA-C allele, but a neoantigen of the present disclosure binds, or is predicted to bind, an MHC-1 molecule of an HLA-A allele of the individual. In some embodiments, a sample, tumor, or cancer of the present disclosure has a LOH at an HLA-A and/or HLA-C allele, but a neoantigen of the present disclosure binds, or is predicted to bind, an MHC-I molecule of an HLA-B allele of the individual. Methods for assessing LOH of one or more HLA alleles are known in the art. For example, in some embodiments, HLA-A, HLA-B, and/or HLA-C alleles are assessed for loss-of-heterozygosity (LOH) as described in Sun, J.X. et al. (2018) PLoS Comput. Biol. 14:e1005965.

In some embodiments, the methods of the present disclosure further comprise determining a tumor mutational burden (TMB), e.g., from a sample of the present disclosure. Methods for assessing TMB status are known in the art. For example, in some embodiments, the TMB of a sample or tumor is assessed by polynucleotide sequencing, e.g., quantifying a number of mutations detected per amount of DNA or RNA sequenced. In some embodiments, a sample or tumor with high TMB comprises at least about 10 mutations/Mb, at least about 15 mutations/Mb, at least about 20 mutations/Mb, or at least about 25 mutations/Mb. In some embodiments, a sample or tumor with low TMB comprises less than about 15 mutations/Mb, less than about 10 mutations/Mb, less than about 8 mutations/Mb, or less than about 6 mutations/Mb. In some embodiments, a sample, tumor, or cancer of the present disclosure is characterized by high TMB. In some embodiments, a sample, tumor, or cancer of the present disclosure is not characterized by high TMB. In some embodiments, a sample, tumor, or cancer of the present disclosure is characterized by low TMB. In some embodiments, a sample, tumor, or cancer of the present disclosure is not characterized by low TMB.

In some embodiments, the methods of the present disclosure further comprise determining microsatellite instability (MSI) status of a sample, tumor, or cancer of the present disclosure. Methods for assessing MSI status are known in the art. For example, in some embodiments, MSI status is determined from at least about 50 loci, at least about 60 loci, at least about 70 loci, at least about 80 loci, at least about 90 loci, at least about 100 loci, or about 114 loci. In some embodiments, MSI status is determined by principal component analysis (PCA), the results of which can be used to threshold MSI status. In some embodiments, a sample, tumor, or cancer of the present disclosure is characterized by MSI. In some embodiments, a sample, tumor, or cancer of the present disclosure is not characterized by MSI.

In some embodiments, a sample, tumor, or cancer of the present disclosure does not comprise a loss-of-function mutation in a gene required for or contributing to MHC class 1 function *(e.g.,* antigen presentation), including but not limited to the *CIITA* or *B2M* genes. In some embodiments, the methods of the present disclosure further comprise genotyping, e.g., from a patient sample, one or more gene(s) required for or contributing to MHC class I function *(e.g.,* antigen presentation), including but not limited to the *CIITA* or *B2M* genes.

In some embodiments, a sample of the present disclosure is a blood sample (e.g., a whole blood, plasma, or serum sample). In some embodiments, the sample (e.g., blood sample) obtained from the patient is selected from the group consisting of a whole blood, plasma, serum, or a combination thereof. In some instances, the sample is an archival blood sample, a fresh blood sample, or a frozen blood sample. In some embodiments, a sample of the present disclosure is a bone marrow sample. In some embodiments, a sample of the present disclosure is a tissue sample. In some embodiments, a sample of the present disclosure is from, or comprises, amniotic fluid, blood, plasma, serum, semen, lymphatic fluid, cerebral spinal fluid, ocular fluid, urine, saliva, stool, mucus, sweat, blood, skin, hair, hair follicles, saliva, oral mucous, vaginal mucus, sweat, tears, epithelial tissues, urine, semen, seminal fluid, seminal plasma, prostatic fluid, Cowper's fluid, excreta, biopsy, ascites, cerebrospinal fluid, and/or lymph. In some embodiments, the sample is a solid sample. In some embodiments, the sample is a liquid sample.

In some embodiments, a sample of the present disclosure is a tumor sample. In some embodiments, a sample of the present disclosure is a biopsy sample. In some embodiments, a sample of the present disclosure is a formalin-fixed paraffin-embedded (FFPE) sample.

In some embodiments, cell free DNA (cfDNA) and/or circulating tumor DNA (ctDNA) is isolated from a sample of the present disclosure (e g., a whole blood sample, a plasma sample, a serum sample, or a combination thereof). In some embodiments, the amount of cfDNA isolated from the sample is at least about 5 ng (e.g., at least about 5 ng, at least about 1 0 ng, at least about 15 ng, at least about 20 ng, at least about 25 ng, at least about 30 ng, at least about 35 ng, at least about 40 ng, at least about 45 ng, at least about 50 ng, at least about 75 ng, at least about 1 00 ng, at least about 200 ng, at least about 300 ng, at least about 400 ng, or more). For example, in some embodiments, the amount of cfDNA isolated from the sample is at least about 20 ng of ofDNA. In some embodiments, the amount of cfDNA isolated from the sample is, for example, from about 5 ng to about 1 00 ng (e.g., from about 5 ng to about 1 00 ng, from about 5 ng to about 90 ng, from about 5 ng to about 80 ng, from about 5 ng to about 70 ng, from about 5 ng to about 60 ng, from about 5 ng to about 50 ng, from about 5 ng to about 40 ng, from about 5 ng to about 30 ng, from about 5 ng to about 20 ng, from about 5 ng to about 1 5 ng, from about 5 ng to about 10 ng, from about 10 ng to about 100 ng, from about 10 ng to about 90 ng, from about 10 ng to about 80 ng, from about 10 ng to about 70 ng, from about 10 ng to about 60 ng, from about 10 ng to about 50 ng, from about 10 ng to about 40 ng, from about 10 ng to about 30 ng, from about 1 0 ng to about 20 ng, from about 15 ng to about 100 ng, from about 1 5 ng to about 90 ng, from about 15 ng to about 80 ng, from about 15 ng to about 70 ng, from about 15 ng to about 60 ng, from about 1 5 ng to about 50 ng, from about 20 ng to about 100 ng, from about 20 ng to about 90 ng, from about 20 ng to about 80 ng, from about 20 ng to about 70 ng, from about 20 ng to about 60 ng, from about 20 ng to about 50 ng, from about 20 ng to about 40 ng, from about 20 ng to about 30 ng, from about 25 ng to about 1 00 ng, from about 25 ng to about 90 ng, from about 25 ng to about 80 ng, from about 25 ng to about 70 ng, from about 25 ng to about 60 ng, from about 25 ng to about 50 ng, from about 25 ng to about 40 ng, from about 25 ng to about 30 ng, from about 30 ng to about 100 ng, from about 30 ng to about 90 ng, from about 30 ng to about 80 ng, from about 30 ng to about 70 ng, from about 30 ng to about 60 ng, from about 30 ng to about 50 ng, from about 30 ng to about 40 ng, from about 30 ng to about 35 ng, from about 35 ng to about 100 ng, from about 35 ng to about 90 ng, from about 35 ng to about 80 ng, from about 35 ng to about 70 ng, from about 35 ng to about 60 ng, from about 35 ng to about 50 ng, from about 35 ng to about 40 ng, from about 40 ng to about 100 ng, from about 40 ng to about 90 ng, from about 40 ng to about 80 ng, from about 40 ng to about 70 ng, from about 40 ng to about 60 ng, from about 40 ng to about 50 ng, from about 40 ng to about 45 ng, from about 50 ng to about 1 00 ng, from about 50 ng to about 90 ng, from about 50 ng to about 80 ng, from about 50 ng to about 70 ng, from about 50 ng to about 60 ng, from about 60 ng to about 100 ng, from about 60 ng to about 90 ng, from about 60 ng to about 80 ng, from about 60 ng to about 70 ng, from about 70 ng to about 1 00 ng, from about 70 ng to about 90 ng, from about 70 ng to about 80 ng, from about 80 ng to about 1 00 ng, from about 80 ng to about 90 ng, or from 90 ng to about 100 ng). In some embodiments, the amount of cfDNA isolated from the sample is about 100 ng or more (e.g., about 100 ng or more, about 200 ng or more, about 300 ng or more, about 400 ng or more, about 500 ng or more, about 600 ng or more, about 700 ng or more, about 800 ng or more, about 900 ng or more, or higher).

Any suitable sample volume may be used in any of the methods described herein. For example, in some instances, the sample (e.g.. a whole blood sample, a plasma sample, a serum sample, or a combination thereof) may have a volume of about 1 mL to about 50 mL, *e.g*., about 1 mL, about 2 mL. about 3 mL. about 4 mL, about 5 mL, about 6 mL, about 7 mL, about 8 mL, about 9 mL, about 10 mL, about 1 1 mL, about 12 mL, about 13 mL, about 14 mL, about 15 mL, about 16 mL. about 17 mL, about 18 mL, about 19 mL, about 20 mL, about 22 mL, about 24 mL, about 26 mL. about 28 mL, about 30 mL, about 32 mL, about 34 mL, about 36 mL, about 38 mL, about 40 mL, about 42 mL. about 44 mL. about 46 mL, about 48 mL, or about 50 mL. In some instances, the sample (e.g., a whole blood sample, a plasma sample, a serum sample, or a combination thereof) may have a volume of from about 1 mL to about 50 mL. from about 1 mL to about 40 mL, from about 1 mL to about 30 mL, from about 1 mL to about 20 mL. from about 1 mL to about 1 0 mL, from about 5 mL to about 50 mL, from about 5 mL to about 40 mL, from about 5 mL to about 30 mL, from about 5 mL to about 20 mL, from about 5 mL to about 10 mL, from about 6 mL to about 50 mL. from about 6 mL to about 40 mL, from about 6 mL to about 30 mL, from about 6 mL to about 20 mL, from about 6 mL to about 10 mL, from about 7 mL to about 50 mL, from about 7 mL to about 40 mL, from about 7 mL to about 30 mL, from about 7 mL to about 20 mL, from about 7 mL to about 10 mL, from about 8 mL to about 50 mL, from about 8 mL to about 40 mL, from about 8 mL to about 30 mL, from about 8 mL to about 20 mL, from about 8 mL to about 10 mL, from about 9 mL to about 50 mL, from about 9 mL to about 40 mL, from about 9 mL to about 30 mL, from about 9 mL to about 20 mL, from about 9 mL to about 10 mL, from about 5 mL to about 15 mL, from about 5 mL to about 14 mL, from about 5 mL to about 13 mL, from about 5 mL to about 12 mL, from about 5 mL to about 1 1 mL, from about 6 mL to about 15 mL, from about 6 mL to about 14 mL, from about 6 mL to about 13 mL, from about 6 mL to about 12 mL, from about 6 mL to about 1 1 mL, from about 7 mL to about 15 mL, from about 7 mL to about 14 mL, from about 7 mL to about 13 mL, from about 7 mL to about 12 mL, from about 7 mL to about 1 1 mL, from about 8 mL to about 15 mL, from about 8 mL to about 14 mL, from about 8 mL to about 13 mL, from about 8 mL to about 12 mL, from about 8 mL to about 1 1 mL, from about 9 mL to about 15 mL. from about 9 mL to about 14 mL, from about 9 mL to about 13 mL, from about 9 mL to about 12 mL, or from about 9 mL to about 11 mL. In some instances, the sample (e.g., a whole blood sample, a plasma sample, a serum sample, or a combination thereof) has a volume of about 10 mL. For example, in some instances, a plasma sample has a volume of 10 mL.

In some embodiments, the sample comprises a surface area of greater than or equal to about 25mm². In some embodiments, the sample comprises a volume of greater than or equal to about 1mm³. In some embodiments, the sample comprises at least about 30,000 cells. In some embodiments, the sample comprises at least about 80% cells. In some embodiments, the sample comprises greater than or equal to about 20% tumor content. In some embodiments, at least about 50ng of dsDNA is obtained from the sample.

In some embodiments of any of the methods described herein, the sample from the individual is obtained from the individual prior to administration of a treatment of the present disclosure, *e.g*., comprising a cancer immunotherapy. In other words, the sample may be a baseline sample.

Various methods for detecting DNA and/or RNA, are known in the art. In some embodiments, methods for detecting DNA and/or RNA can be performed using techniques known in the art including, but not limited to, sequence analysis, and electrophoretic analysis. Non-limiting examples of sequence analysis include Maxam-Gilbert sequencing, Sanger sequencing, capillary array DNA sequencing, thermal cycle sequencing (Sears et al., Biotechniques, 13:626-633 (1992)), solid-phase sequencing (Zimmerman et al., Methods Mol. Cell Biol, 3:39-42 (1992)), sequencing with mass spectrometry such as matrix-assisted laser desorption/ionization time-of-flight mass spectrometry (MALDI-TOF/MS; Fu et al., Nat. Biotechnol, 16:381-384 (1998)), and sequencing by hybridization. Chee et al., Science, 274:610-614 (1996); Drmanac et al., Science, 260:1649-1652 (1993); Drmanac et al., Nat. Biotechnol, 16:54-58 (1998). Non-limiting examples of electrophoretic analysis include slab gel electrophoresis such as agarose or polyacrylamide gel electrophoresis, capillary electrophoresis, and denaturing gradient gel electrophoresis. In some embodiments, next-generation sequencing methods can be performed using commercially available kits and instruments from companies such as the Life Technologies/Ion Torrent PGM or Proton, the Illumina HiSEQ or MiSEQ, and the Roche/454 next-generation sequencing system.

In some embodiments, DNA and/or RNA can be detected by the use of nucleic acid amplification techniques that are well known in the art. In some embodiments, the starting material may be genomic DNA, cDNA, RNA or mRNA. In some embodiments, the nucleic acid amplification can be linear or exponential. In some embodiments, the specific variants or mutations may be detected by the use of amplification methods with the aid of oligonucleotide primers or probes designed to interact with or hybridize to a particular target sequence in a specific manner, thus amplifying only the target variant.

Non-limiting examples of nucleic acid amplification techniques include polymerase chain reaction (PCR), reverse transcriptase polymerase chain reaction (RT-PCR), nested PCR, ligase chain reaction (see Abravaya, K. et al., Nucleic Acids Res. (1995), 23:675-682), branched DNA signal amplification (see Urdea, M. S. et al., AIDS (1993), 7(suppl 2):S11-S14), amplifiable RNA reporters, Q-beta replication, transcription-based amplification, boomerang DNA amplification, strand displacement activation, cycling probe technology, isothermal nucleic acid sequence based amplification (NASBA) (see Kievits, T. et al., J Virological Methods (1991), 35:273-286), Invader Technology, next-generation sequencing technology or other sequence replication assays or signal amplification assays.

In some embodiments, DNA and/or RNA may be detected by next-generation sequencing (NGS). Next-generation sequencing includes any sequencing method that determines the nucleotide sequence of either individual nucleic acid molecules or clonally expanded proxies for individual nucleic acid molecules in a highly parallel fashion (*e.g*., greater than 10⁵ molecules are sequenced simultaneously). In one embodiment, the relative abundance of the nucleic acid species in the library can be estimated by counting the relative number of occurrences of their cognate sequences in the data generated by the sequencing experiment. Next generation sequencing methods are known in the art, and are described, *e.g.*, in Metzker, M. (2010) Nature Biotechnology Reviews 11:31-46, incorporated herein by reference. In one embodiment, the next-generation sequencing allows for the determination of the nucleotide sequence of an individual nucleic acid molecule (*e.g*., Helicos BioSciences' HeliScope Gene Sequencing system, and Pacific Biosciences' PacBio RS system). In other embodiments, the sequencing method determines the nucleotide sequence of clonally expanded proxies for individual nucleic acid molecules (*e.g.*, the Solexa sequencer, Illumina Inc., San Diego, Calif; 454 Life Sciences (Branford, Conn.), and Ion Torrent), *e.g*., massively parallel short-read sequencing (*e.g*., the Solexa sequencer, Illumina Inc., San Diego, Calif.), which generates more bases of sequence per sequencing unit than other sequencing methods that generate fewer but longer reads. Other methods or machines for next-generation sequencing include, e.g., the sequencers provided by 454 Life Sciences (Branford, Conn.), Applied Biosystems (Foster City, Calif.; SOLiD sequencer), and Helicos BioSciences Corporation (Cambridge, Mass.). Platforms for next-generation sequencing include, e.g., Roche/454's Genome Sequencer (GS) FLX System, Illumina/Solexa's Genome Analyzer (GA), Illumina's HiSeq 2500, HiSeq 3000, HiSeq 4000 and NovaSeq 6000 Sequencing Systems, Life/APG's Support Oligonucleotide Ligation Detection (SOLiD) system, Polonator's G.007 system, Helicos BioSciences' HeliScope Gene Sequencing system, and Pacific Biosciences' PacBio RS system. NGS technologies can include one or more of steps, e.g., template preparation, sequencing and imaging, and data analysis.

### Cancer Immunotherapies

Certain aspects of the present disclosure relate to cancer immunotherapies. In some embodiments, the cancer immunotherapy comprises one or more of: a checkpoint inhibitor, cancer vaccine, cell-based therapy, T cell receptor (TCR)-based therapy, adjuvant immunotherapy, cytokine immunotherapy, and oncolytic virus therapy. In some embodiments, the cancer immunotherapy comprises small molecule, nucleic acid, polypeptide, carbohydrate, toxin, cell-based, or binding agent therapeutic agent. Examples of cancer immunotherapies are described in greater detail *infra* but are not intended to be limiting. In some embodiments, the cancer immunotherapy activates one or more aspects of the immune system to attack a cell (*e.g.*, a tumor cell) that expresses a neoantigen of the present disclosure. The cancer immunotherapies of the present disclosure are contemplated for use as monotherapies, or in combination approaches comprising two or more in any combination or number, subject to medical judgement. Any of the cancer immunotherapies (optionally as monotherapies or in combination with another cancer immunotherapy or other therapeutic agent described herein) may find use in any of the methods described herein.

In some embodiments, the cancer immunotherapy comprises a cancer vaccine. A range of cancer vaccines have been tested that employ different approaches to promoting an immune response against the tumor (see, e.g., Emens L A, Expert Opin Emerg Drugs 13(2): 295-308 (2008) and US20190367613). Approaches have been designed to enhance the response of B cells, T cells, or professional antigen-presenting cells against tumors. Exemplary types of cancer vaccines include, but are not limited to, DNA-based vaccines, RNA-based vaccines, virus transduced vaccines, peptide-based vaccines, dendritic cell vaccines, oncolytic viruses, whole tumor cell vaccines, tumor antigen vaccines, etc. In some embodiments, the cancer vaccine can be prophylactic or therapeutic. In some embodiments, the cancer vaccine is formulated as a peptide-based vaccine, a nucleic acid- based vaccine, an antibody based vaccine, or a cell based vaccine. For example, a vaccine composition can include naked cDNA in cationic lipid formulations; lipopeptides (e.g., Vitiello, A. et ah, J. Clin. Invest. 95:341, 1995), naked cDNA or peptides, encapsulated e.g., in poly(DL-lactide-co-glycolide) ("PLG") microspheres (see, e.g., Eldridge, et ah, Molec. Immunol. 28:287-294, 1991: Alonso et al, Vaccine 12:299- 306, 1994; Jones et al, Vaccine 13:675-681, 1995); peptide composition contained in immune stimulating complexes (ISCOMS) (e.g., Takahashi et al, Nature 344:873-875, 1990; Hu et al, Clin. Exp. Immunol. 113:235-243, 1998); or multiple antigen peptide systems (MAPs) (see e.g., Tam, J. P., Proc. Natl Acad. Sci. U.S.A. 85:5409-5413, 1988; Tam, J.P., J. Immunol. Methods 196: 17-32, 1996). In some embodiments, a cancer vaccine is formulated as a peptide-based vaccine, or nucleic acid based vaccine in which the nucleic acid encodes the polypeptides. In some embodiments, a cancer vaccine is formulated as an antibody based vaccine. In some embodiments, a cancer vaccine is formulated as a cell based vaccine. In some embodiments, the cancer vaccine is a peptide cancer vaccine, which in some embodiments is a personalized peptide vaccine. In some embodiments, the cancer vaccine is a multivalent long peptide, a multiple peptide, a peptide mixture, a hybrid peptide, or a peptide pulsed dendritic cell vaccine (see, e.g., Yamada et al, Cancer Sci, 1 04: 14-21), 2013). In some embodiments, such cancer vaccines augment the anti-tumor response.

In some embodiments, the cancer vaccine comprises a polynucleotide that encodes a neoantigen of the present disclosure, *e.g*., SEQ ID NO:1 or 2, or 5, 6, 7, 8, or 9 contiguous amino acids therefrom. In some embodiments, the cancer vaccine comprises DNA that encodes a neoantigen of the present disclosure, *e.g*., SEQ ID NO:1 or 2, or 5, 6, 7, 8, or 9 contiguous amino acids therefrom. In some embodiments, the cancer vaccine comprises RNA that encodes a neoantigen of the present disclosure, *e.g*., SEQ ID NO:1 or 2, or 5, 6, 7, 8, or 9 contiguous amino acids therefrom. In some embodiments, the cancer vaccine comprises a polynucleotide that encodes a neoantigen of the present disclosure as well as one or more additional antigens, neoantigens, or other sequences that promote antigen presentation and/or an immune response. In some embodiments, the polynucleotide is complexed with one or more additional agents, such as a liposome or lipoplex. In some embodiments, the polynucleotide(s) are taken up and translated by antigen presenting cells (APCs), which then present a neoantigen of the present disclosure via MHC class I on the APC cell surface.

In some embodiments, the cancer vaccine comprises a neoantigen of the present disclosure, *e.g.*, SEQ ID NO:1 or 2, or *5*, 6, 7, 8, or 9 contiguous amino acids therefrom. In some embodiments, the cancer vaccine comprises a neoantigen of the present disclosure as well as one or more additional antigens, neoantigens, or other sequences that promote antigen presentation and/or an immune response.

In some embodiments, the cancer vaccine is selected from sipuleucel-T (Provenge^{®}, Dendreon/Valeant Pharmaceuticals), which has been approved for treatment of asymptomatic, or minimally symptomatic metastatic castrate-resistant (hormone-refractory) prostate cancer; and talimogene laherparepvec (Imlygic^{®}, BioVex/Amgen, previously known as T-VEC), a genetically modified oncolytic viral therapy approved for treatment of unresectable cutaneous, subcutaneous and nodal lesions in melanoma. In some embodiments, the cancer vaccine is selected from an oncolytic viral therapy such as pexastimogene devacirepvec (PexaVec/JX-594, SillaJen/formerly Jennerex Biotherapeutics), a thymidine kinase- (TK-) deficient vaccinia virus engineered to express GM-CSF, for hepatocellular carcinoma (NCT02562755) and melanoma (NCT00429312); pelareorep (Reolysin^{®}, Oncolytics Biotech), a variant of respiratory enteric orphan virus (reovirus) which does not replicate in cells that are not RAS-activated, in numerous cancers, including colorectal cancer (NCT01622543); prostate cancer (NCT01619813); head and neck squamous cell cancer (NCT01166542); pancreatic adenocarcinoma (NCT00998322); and non-small cell lung cancer (NSCLC) (NCT 00861627); enadenotucirev (NG-348, PsiOxus, formerly known as ColoAdl), an adenovirus engineered to express a full length CD80 and an antibody fragment specific for the T-cell receptor CD3 protein, in ovarian cancer (NCT02028117); metastatic or advanced epithelial tumors such as in colorectal cancer, bladder cancer, head and neck squamous cell carcinoma and salivary gland cancer (NCT02636036); ONCOS-102 (Targovax/formerly Oncos), an adenovirus engineered to express GM-CSF, in melanoma (NCT03003676); and peritoneal disease, colorectal cancer or ovarian cancer (NCT02963831); GL-ONC1 (GLV-1h68/GLV-1h153, Genelux GmbH), vaccinia viruses engineered to express beta-galactosidase (beta-gal)/beta-glucoronidase or beta-gal/human sodium iodide symporter (hNIS), respectively, were studied in peritoneal carcinomatosis (NCT01443260); fallopian tube cancer, ovarian cancer (NCT 02759588); or CG0070 (Cold Genesys), an adenovirus engineered to express GM-CSF, in bladder cancer (NCT02365818); anti-gp100; STINGVAX; GVAX; DCVaxL; and DNX-2401. In some embodiments, the cancer vaccine is selected from JX-929 (SillaJen/formerly Jennerex Biotherapeutics), a TK- and vaccinia growth factor-deficient vaccinia virus engineered to express cytosine deaminase, which is able to convert the prodrug 5-fluorocytosine to the cytotoxic drug 5-fluorouracil; TGO1 and TG02 (Targovax/formerly Oncos), peptide-based immunotherapy agents targeted for difficult-to-treat RAS mutations; and TILT-123 (TILT Biotherapeutics), an engineered adenovirus designated: Ad5/3-E2F-delta24-hTNFα-IRES-hIL20; and VSV-GP (ViraTherapeutics) a vesicular stomatitis virus (VSV) engineered to express the glycoprotein (GP) of lymphocytic choriomeningitis virus (LCMV), which can be further engineered to express antigens designed to raise an antigen-specific CD8⁺ T cell response. In some embodiments, the cancer vaccine comprises a vector-based tumor antigen vaccine. Vector-based tumor antigen vaccines can be used as a way to provide a steady supply of antigens to stimulate an anti-tumor immune response. In some embodiments, vectors encoding for tumor antigens are injected into the patient (possibly with proinflammatory or other attractants such as GM-CSF), taken up by cells in vivo to make the specific antigens, which would then provoke the desired immune response. In some embodiments, vectors may be used to deliver more than one tumor antigen at a time, to increase the immune response. In addition, recombinant virus, bacteria or yeast vectors should trigger their own immune responses, which may also enhance the overall immune response.

In some embodiments, the cancer vaccine comprises a DNA-based vaccine. In some embodiments, DNA-based vaccines can be employed to stimulate an anti-tumor response. The ability of directly injected DNA, that encodes an antigenic protein, to elicit a protective immune response has been demonstrated in numerous experimental systems. Vaccination through directly injecting DNA, that encodes an antigenic protein, to elicit a protective immune response often produces both cell-mediated and humoral responses. Moreover, reproducible immune responses to DNA encoding various antigens have been reported in mice that last essentially for the lifetime of the animal (see, e.g., Yankauckas et al. (1993) DNA Cell Biol., 12: 771-776). In some embodiments, plasmid (or other vector) DNA that includes a sequence encoding a protein operably linked to regulatory elements required for gene expression is administered to individuals (e.g. human patients, non-human mammals, etc.). In some embodiments, the cells of the individual take up the administered DNA and the coding sequence is expressed. In some embodiments, the antigen so produced becomes a target against which an immune response is directed.

In some embodiments, the cancer vaccine comprises an RNA-based vaccine. In some embodiments, RNA-based vaccines can be employed to stimulate an anti-tumor response. In some embodiments, RNA-based vaccines comprise a self-replicating RNA molecule. In some embodiments, the self-replicating RNA molecule may be an alphavirus-derived RNA replicon. Self-replicating RNA (or "SAM") molecules are well known in the art and can be produced by using replication elements derived from, e.g., alphaviruses, and substituting the structural viral proteins with a nucleotide sequence encoding a protein of interest. A self-replicating RNA molecule is typically a +-strand molecule which can be directly translated after delivery to a cell, and this translation provides a RNA-dependent RNA polymerase which then produces both antisense and sense transcripts from the delivered RNA. Thus, the delivered RNA leads to the production of multiple daughter RNAs. These daughter RNAs, as well as collinear subgenomic transcripts, may be translated themselves to provide in situ expression of an encoded polypeptide (i.e. comprising HPV antigens), or may be transcribed to provide further transcripts with the same sense as the delivered RNA which are translated to provide in situ expression of the antigen.

In some embodiments, the cancer immunotherapy comprises a cell-based therapy. In some embodiments, the cancer immunotherapy comprises a T cell-based therapy. In some embodiments, the cancer immunotherapy comprises an adoptive therapy, e.g., an adoptive T cell-based therapy. In some embodiments, the T cells are autologous or allogeneic to the recipient. In some embodiments, the T cells are CD8+ T cells. In some embodiments, the T cells are CD4+ T cells. Adoptive immunotherapy refers to a therapeutic approach for treating cancer or infectious diseases in which immune cells are administered to a host with the aim that the cells mediate either directly or indirectly specific immunity to (i.e., mount an immune response directed against) tumor cells. In some embodiments, the immune response results in inhibition of tumor and/or metastatic cell growth and/or proliferation and in related embodiments results in neoplastic cell death and/or resorption. The immune cells can be derived from a different organism/host (exogenous immune cells) or can be cells obtained from the subject organism (autologous immune cells). In some embodiments the immune cells (e.g., autologous or allogeneic T cells (e.g., regulatory T cells, CD4+ T cells, CD8+ T cells, or gamma-delta T cells), NK cells, invariant NK cells, or NKT cells) can be genetically engineered to express antigen receptors such as engineered TCRs and/or chimeric antigen receptors (CARs). For example, the host cells (e.g., autologous or allogeneic T-cells) are modified to express a T cell receptor (TCR) having antigenic specificity for a cancer antigen. In some embodiments, NK cells are engineered to express a TCR. The NK cells may be further engineered to express a CAR. Multiple CARs and/or TCRs, such as to different antigens, may be added to a single cell type, such as T cells or NK cells. In some embodiments, the cells comprise one or more nucleic acids/expression constructs/vectors introduced via genetic engineering that encode one or more antigen receptors, and genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature (e.g. chimeric). In some embodiments, the population of immune cells can be obtained from a subject in need of therapy or suffering from a disease associated with reduced immune cell activity. Thus, the cells will be autologous to the subject in need of therapy. In some embodiments, the population of immune cells can be obtained from a donor, such as a histocompatibility matched donor. In some embodiments, the immune cell population can be harvested from the peripheral blood, cord blood, bone marrow, spleen, or any other organ/tissue in which immune cells reside in said subject or donor. In some embodiments, the immune cells can be isolated from a pool of subjects and/or donors, such as from pooled cord blood. In some embodiments, when the population of immune cells is obtained from a donor distinct from the subject, the donor may be allogeneic, provided the cells obtained are subject- compatible in that they can be introduced into the subject. In some embodiments, allogeneic donor cells may or may not be human-leukocyte-antigen (HLA)-compatible. In some embodiments, to be rendered subject-compatible, allogeneic cells can be treated to reduce immunogenicity.

In some embodiments, the cell-based therapy comprises a T cell-based therapy. Several basic approaches for the derivation, activation and expansion of functional anti-tumor effector cells have been described in the last two decades. These include: autologous cells, such as tumor-infiltrating lymphocytes (TILs); T cells activated ex-vivo using autologous DCs, lymphocytes, artificial antigen-presenting cells (APCs) or beads coated with T cell ligands and activating antibodies, or cells isolated by virtue of capturing target cell membrane; allogeneic cells naturally expressing anti-host tumor T cell receptor (TCR); and non-tumor-specific autologous or allogeneic cells genetically reprogrammed or "redirected" to express tumor-reactive TCR or chimeric TCR molecules displaying antibody-like tumor recognition capacity known as "T-bodies". In some embodiments, the T cells are derived from the blood, bone marrow, lymph, umbilical cord, or lymphoid organs. In some aspects, the cells are human cells. In some embodiments, the cells are primary cells, such as those isolated directly from a subject and/or isolated from a subject and frozen. In some embodiments, the cells include one or more subsets of T cells or other cell types, such as whole T cell populations, CD4⁺ cells, CD8⁻ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. In some embodiments, the cells may be allogeneic and/or autologous. In some embodiments, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, as described herein, and reintroducing them into the same patient, before or after cryopreservation. In some embodiments, the sub-types and subpopulations of T cells (e.g. ., CD4⁺ and/or CD8⁺ T cells) are naive T (TN) cells, effector T cells (TEFF), memory T cells and sub-types thereof, such as stem cell memory T (TSCM), central memory T (TCM), effector memory T (TEM), or terminally differentiated effector memory T cells, tumor-infiltrating lymphocytes (TTL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells. In some embodiments, one or more of the T cell populations is enriched for or depleted of cells that are positive for a specific marker, such as surface markers, or that are negative for a specific marker. In some embodiments, such markers are those that are absent or expressed at relatively low levels on certain populations of T cells (e.g., non-memory cells) but are present or expressed at relatively higher levels on certain other populations of T cells (e.g., memory cells). In some embodiments, T cells are separated from a PBMC sample by negative selection of markers expressed on non-T cells, such as B cells, monocytes, or other white blood cells, such as CD 14. In some embodiments, a CD4⁺ or CD8⁺ selection step is used to separate CD4⁺ helper and CD8⁺ cytotoxic T cells. Such CD4⁺ and CD8⁺ populations can be further sorted into subpopulations by positive or negative selection for markers expressed or expressed to a relatively higher degree on one or more naive, memory, and/or effector T cell subpopulations. In some embodiments, CD8⁺ T cells are further enriched for or depleted of naive, central memory, effector memory, and/or central memory stem cells, such as by positive or negative selection based on surface antigens associated with the respective subpopulation. In some embodiments, the T cells are autologous T cells. In this method, tumor samples are obtained from patients and a single cell suspension is obtained. The single cell suspension can be obtained in any suitable manner, e.g., mechanically (disaggregating the tumor using, e.g., a gentleMACS^{™} Dissociator, Miltenyi Biotec, Auburn, Calif.) or enzymatically (e.g., collagenase or DNase). Single-cell suspensions of tumor enzymatic digests are cultured in interleukin-2 (IL-2). The cells are cultured until confluence (e.g., about 2x 10⁶ lymphocytes), e.g., from about 5 to about 21 days, such as from about 10 to about 14 days.

In some embodiments, the cultured T cells can be pooled and rapidly expanded. Rapid expansion provides an increase in the number of antigen-specific T-cells, *e.g.*, of at least about 50- fold (e.g., 50-, 60-, 70-, 80-, 90-, or 100-fold, or greater) over a period of about 10 to about 14 days. In some embodiments, expansion can be accomplished by any of a number of methods as are known in the art. For example, T cells can be rapidly expanded using non-specific T-cell receptor stimulation in the presence of feeder lymphocytes and either interleukin-2 (IL-2) or interleukin- 15 (IL-15), with IL-2 being particularly contemplated. The non-specific T-cell receptor stimulus can include around 30 ng/ml of OKT3, a mouse monoclonal anti-CD3 antibody (available from Ortho-McNeil^{®}, Raritan, N.J.). In some embodiments, T cells can be rapidly expanded by stimulation of peripheral blood mononuclear cells (PBMC) in vitro with one or more antigens (including antigenic portions thereof, such as epitope(s), or a cell) of the cancer, which can be optionally expressed from a vector, such as a human leukocyte antigen A2 (HLA-A2) binding peptide, in the presence of a T-cell growth factor, such as 300 IU/ml IL-2 or IL- 15, with IL-2 being contemplated. The in vv/ro- induced T-cells are rapidly expanded by re stimulation with the same antigen(s) of the cancer pulsed onto HLA-A2-expressing antigen-presenting cells. In some embodiments, the T cells can be re-stimulated with irradiated, autologous lymphocytes or with irradiated HLA-A2+ allogeneic lymphocytes and IL-2, for example. In some embodiments, the autologous T-cells can be modified to express a T-cell growth factor that promotes the growth and activation of the autologous T-cells. In some embodiments, suitable T-cell growth factors include, for example, interleukin (IL)-2, IL-7, IL-15, and IL-12. Suitable methods of modification are known in the art. See, for instance, Sambrook et al, MOLECULAR CLONING: A LABORATORY MANUAL, 3rd ed., Cold Spring Harbor Press, Cold Spring Harbor, N.Y. 2001; and Ausubel et al, CURRENT PROTOCOLS IN MOLECULAR BIOLOGY, Greene Publishing Associates and John Wiley & Sons, NY, 1994. In some embodiments, modified autologous T- cells express the T-cell growth factor at high levels. T-cell growth factor coding sequences, such as that of IL-12, are readily available in the art, as are promoters, the operable linkage of which to a T-cell growth factor coding sequence promote high-level expression. In some embodiments, autologous T cells may be engineered to express a defined T cell receptor (TCR) that are directed against target TAAs, either wild-type TCR, or mutated/engineered TCR towards a higher affinity to the antigen peptide/MHC molecule complexes. In some embodiments, autologous T cells may be engineered to express a CAR, *e.g.*, as described *infra.*

In some embodiments, the T cell-based therapy comprises a chimeric antigen receptor (CAR)-T-based therapy. This approach involves engineering a CAR that specifically binds to an antigen of interest and comprises one or more intracellular signaling domains for T cell activation. The CAR is then expressed on the surface of engineered T cells (CAR-T) and administered to a patient, leading to a T-cell-specific immune response against cancer cells expressing the antigen. In some embodiments, the CAR specifically binds a neoantigen of the present disclosure.

In some embodiments, the T cell-based therapy comprises T cells expressing a recombinant T cell receptor (TCR). This approach involves identifying a TCR that specifically binds to an antigen of interest, which is then used to replace the endogenous or native TCR on the surface of engineered T cells that are administered to a patient, leading to a T-cell-specific immune response against cancer cells expressing the antigen. In some embodiments, the recombinant TCR specifically binds a neoantigen of the present disclosure.

In some embodiments, the T cell-based therapy comprises tumor-infiltrating lymphocytes (TILs). For example, TILs can be isolated from a tumor or cancer of the present disclosure, then isolated and expanded *in vitro.* Some or all of these TILs may specifically recognize a neoantigen of the present disclosure. In some embodiments, the TILs are exposed to one or more neoantigens of the present disclosure *in vitro* after isolation. TILs are then administered to the patient (optionally in combination with one or more cytokines or other immune-stimulating substances).

In some embodiments, the cell-based therapy comprises a natural killer (NK) cell-based therapy. Natural killer (NK) cells are a subpopulation of lymphocytes that have spontaneous cytotoxicity against a variety of tumor cells, virus-infected cells, and some normal cells in the bone marrow and thymus. NK cells are critical effectors of the early innate immune response toward transformed and virus-infected cells. NK cells constitute about 10% of the lymphocytes in human peripheral blood. When lymphocytes are cultured in the presence of interleukin 2 (IL-2), strong cytotoxic reactivity develops. NK cells are effector cells known as large granular lymphocytes because of their larger size and the presence of characteristic azurophilic granules in their cytoplasm. NK cells differentiate and mature in the bone marrow, lymph nodes, spleen, tonsils, and thymus. NK cells can be detected by specific surface markers, such as CD 16, CD56, and CD8 in humans. NK cells do not express T-cell antigen receptors, the pan T marker CD3, or surface immunoglobulin B cell receptors. In some embodiments, NK cells are derived from human peripheral blood mononuclear cells (PBMC), unstimulated leukapheresis products (PBSC), human embryonic stem cells (hESCs), induced pluripotent stem cells (iPSCs), bone marrow, or umbilical cord blood by methods well known in the art. In some embodiments, umbilical CB is used to derive NK cells. In some embodiments, the NK cells are isolated and expanded by the previously described method of ex vivo expansion of NK cells (Spanholtz et al, 2011; Shah et al, 2013). In some embodiments, CB mononuclear cells are isolated by ficoll density gradient centrifugation and cultured in a bioreactor with IL-2 and artificial antigen presenting cells (aAPCs). After 7 days, the cell culture is depleted of any cells expressing CD3 and re-cultured for an additional 7 days. The cells are again CD3-depleted and characterized to determine the percentage of CD56⁺/CD3 cells or NK cells. In some embodiments, umbilical CB is used to derive NK cells by the isolation of CD34⁺ cells and differentiation into CD56⁺/CD3 cells by culturing in medium contain SCF, IL-7, IL-15, and IL-2.

In some embodiments, the cell-based therapy comprises a dendritic cell-based therapy, *e.g.*, a dendritic cell vaccine. In some embodiments, the DC vaccine comprises antigen-presenting cells that are able to induce specific T cell immunity, which are harvested from the patient or from a donor. In some embodiments, the DC vaccine can then be exposed in vitro to a peptide antigen, for which T cells are to be generated in the patient. In some embodiments, dendritic cells loaded with the antigen are then injected back into the patient. In some embodiments, immunization may be repeated multiple times if desired. Methods for harvesting, expanding, and administering dendritic cells are known in the art; *see*, *e.g.*, WO2019178081. Dendritic cell vaccines (such as Sipuleucel-T, also known as APC8015 and PROVENGE^{®}) are vaccines that involve administration of dendritic cells that act as APCs to present one or more cancer-specific antigens, *e.g*., a neoantigen of the present disclosure, to the patient's immune system. In some embodiments, the vaccine comprises dendritic cells that have been exposed to one or more neoantigens of the present disclosure. In some embodiments, the vaccine comprises dendritic cells that present one or more neoantigens of the present disclosure, *e.g*., via MHC class I. In some embodiments, the dendritic cells are autologous or allogeneic to the recipient.

In some embodiments, the cancer immunotherapy comprises a TCR-based therapy. In some embodiments, the cancer immunotherapy comprises administration of one or more TCRs or TCR-based biologics that specifically bind a neoantigen of the present disclosure. For example, the TCR-based therapeutic may comprise a TCR or extracellular portion thereof that specifically binds a neoantigen of the present disclosure (*e.g*., as presented on a cell surface via MHC class I) as well as a moiety that binds an immune cell (*e.g.*, a T cell), such as an antibody or antibody fragment that specifically binds a T cell surface protein or receptor (*e.g*., an anti-CD3 antibody or antibody fragment).

In some embodiments, the cancer immunotherapy comprises adjuvant immunotherapy. Adjuvant immunotherapy comprises the use of one or more agents that activate components of the innate immune system, *e.g*., HILTONOL^{®} (imiquimod), which targets the TLR7 pathway.

In some embodiments, the cancer immunotherapy comprises cytokine immunotherapy. Cytokine immunotherapy comprises the use of one or more cytokines that activate components of the immune system. Examples include, but are not limited to, aldesleukin (PROLEUKIN^{®}; interleukin-2), interferon alfa-2a (ROFERON^{®}-A), interferon alfa-2b (INTRON^{®}-A), and peginterferon alfa-2b (PEGINTRON^{®}).

In some embodiments, the cancer immunotherapy comprises oncolytic virus therapy. Oncolytic virus therapy uses genetically modified viruses to replicate in and kill cancer cells, leading to the release of antigens (*e.g*., a neoantigen of the present disclosure) that stimulate an immune response. In some embodiments, replication-competent oncolytic viruses expressing a tumor antigen comprise any naturally occurring (e.g. from a "field source") or modified replication-competent oncolytic virus. In some embodiments, the oncolytic virus, in addition to expressing a tumor antigen, may be modified to increase selectivity of the virus for cancer cell. In some embodiments, replication-competent oncolytic viruses include, but are not limited to, oncolytic viruses that are a member in the family of myoviridae, siphoviridae, podpviridae, teciviridae, corticoviridae, plasmaviridae, lipothrixviridae, fuselloviridae, poxyiridae, iridoviridae, phycodnaviridae, baculoviridae, herpesviridae, adnoviridae, papovaviridae, polydnaviridae, inoviridae, microviridae, geminiviridae, circoviridae, parvoviridae, hcpadnaviridae, retroviridae, cyctoviridae, reoviridae, birnaviridae, paramyxoviridae, rhabdoviridae, filoviridae, orthomyxoviridae, bunyaviridae, arenaviridae, Leviviridae, picornaviridae, sequiviridae, comoviridae, potyviridae, caliciviridae, astroviridae, nodaviridae, tetraviridae, tombusviridae, coronaviridae, glaviviridae, togaviridae, and barnaviridae. In some embodiments, replication-competent oncolytic viruses include adenovirus, retrovirus, reovirus, rhabdovirus, Newcastle Disease virus (NDV), polyoma virus, vaccinia virus (VacV), herpes simplex virus, picornavirus, coxsackie virus and parvovirus. In some embodiments, the replicative oncolytic vaccinia virus expressing a tumor antigen may be engineered to lack one or more functional genes in order to increase the cancer selectivity of the virus. In some embodiments, the oncolytic vaccinia virus is engineered to lack thymidine kinase (TK) activity. In some embodiments, the oncolytic vaccinia virus may be engineered to lack vaccinia virus growth factor (VGF). In some embodiments, the oncolytic vaccinia virus may be engineered to lack both VFG and TK activity. In some embodiments, the oncolytic vaccinia virus may be engineered to lack one or more genes involved in evading host interferon (IFN) response such as E3L, K3L, B18R, or B8R. In some embodiments, the replicative oncolytic vaccinia virus is a Western Reserve, Copenhagen, Lister or Wyeth strain and lacks a functional TK gene. In some embodiments, the oncolytic vaccinia virus is a Western Reserve, Copenhagen, Lister or Wyeth strain lacking a functional B18R and/or B8R gene. In some embodiments, a replicative oncolytic vaccinia virus expressing a tumor antigen of the combination may be locally or systemically administered to a subject, e.g. via intratumoral, intraperitoneal, intravenous, intra-arterial, intramuscular, intradermal, intracranial, subcutaneous, or intranasal administration.

In some embodiments, the cancer immunotherapy comprises a checkpoint inhibitor. As is known in the art, a checkpoint inhibitor targets at least one immune checkpoint protein to alter the regulation of an immune response, e.g., down-modulating or inhibiting an immune response. Immune checkpoint proteins include, *e.g*., CTLA4, PD-L1, PD-1, PD-L2, VISTA, B7-H2, B7-H3, B7-H4, B7-H6, 2B4, ICOS, HVEM. CEACAM, LAIR1, CD80, CD86, CD276, VTCN1, MHC class I, MHC class II, GALS, adenosine, TGFR, CSF1R, MICA/B, arginase, CD160, gp49B, PIR-B, KIR family receptors, TIM-1 , TIM-3, TIM-4, LAG-3, BTLA, SIRPalpha (CD47), CD48, 2B4 (CD244), B7.1 , B7.2, ILT-2, ILT-4, TIGIT, LAG-3, BTLA, IDO, OX40, and A2aR. In some embodiments, molecules involved in regulating immune checkpoints include, but are not limited to: PD-1 (CD279), PD-L1 (B7-H1, CD274), PD-L2 (B7-CD, CD273), CTLA-4 (CD152), HVEM, BTLA (CD272), a killer-cell immunoglobulin-like receptor (KIR), LAG-3 (CD223), TIM-3 (HAVCR2), CEACAM, CEACAM-1, CEACAM-3, CEACAM-5, GAL9, VISTA (PD-1H), TIGIT, LAIR1, CD160, 2B4, TGFRbeta, A2AR, GITR (CD357), CD80 (B7-1), CD86 (B7-2), CD276 (B7-H3), VTCNI (B7-H4), MHC class I, MHC class II, GALS, adenosine, TGFR, B7-H1, OX40 (CD134), CD94 (KLRD1), CD137 (4-1BB), CD137L (4-1BBL), CD40, IDO, CSF1R, CD40L, CD47, CD70 (CD27L), CD226, HHLA2, ICOS (CD278), ICOSL (CD275), LIGHT (TNFSF 14, CD258), NKG2a, NKG2d, OX40L (CD 134L), PVR (NECLS, CD155), SIRPa, MICA/B, and/or arginase. In some embodiments, a checkpoint inhibitor decreases the activity of a checkpoint protein that negatively regulates immune cell function, *e.g*.., in order to enhance T cell activation and/or an anti-cancer immune response; in other embodiments, a checkpoint inhibitor increases the activity of a checkpoint protein that positively regulates immune cell function, *e.g*.., in order to enhance T cell activation and/or an anti-cancer immune response. In some embodiments, the checkpoint inhibitor is an antibody. In some embodiments, the checkpoint inhibitor is an antibody. Examples of checkpoint inhibitors include, without limitation, a PD-L1 axis binding antagonist (e.g., an anti-PD-L1 antibody, e.g., atezolizumab (MPDL3280A)), an antagonist directed against a co-inhibitory molecule (e.g., a CTLA4 antagonist (e.g., an anti-CTLA4 antibody), a TIM-3 antagonist (e.g., an anti-TIM-3 antibody), or a LAG-3 antagonist (e.g.. an anti-LAG-3 antibody)), or any combination thereof. In some embodiments, the immune checkpoint inhibitors comprise drugs such as small molecules, recombinant forms of ligand or receptors, or, in particular, are antibodies, such as human antibodies (e.g., International Patent Publication WO2015016718; Pardoll, Nat Rev Cancer, 12(4): 252-64, 2012; both incorporated herein by reference). In some embodiments, known inhibitors of the immune checkpoint proteins or analogs thereof may be used, in particular chimerized, humanized or human forms of antibodies may be used.

In some embodiments, the checkpoint inhibitor is a PD-L1 axis binding antagonist, e.g., a PD-1 binding antagonist, a PD-L1 binding antagonist, or a PD-L2 binding antagonist. PD-1 (programmed death 1) is also referred to in the art as "programmed cell death 1 ," "PUCD1 ," "CD279," and "SLEB2." An exemplary human PD-1 is shown in UniProtKB/Swiss-Prot Accession No. Q15116. PD-L1 (programmed death ligand 1 ) is also referred to in the art as "programmed cell death 1 ligand 1," "PDCD1 LG1 ," "CD274," "B7-H," and "PDL1 ." An exemplary human PD-L1 is shown in UniProtKB/Swiss-Prot Accession No.Q9NZQ7.1 . PD-L2 (programmed death ligand 2) is also referred to in the art as "programmed cell death 1 ligand 2," "PDCD1 LG2," "CD273," "B7-DC," "Btdc," and "PDL2." An exemplary human PD-L2 is shown in UniProtKB/Swiss-Prot Accession No. Q9BQ51 . In some instances, PD-1 , PD-L1 , and PD-L2 are human PD-1 , PD-L1 and PD-L2.

In some instances, the PD-1 binding antagonist is a molecule that inhibits the binding of PD-1 to its ligand binding partners In a specific aspect the PD-1 ligand binding partners are PD-L1 and/or PD-L2 In another instance, a PD-L1 binding antagonist is a molecule that inhibits the binding of PD-L1 to its binding ligands. In a specific aspect, PD-L1 binding partners are PD-1 and/or B7-1 In another instance, the PD-L2 binding antagonist is a molecule that inhibits the binding of PD-L2 to its ligand binding partners. In a specific aspect, the PD-L2 binding ligand partner is PD-1. The antagonist may be an antibody, an antigen binding fragment thereof, an immunoadhesin, a fusion protein, or oligopeptide. In some embodiments, the PD-1 binding antagonist is a small molecule, a nucleic acid, a polypeptide (*e.g*., antibody), carbohydrate, a lipid, a metal, or a toxin.

In some instances, the PD-1 binding antagonist is an anti-PD-1 antibody (e g., a human antibody, a humanized antibody, or a chimeric antibody), for example, as described below In some instances, the anti-PD-1 antibody is selected from the group consisting of MDX-1 106 (nivolumab), MK-3475 (pembrolizumab), MEDI-0680 (AMP-514), PDR001, REGN2910, MGA-012, JNJ-63723283, BI 754091, and BGB-108. MDX-1 106, also known as MDX- 1 106-04, ONO-4538, BMS-936558, or nivolumab, is an anti-PD-1 antibody described in WO2006/121 168. MK-3475, also known as pembrolizumab or lambrolizumab, is an anti-PD-1 antibody described in WO 2009/1 14335. In some instances, the PD-1 binding antagonist is an immunoadhesin (e.g., an immunoadhesin comprising an extracellular or PD-1 binding portion of PD-L1 or PD-L2 fused to a constant region (e.g., an Fc region of an immunoglobulin sequence). In some instances, the PD-1 binding antagonist is AMP-224. AMP-224, also known as B7-DCIg, is a PD-L2-Fc fusion soluble receptor described in WO 2010/027827 and WO 2011 /066342.

In some embodiments, the anti-PD-1 antibody is nivolumab (CAS Registry Number: 946414-94-4). Nivolumab (Bristol-Myers Squibb/Ono), also known as MDX-1106-04, MDX-1106, ONO-4538, BMS-936558, and OPDIVO^{®}, is an anti-PD-1 antibody described in WO2006/121168. In some embodiments, the anti-PD-1 antibody comprises a heavy chain and a light chain sequence, wherein:
(a) the heavy chain sequence has at least 85%, at least 90%, at least 91 %, at least 92%, at least 93%, at least 94%, at least 95%, at least 96%, at least 97%, at least 98%, at least 99% or 100% sequence identity to the heavy chain sequence: 
QVQLVESGGGVVQPGRSLRLDCKASGITFSNSGMHWVRQAPGKGLEWVAVIWY
DGSKRYYADSVKGRFTISRDNSKNTLFLQMNSLRAEDTAVYYCATNDDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQ
EEMTKNQVSLTCLVKGFYPSDIAVEWESNGQPENNYKTTPPVLDSDGSFFLYSRLTVDK
SRWQEGNVFSCSVMHEALHNHYTQKSLSLSLG (SEQ ID NO:12), and
(b) the light chain sequences has at least 85%, at least 90%, at least 91 %, at least 92%, at least 93%, at least 94%, at least 95%, at least 96%, at least 97%, at least 98%, at least 99% or 100% sequence identity to the light chain sequence:

EIVLTQSPATLSLSPGERATLSCRASQSVSSYLAWYQQKPGQAPRLLIYDASNRAT
GIPARFSGSGSGTDFTLTISSLEPEDFAVYYCQQSSNWPRTFGQGTKVEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO:13).

In some embodiments, the anti-PD-1 antibody comprises the six HVR sequences from SEQ ID NO:12 and SEQ ID NO:13 (*e.g.*, the three heavy chain HVRs from SEQ ID NO:12 and the three light chain HVRs from SEQ ID NO:13). In some embodiments, the anti-PD-1 antibody comprises the heavy chain variable domain from SEQ ID NO:12 and the light chain variable domain from SEQ ID NO:13.

In some embodiments, the anti-PD-1 antibody is pembrolizumab (CAS Registry Number: 1374853-91-4). Pembrolizumab (Merck), also known as MK-3475, Merck 3475, lambrolizumab, KEYTRUDA^{®}, and SCH-900475, is an anti-PD-1 antibody described in WO2009/114335. In some embodiments, the anti-PD-1 antibody comprises a heavy chain and a light chain sequence, wherein:
(a) the heavy chain sequence has at least 85%, at least 90%, at least 91 %, at least 92%, at least 93%, at least 94%, at least 95%, at least 96%, at least 97%, at least 98%, at least 99% or 100% sequence identity to the heavy chain sequence: 
QVQLVQSGVEVKKPGASVKVSCKASGYTFTNYYMYWVRQAPGQGLEWMGG
INPSNGGTNFNEKFKNRVTLTTDSSTTTAYMELKSLQFDDTAVYYCARRDYRFDMGFD
YWGQGTTVTVSSASTKGPSVFPLAPCSRSTSESTAALGCLVKDYFPEPVTVSWNSGALT
SGVHTFPAVLQSSGLYSLSSVVTVPSSSLGTKTYTCNVDHKPSNTKVDKRVESKYGPPCP
PCPAPEFLGGPSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTISKAK
GQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSLG
(SEQ ID NO:14), and
(b) the light chain sequences has at least 85%, at least 90%, at least 91 %, at least 92%, at least 93%, at least 94%, at least 95%, at least 96%, at least 97%, at least 98%, at least 99% or 100% sequence identity to the light chain sequence:

EIVLTQSPAT LSLSPGERATLSCRASKGVSTSGYSYLHWYQQKPGQAPRLLIYLASYLES
GVPARFSGSGSGTDFTLTISSLEPEDFAVYYCQHSRDLPLTFGGGTKVEIKRTVAAPSV
F IFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC (SEQ ID
NO:15).

In some embodiments, the anti-PD-1 antibody comprises the six HVR sequences from SEQ ID NO: 14 and SEQ ID NO: 15 (*e.g.*, the three heavy chain HVRs from SEQ ID NO:14 and the three light chain HVRs from SEQ ID NO:15). In some embodiments, the anti-PD-1 antibody comprises the heavy chain variable domain from SEQ ID NO:14 and the light chain variable domain from SEQ ID NO:15.

Other examples of anti-PD-1 antibodies include, but are not limited to, MEDI-0680 (AMP-514; AstraZeneca), PDR001 (CAS Registry No. 1859072-53-9; Novartis), REGN2810 (LIBTAYO^{®} or cemiplimab-rwlc; Regeneron), BGB-108 (BeiGene), BGB-A317 (BeiGene), BI 754091, JS-001 (Shanghai Junshi), STI-A1110 (Sorrento), INCSHR-1210 (Incyte), PF-06801591 (Pfizer), TSR-042 (also known as ANB011; Tesaro/AnaptysBio), AM0001 (ARMO Biosciences), ENUM 244C8 (Enumeral Biomedical Holdings), ENUM 388D4 (Enumeral Biomedical Holdings). In some embodiments, the PD-1 axis binding antagonist comprises tislelizumab (BGB-A317), BGB-108, STI-A1110, AM0001, BI 754091, sintilimab (IBI308), cetrelimab (JNJ-63723283), toripalimab (JS-001), camrelizumab (SHR-1210, INCSHR-1210, HR-301210), MEDI-0680 (AMP-514), MGA-012 (INCMGA 0012), nivolumab (BMS-936558, MDX1106, ONO-4538), spartalizumab (PDR001), pembrolizumab (MK-3475, SCH 900475), PF-06801591, cemiplimab (REGN-2810, REGEN2810), dostarlimab (TSR-042, ANB011), FITC-YT-16 (PD-1 binding peptide), APL-501 or CBT-501 or genolimzumab (GB-226), AB-122, AK105, AMG 404, BCD-100, F520, HLX10, HX008, JTX-4014, LZM009, Sym021, PSB205, AMP-224 (fusion protein targeting PD-1), CX-188 (PD-1 probody), AGEN-2034, GLS-010, budigalimab (ABBV-181), AK-103, BAT-1306, CS-1003, AM-0001, TILT-123, BH-2922, BH-2941, BH-2950, ENUM-244C8, ENUM-388D4, HAB-21, H EISCOI 11-003, IKT-202, MCLA-134, MT-17000, PEGMP-7, PRS-332, RXI-762, STI-1110, VXM-10, XmAb-23104, AK-112, HLX-20, SSI-361, AT-16201, SNA-01, AB122, PD1-PIK, PF-06936308, RG-7769, CAB PD-1 Abs, AK-123, MEDI-3387, MEDI-5771, 4H1128Z-E27, REMD-288, SG-001, BY-24.3, CB-201, IBI-319, ONCR-177, Max-1, CS-4100, JBI-426, CCC-0701, CCX- 4503, or a derivative thereof. In some embodiments, the PD-1 binding antagonist is a peptide or small molecule compound. In some embodiments, the PD-1 binding antagonist is AUNP-12 (PierreFabre/Aurigene). In some embodiments, the PD-1 axis binding antagonist comprises a small molecule PD-1 axis binding antagonist described in Guzik et al., Molecules (2019) May 30;24(11). Other PD-1 inhibitors for use in the methods provided herein are known in the art such as described in U.S. Patent Nos. 8,735,553, 8,354,509, and 8,008,449.

In some embodiments, the PD-L1 binding antagonist is a small molecule that inhibits PD-1. In some embodiments, the PD-L1 binding antagonist is a small molecule that inhibits PD-L1. In some embodiments, the PD-L1 binding antagonist is a small molecule that inhibits PD-L1 and VISTA or PD-L1 and TIM3. In some embodiments, the PD-L1 binding antagonist is CA-170 (also known as AUPM-170). In any of the instances herein, the isolated anti-PD-L1 antibody can bind to a human PD-L1 , for example a human PD-L1 as shown in UniProtKB/Swiss-Prot Accession No.Q9NZQ7.1, or a variant thereof. In some embodiments, the PD-L1 binding antagonist is a small molecule, a nucleic acid, a polypeptide (*e.g*., antibody), carbohydrate, a lipid, a metal, or a toxin.

In some instances, the PD-L1 binding antagonist is an anti-PD-L1 antibody, for example, as described below. In some instances, the anti-PD-L1 antibody is capable of inhibiting binding between PD-L1 and PD-1 and/or between PD-L1 and B7-1 . In some instances, the anti-PD-L1 antibody is a monoclonal antibody. In some instances, the anti-PD-L1 antibody is an antibody fragment selected from the group consisting of Fab, Fab'-SH, Fv, scFv, and (Fab')2 fragments. In some instances, the anti-PD-L1 antibody is a humanized antibody. In some instances, the anti-PD-L1 antibody is a human antibody. In some instances, the anti-PD-L1 antibody is selected from the group consisting of YW243.55.S70, MPDL3280A (atezolizumab), MDX-1 105, and MEDI4736 (durvalumab), and MSB0010718C (avelumab). Antibody YW243 55.S70 is an anti-PD-L1 described in WO 2010/077634. MDX -1 105, also known as BMS-936559, is an anti-PD-L1 antibody described in WO2007/005874. MEDI4736 (durvalumab) is an anti-PD-L1 monoclonal antibody described in WO201 1 /066389 and US2013/034559. Examples of anti-PD-L1 antibodies useful for the methods of this disclosure, and methods for making thereof are described in PCT patent application WO 2010/077634, WO 2007/005874, WO 2011/066389, U.S. Pat. No. 8,217,149, and US2013/034559. In some embodiments, the PD-L1 axis binding antagonist comprises atezolizumab, avelumab, durvalumab (imfinzi), BGB-A333, SHR-1316 (HTI-1088), CK-301, BMS-936559, envafolimab (KN035, ASC22), CS1001, MDX-1105 (BMS-936559), LY3300054, STI-A1014, FAZ053, CX-072, INCB086550, GNS-1480, CA-170, CK-301, M-7824, HTI-1088 (HTI-131 , SHR-1316), MSB-2311, AK- 106, AVA-004, BBI-801, CA-327, CBA-0710, CBT-502, FPT-155, IKT-201, IKT-703, 10-103, JS-003, KD-033, KY-1003, MCLA-145, MT-5050, SNA-02, BCD-135, APL-502 (CBT-402 or TQB2450), IMC-001, KD-045, INBRX-105, KN-046, IMC-2102, IMC-2101, KD-005, IMM-2502, 89Zr-CX-072, 89Zr-DFO-6E11, KY-1055, MEDI-1109, MT-5594, SL-279252, DSP-106, Gensci-047, REMD-290, N-809, PRS-344, FS-222, GEN-1046, BH-29xx, FS-118, or a derivative thereof.

In some embodiments, the anti-PDL1 antibody comprises a heavy chain variable region and a light chain variable region, wherein:
(a) the heavy chain variable region comprises an HVR-H1, HVR-H2, and HVR-H3 sequence of GFTFSDSWIH (SEQ ID NO:16), AWISPYGGSTYYADSVKG (SEQ ID NO:17) and RHWPGGFDY (SEQ ID NO:18), respectively, and
(b) the light chain variable region comprises an HVR-L1, HVR-L2, and HVR-L3 sequence of RASQDVSTAVA (SEQ ID NO:19), SASFLYS (SEQ ID NO:20) and QQYLYHPAT (SEQ ID NO:21), respectively.

In some embodiments, the anti-PDL1 antibody is MPDL3280A, also known as atezolizumab and TECENTRIQ^{®} (CAS Registry Number: 1422185-06-5). In some embodiments, the anti-PDL1 antibody comprises a heavy chain and a light chain sequence, wherein:
(a) the heavy chain sequence has at least 85%, at least 90%, at least 91 %, at least 92%, at least 93%, at least 94%, at least 95%, at least 96%, at least 97%, at least 98%, at least 99% or 100% sequence identity to the heavy chain sequence: 


 (SEQ ID NO:22), and
(b) the light chain sequence has at least 85%, at least 90%, at least 91 %, at least 92%, at least 93%, at least 94%, at least 95%, at least 96%, at least 97%, at least 98%, at least 99% or 100% sequence identity to the light chain sequence:

DIQMTQSPSSLSASVGDRVTITCRASQDVSTAVAWYQQKPGKAPKLLIY SASF
LYSGVPSRFSGSGSGTDFTLTISSLQPEDFATYYCQQYLYHPATFGQGTKVEIKR (SEQ
ID NO:23).

In some embodiments, the anti-PDL1 antibody comprises a heavy chain and a light chain sequence, wherein:
(a) the heavy chain sequence has at least 85%, at least 90%, at least 91 %, at least 92%, at least 93%, at least 94%, at least 95%, at least 96%, at least 97%, at least 98%, at least 99% or 100% sequence identity to the heavy chain sequence: 
EVQLVESGGGLVQPGGSLRLSCAASGFTFSDSWIHWVRQAPGKGLEWVAWISPYGGST
YYADSVKGRFTISADTSKNTAYLQMNSLRAEDTAVYYCARRHWPGGFDYWGQGTLVT
 
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYASTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLYSKLT
VDKSRWQQGNVFSCSVMHEALHNHYTQKSLSLSPG (SEQ ID NO:24), and
(b) the light chain sequence has at least 85%, at least 90%, at least 91 %, at least 92%, at least 93%, at least 94%, at least 95%, at least 96%, at least 97%, at least 98%, at least 99% or 100% sequence identity to the light chain sequence:

DIQMTQSPSSLSASVGDRVTITCRASQDVSTAVAWYQQKPGKAPKLLIYSASFLYSGVPS
RFSGSGSGTDFTLTISSLQPEDFATYYCQQYLYHPATFGQGTKVEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC (SEQ ID NO:25).

In some instances, provided is an isolated anti-PD-L1 antibody comprising a heavy chain and a light chain sequence, wherein the light chain sequence has at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the amino acid sequence of SEQ ID NO:25. In some instances, provided is an isolated anti-PD-L1 antibody comprising a heavy chain and a light chain sequence, wherein the heavy chain sequence has at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the amino acid sequence of SEQ ID NO. 24. In some instances, provided is an isolated anti-PD-L1 antibody comprising a heavy chain and a light chain sequence, wherein the light chain sequence has at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the amino acid sequence of SEQ ID NO:25 and the heavy chain sequence has at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the amino acid sequence of SEQ ID NO:24.

In a still further specific aspect, the antibody further comprises a human or murine constant region. In a still further aspect, the human constant region is selected from the group consisting of lgG1 , lgG2, lgG2, IgG3. and lgG4. In a still further specific aspect, the human constant region is lgG1 . In a still further aspect, the murine constant region is selected from the group consisting of lgG1 . IgG2A, IgG2B, and lgG3. In a still further aspect, the murine constant region in lgG2A. In a still further specific aspect, the antibody has reduced or minimal effector function. In a still further specific aspect the minimal effector function results from an "effector-less Fc mutation" or aglycosylation. In still a further instance, the effector-less Fe mutation is an N297A or D265A/N297A substitution in the constant region.

In some instances, the isolated anti-PD-L1 antibody is aglycosylated. Glycosylation of antibodies is typically either N-linked or O-linked. N-linked refers to the attachment of the carbohydrate moiety to the side chain of an asparagine residue The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Removal of glycosylation sites form an antibody is conveniently accomplished by altering the amino acid sequence such that one of the above-described tripeptide sequences (for N-linked glycosylation sites) is removed The alteration may be made by substitution of an asparagine, serine or threonine residue within the glycosylation site another amino acid residue (e g., glycine, alanine or a conservative substitution).

In some embodiments, the anti-PDL1 antibody is avelumab (CAS Registry Number: 1537032-82-8). Avelumab, also known as MSB0010718C, is a human monoclonal IgG1 anti-PDL1 antibody (Merck KGaA, Pfizer). In some embodiments, the anti-PDL1 antibody comprises a heavy chain and a light chain sequence, wherein:
(a) the heavy chain sequence has at least 85%, at least 90%, at least 91 %, at least 92%, at least 93%, at least 94%, at least 95%, at least 96%, at least 97%, at least 98%, at least 99% or 100% sequence identity to the heavy chain sequence: EVQLLESGGGLVQPGGSLRLSCAASGFTFSSYIMMWVRQAPGKGLEWVSSIYPSGGITF 
YADTVKGRFTISRDNSKNTLYLQMNSLRAEDTAVYYCARIKLGTVTTVDYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPG (SEQ ID NO:26), and
(b) the light chain sequence has at least 85%, at least 90%, at least 91 %, at least 92%, at least 93%, at least 94%, at least 95%, at least 96%, at least 97%, at least 98%, at least 99% or 100% sequence identity to the light chain sequence:

QSALTQPASVSGSPGQSITISCTGTSSDVGGYNYVSWYQQHPGKAPKLMIYDVSNRPSG
VSNRFSGSKSGNTASLTISGLQAEDEADYYCSSYTSSSTRVFGTGTKVTVLGQPKANPTV
TLFPPSSEELQANKATLVCLISDFYPGAVTVAWKADGSPVKAGVETTKPSKQSNNKYAA
SSYLSLTPEQWKSHRSYSCQVTHEGSTVEKTVAPTECS (SEQ ID NO:27).

In some embodiments, the anti-PDL1 antibody comprises the six HVR sequences from SEQ ID NO:26 and SEQ ID NO:27 (*e.g.*, the three heavy chain HVRs from SEQ ID NO:26 and the three light chain HVRs from SEQ ID NO:27). In some embodiments, the anti-PDL1 antibody comprises the heavy chain variable domain from SEQ ID NO:26 and the light chain variable domain from SEQ ID NO:27.

In some embodiments, the anti-PDL1 antibody is durvalumab (CAS Registry Number: 1428935-60-7). Durvalumab, also known as MEDI4736, is an Fc optimized human monoclonal IgG1 kappa anti-PDL1 antibody (MedImmune, AstraZeneca) described in WO2011/066389 and US2013/034559. In some embodiments, the anti-PDL1 antibody comprises a heavy chain and a light chain sequence, wherein:
(a) the heavy chain sequence has at least 85%, at least 90%, at least 91 %, at least 92%, at least 93%, at least 94%, at least 95%, at least 96%, at least 97%, at least 98%, at least 99% or 100% sequence identity to the heavy chain sequence: 
EVQLVESGGGLVQPGGSLRLSCAASGFTFSRYWMSWVRQAPGKGLEWVANIKQDGSE
KYYVDSVKGRFTISRDNAKNSLYLQMNSLRAEDTAVYYCAREGGWFGELAFDYWGQG
TLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKRVEPKSCDKTHTCPPCP
 
APEFEGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPASIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO:28), and
(b) the light chain sequence has at least 85%, at least 90%, at least 91 %, at least 92%, at least 93%, at least 94%, at least 95%, at least 96%, at least 97%, at least 98%, at least 99% or 100% sequence identity to the light chain sequence:

EIVLTQSPGTLSLSPGERATLSCRASQRVSSSYLAWYQQKPGQAPRLLIYDASSRATGI
PDRFSGSGSGTDFTLTISRLEPEDFAVYYCQQYGSLPWTFGQGTKVEIKRTVAAPSVFI
FPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO:29).

In some embodiments, the anti-PDL1 antibody comprises the six HVR sequences from SEQ ID NO:28 and SEQ ID NO:29 (*e.g*., the three heavy chain HVRs from SEQ ID NO:28 and the three light chain HVRs from SEQ ID NO:29). In some embodiments, the anti-PDL1 antibody comprises the heavy chain variable domain from SEQ ID NO:28 and the light chain variable domain from SEQ ID NO:29.

Other examples of anti-PD-L1 antibodies include, but are not limited to, MDX-1 105 (BMS-936559; Bristol Myers Squibb), LY3300054 (Eli Lilly), STI-A1014 (Sorrento), KN035 (Suzhou Alphamab), FAZ053 (Novartis), or CX-072 (CytomX Therapeutics).

In some embodiments, the PD-L1 axis binding antagonist comprises small molecule PD-L1 axis binding antagonist GS-4224. In some embodiments, the PD-L1 axis binding antagonist comprises a small molecule PD-L1 axis binding antagonist described in PCT/US2019/017721.

In some embodiments, the checkpoint inhibitor is CT-011, also known as hBAT, hBAT-1 or pidilizumab, an antibody described in WO 2009/101611.

In some embodiments, the checkpoint inhibitor is an antagonist of CTLA4. In some embodiments, the checkpoint inhibitor is a small molecule antagonist of CTLA4. In some embodiments, the checkpoint inhibitor is an anti-CTLA4 antibody. CTLA4 is part of the CD28-B7 immunoglobulin superfamily of immune checkpoint molecules that acts to negatively regulate T cell activation, particularly CD28-dependent T cell responses. CTLA4 competes for binding to common ligands with CD28, such as CD80 (B7-1) and CD86 (B7-2), and binds to these ligands with higher affinity than CD28. Blocking CTLA4 activity (*e.g*., using an anti-CTLA4 antibody) is thought to enhance CD28-mediated costimulation (leading to increased T cell activation/priming), affect T cell development, and/or deplete Tregs (such as intratumoral Tregs). In some embodiments, the CTLA4 antagonist is a small molecule, a nucleic acid, a polypeptide (*e.g*., antibody), carbohydrate, a lipid, a metal, or a toxin.

In some embodiments, the anti-CTLA4 antibody is ipilimumab (YERVOY^{®}; CAS Registry Number: 477202-00-9). Ipilimumab, also known as BMS-734016, MDX-010, and MDX-101, is a fully human monoclonal IgG1 kappa anti-CTLA4 antibody (Bristol-Myers Squibb) described in WO2001/14424. In some embodiments, the anti-CTLA4 antibody comprises a heavy chain and a light chain sequence, wherein:
(a) the heavy chain sequence has at least 85%, at least 90%, at least 91 %, at least 92%, at least 93%, at least 94%, at least 95%, at least 96%, at least 97%, at least 98%, at least 99% or 100% sequence identity to the heavy chain sequence: 
QVQLVESGGGVVQPGRSLRLSCAASGFTFSSYTMHWVRQAPGKGLEWVTFISYDGNNK
YYADSVKGRFTISRDNSKNTLYLQMNSLRAEDTAIYYCARTGWLGPFDYWGQGTLVTV
SS (SEQ ID NO:30), and
(b) the light chain sequence has at least 85%, at least 90%, at least 91 %, at least 92%, at least 93%, at least 94%, at least 95%, at least 96%, at least 97%, at least 98%, at least 99% or 100% sequence identity to the light chain sequence:

EIVLTQSPGTLSLSPGERATLSCRASQSVGSSYLAWYQQKPGQAPRLLIYGAFSRATGIPD
RFSGSGSGTDFTLTISRLEPEDFAVYYCQQYGSSPWTFGQGTKVEIK (SEQ ID NO:31).

In some embodiments, the anti-CTLA4 antibody comprises the six HVR sequences from SEQ ID NO:30 and SEQ ID NO:31 (*e.g.,* the three heavy chain HVRs from SEQ ID NO:30 and the three light chain HVRs from SEQ ID NO:31). In some embodiments, the anti-CTLA4 antibody comprises the heavy chain variable domain from SEQ ID NO:30 and the light chain variable domain from SEQ ID NO:31.

Other examples of anti-CTLA4 antibodies include, but are not limited to, APL-509, AGEN1884, and CS1002. In some embodiments, the CTLA-4 inhibitor comprises ipilimumab (IB1310, BMS-734016, MDX010, MDX-CTLA4, MED14736), tremelimumab (CP-675, CP-675,206), APL-509, AGEN1884, and CS1002, AGEN1181, Abatacept (Orencia, BMS-188667, RG2077), BCD-145, ONC-392, ADU-1604, REGN4659, ADG116, KN044, KN046, or a derivative thereof.

In some embodiments, the immune checkpoint inhibitor comprises a LAG-3 inhibitor (e.g., an antibody, an antibody conjugate, or an antigen-binding fragment thereof). In some embodiments, the LAG-3 inhibitor comprises a small molecule, a nucleic acid, a polypeptide (e.g., an antibody), a carbohydrate, a lipid, a metal, or a toxin. In some embodiments, the LAG-3 inhibitor comprises a small molecule. In some embodiments, the LAG-3 inhibitor comprises a LAG-3 binding agent. In some embodiments, the LAG-3 inhibitor comprises an antibody, an antibody conjugate, or an antigen-binding fragment thereof. In some embodiments, the LAG-3 inhibitor comprises eftilagimod alpha (IMP321, IMP-321, EDDP-202, EOC-202), relatlimab (BMS-986016), GSK2831781 (IMP-731), LAG525 (IMP701), TSR-033, EVIP321 (soluble LAG-3 protein), BI 754111, IMP761, REGN3767, MK-4280, MGD-013, XmAb22841, INCAGN-2385, ENUM-006, AVA-017, AM-0003, iOnctura anti-LAG-3 antibody, Arcus Biosciences LAG-3 antibody, Sym022, a derivative thereof, or an antibody that competes with any of the preceeding.

In some embodiments, the immune checkpoint inhibitor is monovalent and/or monospecific. In some embodiments, the immune checkpoint inhibitor is multivalent and/or multispecific.

In some embodiments, the immunotherapy comprises an immunoregulatory molecule or cytokine. An immunoregulatory profile is required to trigger an efficient immune response and balance the immunity in a subject. In some embodiments, the immunoregulatory molecule is in included with any of the treatments detailed herein. Examples of suitable immunoregulatory cytokines include, but are not limited to, interferons (e.g., IFNα, IFNβ and IFN-γ), interleukins (e.g., IL-1, IL-2, IL-3, IL-4, IL-5, IL-6, IL-7, IL-8, IL-9, IL-10, IL-12 and IL-20), tumor necrosis factors (e.g., TNFα and TNFβ), erythropoietin (EPO), FLT-3 ligand, gIp10, TCA-3, MCP-1, MIF, MIP-1α, MIP-1β, Rantes, macrophage colony stimulating factor (M-CSF), granulocyte colony stimulating factor (G-CSF), and granulocyte-macrophage colony stimulating factor (GM-CSF), as well as functional fragments thereof. In some embodiments, any immunomodulatory chemokine that binds to a chemokine receptor, i.e., a CXC, CC, C, or CX3C chemokine receptor, can be used in the context of the present invention. Examples of chemokines include, but are not limited to, MIP-3a (Lax), MIP-3β, Hcc-1, MPIF-1, MPIF-2, MCP-2, MCP-3, MCP-4, MCP-5, Eotaxin, Tarc, Elc, I309, IL-8, GCP-2 Groα., Gro-β., Nap-2, Ena-78, Ip-10, MIG, I-Tac, SDF-1, and BCA-1 (Blc), as well as functional fragments thereof.

The compositions utilized in the methods described herein (e.g.. cancer immunotherapies) can be administered by any suitable method, including, for example, intravenously, intramuscularly, subcutaneously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subconjunctival, intravesicularly, mucosally, intrapericardially, intraumbilically, intraocularly, intraorbitally, orally, topically, transdermal, intravitreally (e.g., by intravitreal injection), by eye drop, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the compound or composition being administered and the severity of the condition, disease, or disorder being treated) In some instances, the checkpoint inhibitor is administered intravenously, intramuscularly, subcutaneously, topically, orally, transdermal, intraperitoneally, intraorbitally, by implantation, by inhalation, intrathecally, intraventricularly, or intranasally. Dosing can be by any suitable route, e g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

Cancer immunotherapies (e.g., an antibody, binding polypeptide, and/or small molecule) described herein (any additional therapeutic agent) may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutic agent need not be, but is optionally formulated with and/or administered concurrently with one or more agents currently used to prevent or treat the disorder in question. The effective amount of such other agents depends on the amount of the checkpoint inhibitor present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

The progress of this therapy is easily monitored by conventional techniques and assays. For example, as a general proposition, the therapeutically effective amount of an immune checkpoint inhibitor, for example, a PD-L1 axis binding antagonist antibody, an anti-CTLA-4 antibody, an anti-TIM-3 antibody, or an anti-LAG-3 antibody, administered to human will be in the range of about 0.01 to about 50 mg/kg of patient body weight, whether by one or more administrations. In some instances, the antibody used is about 0.01 mg/kg to about 45 mg/kg, about 0.01 mg/kg to about 40 mg/kg, about 0.01 mg/kg to about 35 mg/kg, about 0.01 mg/kg to about 30 mg/kg, about 0.01 mg/kg to about 25 mg/kg, about 0.01 mg/kg to about 20 mg/kg, about 0.01 mg/kg to about 1 5 mg/kg, about 0.01 mg/kg to about 10 mg/kg, about 0.01 mg/kg to about 5 mg/kg, or about 0.01 mg/kg to about 1 mg/kg administered daily, weekly, every two weeks, every three weeks, or monthly, for example. In some instances, the antibody is administered at 1 5 mg/kg. However, other dosage regimens may be useful. In one instance, an anti-PD-L1 antibody described herein is administered to a human at a dose of about 100 mg, about 200 mg, about 300 mg, about 400 mg, about 500 mg, about 600 mg, about 700 mg, about 800 mg, about 900 mg, about 1000 mg, about 1 100 mg, about 1200 mg, about 1300 mg, about 1400 mg, about 1500 mg, about 1600 mg, about 1700 mg, or about 1800 mg on day 1 of 21 -day cycles (every three weeks, q3w). In some instances, anti-PD-L1 antibody MPDL3280A is administered at 1200 mg intravenously every three weeks (q3w) The dose may be administered as a single dose or as multiple doses (e.g., 2 or 3 doses), such as infusions. The dose of the antibody administered in a combination treatment may be reduced as compared to a single treatment. The progress of this therapy is easily monitored by conventional techniques.

In some embodiments, the methods further involve administering to the patient an effective amount of an additional therapeutic agent. In some embodiments, the additional anti-cancer therapy comprises one or more of surgery, radiotherapy, chemotherapy, anti-angiogenic therapy, anti-DNA repair therapy, and anti-inflammatory therapy. In some instances, the additional therapeutic agent is selected from the group consisting of an anti-neoplastic agent, a chemotherapeutic agent, a growth inhibitory agent, an anti-angiogenic agent, a radiation therapy, a cytotoxic agent, and combinations thereof. In some instances, a cancer immunotherapy may be administered in conjunction with a chemotherapy or chemotherapeutic agent. In some embodiments, the chemotherapy or chemotherapeutic agent is a platinum-based agent (including without limitation cisplatin, carboplatin, oxaliplatin, and stamplatin). In some mstances, a cancer immunotherapy may be administered in conjunction with a radiation therapy agent. In some instances, a cancer immunotherapy may be administered in conjunction with a targeted therapy or targeted therapeutic agent. In some instances, a cancer immunotherapy may be administered in conjunction with another immunotherapy or immunotherapeutic agent, for example a monoclonal antibody. In some instances, the additional therapeutic agent is an agonist directed against a co-stimulatory molecule. In some instances, the additional therapeutic agent is an antagonist directed against a co-inhibitory molecule. In some instances, the cancer immunotherapy is administered as a monotherapy.

Examples of chemotherapeutic agents include alkylating agents, such as thiotepa and cyclosphosphamide; alkyl sulfonates, such as busulfan, improsulfan, and piposulfan; aziridines, such as benzodopa, carboquone, meturedopa, and uredopa; ethylenimines and methylamelamines, including altretamine, triethylenemelamine, trietylenephosphoramide, triethiylenethiophosphoramide, and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8), dolastatin; duocarmycin (including the synthetic analogues, KW-2189 and CB1-TM1); eleutherobin; pancratistatin; a sarcodictyin; spongistatin; nitrogen mustards, such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, and uracil mustard; nitrosureas, such as carmustine, chlorozotocin, fotemustine, lomustine, nimustine, and ranimnustine; antibiotics, such as the enediyne antibiotics (e.g., calicheamicin, especially calicheamicin gammall and calicheamicin omegall); dynemicin, including dynemicin A; bisphosphonates, such as clodronate; an esperamicin; as well as neocarzinostatin chromophore and related chromoprotein enediyne antiobiotic chromophores, aclacinomysins, actinomycin, authramycin, azaserine, bleomycins, cactinomycin, carabicin, carminomycin, carzinophilin, chromomycinis, dactinomycin, daunorubicin, detorubicin, 6- diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, and zorubicin; anti-metabolites, such as methotrexate and 5-fluorouracil (5-FU); folic acid analogues, such as denopterin, pteropterin, and trimetrexate; purine analogs, such as fludarabine, 6-mercaptopurine, thiamiprine, and thioguanine; pyrimidine analogs, such as ancitabine, azacitidine, 6-azauridine, carmofur, cytarabine, dideoxyuridine, doxifluridine, enocitabine, and floxuridine; androgens, such as calusterone, dromostanolone propionate, epitiostanol, mepitiostane, and testolactone; anti-adrenals, such as mitotane and trilostane; folic acid replenishes such as frolinic acid; acetylatone; aldophosphamide glycoside; aminolevulinic acid; eniluracil; amsacrine, bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids, such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSKpolysaccharide complex; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; taxoids, e.g., paclitaxel and docetaxel gemcitabine; 6-thioguanine; mercaptopurine; platinum coordination complexes, such as cisplatin, oxaliplatin, and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-11); topoisomerase inhibitor RFS 2000; difluorometlhylomithine (DMFO); retinoids, such as retinoic acid; capecitabine; carboplatin, procarbazine, plicomycin, gemcitabien, navelbine, famesyl-protein tansferase inhibitors, transplatinum, and pharmaceutically acceptable salts, acids, or derivatives of any of the above.

Some (non-limiting) examples of chemotherapeutic drugs which can be combined with the present disclosure are carboplatin (Paraplatin), cisplatin (Platinol, Platinol-AQ), cyclophosphamide (Cytoxan, Neosar), docetaxel (Taxotere), doxorubicin (Adriamycin), erlotinib (Tarceva), etoposide (VePesid), fluorouracil (5-FU), gemcitabine (Gemzar), imatinib mesylate (Gleevec), irinotecan (Camptosar), methotrexate (Folex, Mexate, Amethopterin), paclitaxel (Taxol, Abraxane), sorafinib (Nexavar), sunitinib (Sutent), topotecan (Hycamtin), vincristine (Oncovin, Vincasar PFS), and vinblastine (Velban)." "Another large group of potential targets for complementary cancer therapy comprises kinase inhibitors, because the growth and survival of cancer cells is closely interlocked with the deregulation of kinase activity. To restore normal kinase activity and therefor reduce tumor growth a broad range of inhibitors is in used. The group of targeted kinases comprises receptor tyrosine kinases e.g. BCR-ABL, B-Raf, EGFR, HER-2/ErbB2, IGF-IR, PDGFR-a, PDGFR- β, cKit, Flt-4, Flt3, FGFR1, FGFR3, FGFR4, CSF1R, c-Met, RON, c-Ret, ALK, cytoplasmic tyrosine kinases e.g. c-SRC, c-YES, Abl, JAK-2, serine/threonine kinases e.g. ATM, Aurora A & B, CDKs, mTOR, PKCi, PLKs, b-Raf, S6K, STK1 1/LKB1 and lipid kinases e.g. PI3K, SKI. Small molecule kinase inhibitors are e.g. PHA-739358, Nilotinib, Dasatinib, and PD166326, NSC 74341 1, Lapatinib (GW-572016), Canertinib (CI-1033), Semaxinib (SU5416), Vatalanib (PTK787/ZK222584), Sutent (SU1 1248), Sorafenib (BAY 43-9006) and Leflunomide (SU101). For more information see e.g. Zhang et al. 2009: Targeting cancer with small molecule kinase inhibitors. Nature Reviews Cancer 9, 28-39." "Small molecule targeted therapy drugs are generally inhibitors of enzymatic domains on mutated, overexpressed, or otherwise critical proteins within the cancer cell. Prominent and nonlimiting examples are the tyrosine kinase inhibitors imatinib (Gleevec/Glivec) and gefitinib (Iressa).

In some embodiments, the additional anti-cancer therapy comprises anti-angiogenic therapy. Angiogenesis inhibitors prevent the extensive growth of blood vessels (angiogenesis) that tumors require to survive. The angiogenesis promoted by tumor cells to meet their increasing nutrient and oxygen demands for example can be blocked by targeting different molecules. Non-limiting examples of angiogenesis-mediating molecules or angiogenesis inhibitors which may be combined with the present invention are soluble VEGF (VEGF isoforms VEGF121 and VEGF165, receptors VEGFR1, VEGFR2 and co-receptors Neuropilin-1 and Neuropilin-2) 1 and NRP-1, angiopoietin 2, TSP-1 and TSP-2, angiostatin and related molecules, endostatin, vasostatin, calreticulin, platelet factor-4, TIMP and CDAI, Meth-1 and Meth-2, IFNα, -β and -γ , CXCL10, IL-4, -12 and -18, prothrombin (kringle domain-2), antithrombin III fragment, prolactin, VEGI, SPARC, osteopontin, maspin, canstatin, proliferin-related protein, restin and drugs like e.g. bevacizumab, itraconazole, carboxyamidotriazole, TNP-470, CM101, IFN-a,, platelet factor-4, suramin, SU5416, thrombospondin, VEGFR antagonists, angiostatic steroids + heparin, cartilage-derived angiogenesis Inhibitory factor, matrix metalloproteinase inhibitors, 2-methoxyestradiol, tecogalan, tetrathiomolybdate, thalidomide, thrombospondin, prolactina v β3 inhibitors, linomide, and tasquinimod. In some embodiments, known therapeutic candidates include naturally occurring angiogenic inhibitors, including without limitation, angiostatin, endostatin, and platelet factor-4. In another embodiment therapeutic candidates include, without limitation, specific inhibitors of endothelial cell growth, such as TNP-470, thalidomide, and interleukin-12. Still other anti-angiogenic agents include those that neutralize angiogenic molecules, such as including without limitation, antibodies to fibroblast growth factor or antibodies to vascular endothelial growth factor or antibodies to platelet derived growth factor or antibodies or other types of inhibitors of the receptors of EGF, VEGF or PDGF. In some embodiments, antiangiogenic agents include without limitation suramin and its analogs, and tecogalan. In other embodiments, anti-angiogenic agents include without limitation agents that neutralize receptors for angiogenic factors or agents that interfere with vascular basement membrane and extracellular matrix, including, without limitation, metalloprotease inhibitors and angiostatic steroids. Another group of anti-angiogenic compounds includes, without limitation, anti-adhesion molecules, such as antibodies to integrin alpha v beta 3. Still other anti-angiogenic compounds or compositions, include, without limitation, kinase inhibitors, thalidomide, itraconazole, carboxyamidotriazole, CM101, IFN-α, IL-12, SU5416, thrombospondin, cartilage-derived angiogenesis inhibitory factor, 2-methoxyestradiol, tetrathiomolybdate, thrombospondin, prolactin, and linomide. In one particular embodiment, the anti-angiogenic compound is an antibody to VEGF, such as Avastin^{®}/bevacizumab (Genentech).

In some embodiments, the additional anti-cancer therapy comprises anti-DNA repair therapy. In some embodiments, the DNA damage repair and response inhibitor is selected from a PARP inhibitor, a RAD51 inhibitor, or an inhibitor of a DNA damage response kinase selected from CHCK1, ATM, or ATR. In some embodiments, the additional anti-cancer therapy comprises a radiosensitizer. Exemplary radiosensitizers include hypoxia radiosensitizers such as misonidazole, metronidazole, and trans-sodium crocetinate, a compound that helps to increase the diffusion of oxygen into hypoxic tumor tissue. The radiosensitizer can also be a DNA damage response inhibitor interfering with base excision repair (BER), nucleotide excision repair (NER), mismatch repair (MMR), recombinational repair comprising homologous recombination (HR) and non-homologous end-joining (NHEJ), and direct repair mechanisms. SSB repair mechanisms include BER, NER, or MMR pathways whilst DSB repair mechanisms consist of HR and NHEJ pathways. Radiation causes DNA breaks that if not repaired are lethal. Single strand breaks are repaired through a combination of BER, NER and MMR mechanisms using the intact DNA strand as a template. The predominant pathway of SSB repair is the BER utilizing a family of related enzymes termed poly-(ADP-ribose) polymerases (PARP). Thus, the radiosensitizer can include DNA damage response inhibitors such as Poly (ADP) ribose polymerase (PARP) inhibitors. In some embodiments, the additional anti-cancer therapy is a DNA repair and response pathway inhibitor, PARP inhibitor (e.g., Talazoparib, Rucaparib, Olaparib). RAD51 inhibitor (RI-1), or an inhibitor of DNA damage response kinases such as CHCK1 (AZD7762), ATM (KU-55933, KU-60019, NU7026, VE-821), and ATR (NU7026).

In some embodiments, the additional anti-cancer therapy comprises anti-inflammatory agent. In some embodiments, the anti-inflammatory agent is an agent that blocks, inhibits, or reduces inflammation or signaling from an inflammatory signaling pathway In some embodiments, the anti-inflammatory agent inhibits or reduces the activity of one or more of any of the following: IL-1, IL-2, IL-3, IL-4, IL-5, IL-6, IL-7, IL-8, IL-9, IL-10, IL-12, IL-13, IL-15, IL-18, IL-23, interferons (IFNs), e.g., IFNα, IFNβ, IFNγ, IFN-γ inducing factor (IGIF), transforming growth factor-β (TGF-β), transforming growth factor-a (TGF-a), tumor necrosis factors TNF-α, TNF-β, TNF-RI, TNF-RII, CD23, CD30, CD40L, EGF, G-CSF, GDNF, PDGF-BB, RANTES/CCL5, IKK, NF-κB, TLR2, TLR3, TLR4, TL5, TLR6, TLR7, TLR8, TLR8, TLR9, and/or any cognate receptors thereof. In some embodiments, the anti-inflammatory agent is an IL-1 or IL-1 receptor antagonist, such as anakinra (KINERET^{®}), rilonacept, or canakinumab. In some embodiments, the anti-inflammatory agent is an IL-6 or IL-6 receptor antagonist, e.g., an anti-IL-6 antibody or an anti-IL-6 receptor antibody, such as tocilizumab (ACTEMRA^{®}), olokizumab, clazakizumab, sarilumab, sirukumab, siltuximab, or ALX-0061. In some embodiments, the anti-inflammatory agent is a TNF-a antagonist, e.g., an anti-TNFα antibody, such as infliximab (REMICADE^{®}), golimumab (SIMPONI^{®}), adalimumab (HUMIRA^{®}), certolizumab pegol (CIMZIA^{®}) or etanercept.

In some embodiments, the anti-inflammatory agent is a corticosteroid. Exemplary corticosteroids include, but are not limited to, cortisone (hydrocortisone, hydrocortisone sodium phosphate, hydrocortisone sodium succinate, ALA-CORT^{®}, HYDROCORT ACETATE^{®}, hydrocortone phosphate LANACORT^{®}, SOLU-CORTEF^{®}), decadron (dexamethasone, dexamethasone acetate, dexamethasone sodium phosphate, DEXASONE^{®}, DIODEX^{®}, HEXADROL^{®}, MAXIDEX^{®}), methylprednisolone (6-methylprednisolone, methylprednisolone acetate, methylprednisolone sodium succinate, DURALONE^{®}, MEDRALONE^{®}, MEDROL^{®}, M-PREDNISOL^{®}, SOLU-MEDROL^{®}), prednisolone (DELTA-CORTEF^{®}, ORAPRED^{®}, PEDIAPRED^{®}, PREZONE^{®}), and prednisone (DELTASONE^{®}, LIQUID PRED^{®}, METICORTEN^{®}, ORASONE^{®})), and bisphosphonates (e.g., pamidronate (AREDIA^{®}), and zoledronic acid (ZOMETAC^{®}).

Such combination therapies noted above encompass combined administration (where two or more therapeutic agents are included in the same or separate formulations), and separate administration, in which case, administration of a cancer immunotherapy can occur prior to, simultaneously, and/or following, administration of the additional therapeutic agent or agents. In one instance, administration of a cancer immunotherapy and administration of an additional therapeutic agent occur within about one month, or within about one, two or three weeks, or within about one, two, three, four, five, or six days, of each other.

Without wishing to be bound to theory, it is thought that enhancing T-cell stimulation, by promoting a co-stimulatory molecule or by inhibiting a co-inhibitory molecule, may promote tumor cell death thereby treating or delaying progression of cancer. In some instances, an immune checkpoint inhibitor, for example, a PD-L1 axis binding antagonist and/or CTLA4 antagonist, may be administered in conjunction with an agonist directed against a co-stimulatory molecule. In some instances, a co-stimulatory molecule may include CD40, CD226, CD28, OX40, GITR, CD137, CD27, HVEM, or CD127 In some instances, the agonist directed against a co-stimulatory molecule is an agonist antibody that binds to CD40, CD226, CD28, OX40, GITR, CD137, CD27, HVEM, or CD127. In some instances, an immune checkpoint inhibitor, for example, a PD-L1 axis binding antagonist and/or CTLA4 antagonist, may be administered in conjunction with an antagonist directed against a co-inhibitory molecule. In some instances, a co-inhibitory molecule may include CTLA-4 (also known as CD1 52), TIM-3, BTLA. VISTA. LAG-3. B7-H3, 87-H4, IDO, TIGIT, MICA/B, or arginase. In some instances, the antagonist directed against a co-inhibitory molecule is an antagonist antibody that binds to CTLA-4, TIM-3, BILA, VISTA, LAG-3, B7-H3, B7-H4. IDO, TIGIT, MICA/B, or arginase.

In some instances, a PD-L1 axis binding antagonist may be administered in conjunction with an antagonist directed against CTLA-4 (also known as CD152), e.g.. a blocking antibody. In some instances, a PD-L1 axis binding antagonist may be administered in conjunction with ipilimumab (also known as MDX-010, MDX-101 , or YERVOY^{®}). In some instances, a PD-L1 axis binding antagonist may be administered in conjunction with tremelimumab (also known as ticilimumab or CP-675,206). In some instances, a PD-L1 axis binding antagonist may be administered in conjunction with an antagonist directed against B7-H3 (also known as CD276), e.g., a blocking antibody. In some instances, a PD-L1 axis binding antagonist may be administered in conjunction with MGA271 . In some instances, a PD-L1 axis binding antagonist may be administered in conjunction with an antagonist directed against a TGF-beta, e.g., metelimumab (also known as CAT-192), fresolimumab (also known as GC1008), or LY2157299.

In some instances, an immune checkpoint inhibitor, for example, a PD-L1 axis binding antagonist and/or CTLA4 antagonist, may be administered in conjunction with a treatment comprising adoptive transfer of a T-cell (e.g., a cytotoxic T-cell or CTL) expressing a chimeric antigen receptor (CAR). In some instances, an immune checkpoint inhibitor, for example, a PD-L1 axis binding antagonist and/or CTLA4 antagonist, may be administered in conjunction with a treatment comprising adoptive transfer of a T-cell comprising a dominant-negative TGF beta receptor, e.g , a dominant-negative TGF beta type II receptor In some instances, an immune checkpoint inhibitor, for example, a PD-L1 axis binding antagonist and/or CTLA4 antagonist, may be administered in conjunction with a treatment comprising a HERCREEM protocol (see, e g., ClinicalTrials.gov Identifier NCT00889954).

In some instances, an immune checkpoint inhibitor, for example, a PD-L1 axis binding antagonist and/or CTLA4 antagonist, may be administered in conjunction with an agonist directed against CD137 (also known as TNFRSF9, 4-1 BB, or ILA), e.g., an activating antibody. In some instances, an immune checkpoint inhibitor, for example, a PD-L1 axis binding antagonist and/or CTLA4 antagonist, may be administered in conjunction with urelumab (also known as BMS-663513). In some instances, an immune checkpoint inhibitor, for example, a PD-L1 axis binding antagonist and/or CTLA4 antagonist, may be administered in conjunction with an agonist directed against CD40, e.g., an activating antibody. In some instances, an immune checkpoint inhibitor, for example, a PD-L1 axis binding antagonist and/or CTLA4 antagonist, may be administered in conjunction with CP-870893. In some instances, an immune checkpoint inhibitor, for example, a PD-L1 axis binding antagonist and/or CTLA4 antagonist, may be administered in conjunction with an agonist directed against OX40 (also known as CD134), e.g., an activating antibody. In some instances, an immune checkpoint inhibitor, for example, a PD-L1 axis binding antagonist and/or CTLA4 antagonist, may be administered in conjunction with an anti-OX40 antibody (e.g., AgonOX). In some instances, an immune checkpoint inhibitor, for example, a PD-L1 axis binding antagonist and/or CTLA4 antagonist, may be administered in conjunction with an agonist directed against CD27, e.g., an activating antibody. In some instances, an immune checkpoint inhibitor, for example, a PD-L1 axis binding antagonist and/or CTLA4 antagonist, may be administered in conjunction with CDX-1127. In some instances, an immune checkpoint inhibitor, for example, a PD-L1 axis binding antagonist and/or CTLA4 antagonist, may be administered in conjunction with an antagonist directed against indoleamine-2,3-dioxygenase (IDO). In some instances, with the IDO antagonist is 1 -methyl-D-tryptophan (also known as 1 -D-MT).

In some instances, an immune checkpoint inhibitor, for example, a PD-L1 axis binding antagonist and/or CTLA4 antagonist, may be administered in conjunction with an antibody-drug conjugate. In some instances, the antibody-drug conjugate comprises mertansine or monomethyl auristatin E (MMAE). In some instances, an immune checkpoint inhibitor, for example, a PD-L1 axis binding antagonist and/or CTLA4 antagonist, may be administered in conjunction with an anti-NaPi2b antibody-MMAE conjugate (also known as DNIB0600A or RG7599) In some instances, an immune checkpoint inhibitor, for example, a PD-L1 axis binding antagonist and/or CTLA4 antagonist, may be administered in conjunction with trastuzumab emtansine (also known as T-DM 1 , ado-trastuzumab emtansine, or KADCYLA^{®}, Genentech). In some instances, an immune checkpoint inhibitor, for example, a PD-L1 axis binding antagonist and/or CTLA4 antagonist, may be administered in conjunction with DMUC5754A. In some instances, an immune checkpoint inhibitor, for example, a PD-L1 axis binding antagonist and/or CTLA4 antagonist, may be administered in conjunction with an antibody-drug conjugate targeting the endothelin B receptor (EDNBR), e.g., an antibody directed against EDNBR conjugated with MMAE.

In some instances, an immune checkpoint inhibitor, for example, a PD-L1 axis binding antagonist and/or CTLA4 antagonist, may be administered in conjunction with an anti-angiogenesis agent. In some instances, an immune checkpoint inhibitor, for example, a PD-L1 axis binding antagonist and/or CTLA4 antagonist, may be administered in conjunction with an antibody directed against a VEGF, e.g., VEGF-A. In some instances, an immune checkpoint inhibitor, for example, a PD-L1 axis binding antagonist and/or CTLA4 antagonist, may be administered in conjunction with bevacizumab (also known as AVASTIN^{®}, Genentech). In some instances, an immune checkpoint inhibitor, for example, a PD-L1 axis binding antagonist and/or CTLA4 antagonist, may be administered in conjunction with an antibody directed against angiopoietin 2 (also known as Ang2). In some instances, an immune checkpoint inhibitor, for example, a PD-L1 axis binding antagonist and/or CTLA4 antagonist, may be administered in conjunction with MEDI3617.

In some instances, an immune checkpoint inhibitor, for example, a PD-L1 axis binding antagonist and/or CTLA4 antagonist, may be administered in conjunction with an antineoplastic agent. In some instances, an immune checkpoint inhibitor, for example, a PD-L1 axis binding antagonist and/or CTLA4 antagonist, may be administered in conjunction with an agent targeting CSF-1 R (also known as M-CSFR or CD1 15). In some instances, an immune checkpoint inhibitor, for example, a PD-L1 axis binding antagonist and/or CTLA4 antagonist, may be administered in conjunction with anti-CSF-1 R (also known as IMC-CS4). In some instances, an immune checkpoint inhibitor, for example, a PD-L1 axis binding antagonist and/or CTLA4 antagonist, may be administered in conjunction with an interferon, for example interferon alpha or interferon gamma. In some instances, an immune checkpoint inhibitor, for example, a PD-L1 axis binding antagonist and/or CTLA4 antagonist, may be administered in conjunction with Roferon-A (also known as recombinant Interferon alpha-2a). In some instances, an immune checkpoint inhibitor, for example, a PD-L1 axis binding antagonist and/or CTLA4 antagonist, may be administered in conjunction with GM-CSF (also known as recombinant human granulocyte macrophage colony stimulating factor, rhu GM-CSF, sargramostim, or LEUKINE^{®}). In some instances, an immune checkpoint inhibitor, for example, a PD-L1 axis binding antagonist and/or CTLA4 antagonist, may be administered in conjunction with IL-2 (also known as aldesleukin or PROLEUKIN^{®}). In some instances, an immune checkpoint inhibitor, for example, a PD-L1 axis binding antagonist and/or CTLA4 antagonist, may be administered in conjunction with IL-12. In some instances, an immune checkpoint inhibitor, for example, a PD-L1 axis binding antagonist and/or CTLA4 antagonist, may be administered in conjunction with an antibody targeting CD20. In some instances, the antibody targeting CD20 is obinutuzumab (also known as GA 101 or GAZYVA^{®}) or rituximab. In some instances, an immune checkpoint inhibitor, for example, a PD-L1 axis binding antagonist and/or CTLA4 antagonist, may be administered in conjunction with an antibody targeting GITR. In some instances, the antibody targeting GITR is TRX518.

In some instances, an immune checkpoint inhibitor, for example, a PD-L1 axis binding antagonist and/or CTLA4 antagonist, may be administered in conjunction with a cancer vaccine. In some instances, the cancer vaccine is a peptide cancer vaccine, which in some instances is a personalized peptide vaccine. In some instances the peptide cancer vaccine is a multivalent long peptide, a multi-peptide, a peptide cocktail, a hybrid peptide, or a peptide-pulsed dendritic cell vaccine (see, e.g., Yamada et al., Cancer Sci. 104:14-21 , 2013). In some instances, an immune checkpoint inhibitor, for example, a PD-L1 axis binding antagonist and/or CTLA4 antagonist, may be administered in conjunction with an adjuvant. In some instances, an immune checkpoint inhibitor, for example, a PD-L1 axis binding antagonist and/or CTLA4 antagonist, may be administered in conjunction with a treatment comprising a TLR agonist, e.g., Poly-ICLC (also known as HILTONOL^{®}), LPS, MPL, or CpG ODN In some instances, an immune checkpoint inhibitor, for example, a PD-L1 axis binding antagonist and/or CTLA4 antagonist, may be administered in conjunction with tumor necrosis factor (TNF) alpha. In some instances, an immune checkpoint inhibitor, for example, a PD-L1 axis binding antagonist and/or CTLA4 antagonist, may be administered in conjunction with IL-1 . In some instances, an immune checkpoint inhibitor, for example, a PD-L1 axis binding antagonist and/or CTLA4 antagonist, may be administered in conjunction with HMGB1. In some instances, an immune checkpoint inhibitor, for example, a PD-L1 axis binding antagonist and/or CTLA4 antagonist, may be administered in conjunction with an IL-10 antagonist. In some instances, an immune checkpoint inhibitor, for example, a PD-L1 axis binding antagonist and/or CTLA4 antagonist, may be administered in conjunction with an IL-4 antagonist. In some instances, an immune checkpoint inhibitor, for example, a PD-L1 axis binding antagonist and/or CTLA4 antagonist, may be administered in conjunction with an IL-13 antagonist. In some instances, an immune checkpoint inhibitor, for example, a PD-L1 axis binding antagonist and/or CTLA4 antagonist, may be administered in conjunction with an HVEM antagonist. In some instances, an immune checkpoint inhibitor, for example, a PD-L1 axis binding antagonist and/or CTLA4 antagonist, may be administered in conjunction with an ICOS agonist. e.g., by administration of ICOS-L, or an agonistic antibody directed against ICOS. In some instances, an immune checkpoint inhibitor, for example, a PD-L1 axis binding antagonist and/or CTLA4 antagonist, may be administered in conjunction with a treatment targeting CX3CL1 . In some instances, an immune checkpoint inhibitor, for example, a PD-L1 axis binding antagonist and/or CTLA4 antagonist, may be administered in conjunction with a treatment targeting CXCL9. In some instances, an immune checkpoint inhibitor, for example, a PD-L1 axis binding antagonist and/or CTLA4 antagonist, may be administered in conjunction with a treatment targeting CXCL10. In some instances, an immune checkpoint inhibitor, for example, a PD-L1 axis binding antagonist and/or CTLA4 antagonist, may be administered in conjunction with a treatment targeting CCL5. In some instances, an immune checkpoint inhibitor, for example, a PD-L1 axis binding antagonist and/or CTLA4 antagonist, may be administered in conjunction with an LFA-1 or ICAM1 agonist. In some instances, an immune checkpoint inhibitor, for example, a PD-L1 axis binding antagonist and/or CTLA4 antagonist, may be administered in conjunction with a Selectin agonist.

In some instances, an immune checkpoint inhibitor, for example, a PD-L1 axis binding antagonist and/or CTLA4 antagonist, may be administered in conjunction with a targeted therapy In some instances, an immune checkpoint inhibitor, for example, a PD-L1 axis binding antagonist and/or CTLA4 antagonist, may be administered in conjunction with an inhibitor of B-Raf. In some instances, an immune checkpoint inhibitor, for example, a PD-L1 axis binding antagonist and/or CTLA4 antagonist, may be administered in conjunction with vemurafenib (also known as ZELBORAF^{®}). In some instances, an immune checkpoint inhibitor, for example, a PD-L1 axis binding antagonist and/or CTLA4 antagonist, may be administered in conjunction with dabrafenib (also known as TAFINLAR^{®}). In some instances, an immune checkpoint inhibitor, for example, a PD-L1 axis binding antagonist and/or CTLA4 antagonist, may be administered m conjunction with erlotinib (also known as TARCEVA^{®}) In some instances, an immune checkpoint inhibitor, for example, a PD-L1 axis binding antagonist and/or CTLA4 antagonist, may be administered in conjunction with an inhibitor of a MEK, such as MEK1 (also known as MAP2K1 ) or MEK2 (also known as MAP2K2). In some instances, an immune checkpoint inhibitor, for example, a PD-L1 axis binding antagonist and/or CTLA4 antagonist, may be administered in conjunction with cobimetinib (also known as GDC-0973 or XL-518). In some instances, an immune checkpoint inhibitor, for example, a PD-L1 axis binding antagonist and/or CTLA4 antagonist, may be administered in conjunction with trametimb (also known as MEKINIST^{®}). In some instances, an immune checkpoint inhibitor, for example, a PD-L1 axis binding antagonist and/or CTLA4 antagonist, may be administered in conjunction with an inhibitor of K-Ras. In some instances, an immune checkpoint inhibitor, for example, a PD-L1 axis binding antagonist and/or CTLA4 antagonist, may be administered in conjunction with an inhibitor of c-Met. In some instances, an immune checkpoint inhibitor, for example, a PD-L1 axis binding antagonist and/or CTLA4 antagonist, may be administered in conjunction with onartuzumab (also known as MetMAb). In some instances, an immune checkpoint inhibitor, for example, a PD-L1 axis binding antagonist and/or CTLA4 antagonist, may be administered in conjunction with an inhibitor of Alk. In some instances, an immune checkpoint inhibitor, for example, a PD-L1 axis binding antagonist and/or CTLA4 antagonist, may be administered in conjunction with AF802 (also known as CH5424802 or alectinib). In some instances, an immune checkpoint inhibitor, for example, a PD-L1 axis binding antagonist and/or CTLA4 antagonist, may be administered in conjunction with an inhibitor of a phosphatidylinositol 3-kinase (PI3K). In some instances, an immune checkpoint inhibitor, for example, a PD-L1 axis binding antagonist and/or CTLA4 antagonist, may be administered in conjunction with BKM120.

In some instances, an immune checkpoint inhibitor, for example, a PD-L1 axis binding antagonist and/or CTLA4 antagonist, may be administered in conjunction with idelalisib (also known as GS-1101 or CAL-101). In some instances, an immune checkpoint inhibitor, for example, a PD-L1 axis binding antagonist and/or CTLA4 antagonist, may be administered in conjunction with perifosine (also known as KRX-0401). In some instances, an immune checkpoint inhibitor, for example, a PD-L1 axis binding antagonist and/or CTLA4 antagonist, may be administered in conjunction with an inhibitor of an Akt. In some instances, an immune checkpoint inhibitor, for example, a PD-L1 axis binding antagonist and/or CTLA4 antagonist, may be administered in conjunction with MK2206. In some instances, an immune checkpoint inhibitor, for example, a PD-L1 axis binding antagonist and/or CTLA4 antagonist, may be administered in conjunction with GSK690693. In some mstances, an immune checkpoint inhibitor, for example, a PD-L1 axis binding antagonist and/or CTLA4 antagonist, may be administered in conjunction with GDC-0941 . In some instances, an immune checkpoint inhibitor, for example, a PD-L1 axis binding antagonist and/or CTLA4 antagonist, may be administered in conjunction with an inhibitor of mTOR. In some instances, an immune checkpoint inhibitor, for example, a PD-L1 axis binding antagonist and/or CTLA4 antagonist, may be administered in conjunction with sirolimus (also known as rapamycin). In some instances, an immune checkpoint inhibitor, for example, a PD-L1 axis binding antagonist and/or CTLA4 antagonist, may be administered in conjunction with temsirolimus (also known as CCI-779 or TORISEL^{®}). In some instances, an immune checkpoint inhibitor, for example, a PD-L1 axis binding antagonist and/or CTLA4 antagonist, may be administered in conjunction with everolimus (also known as RAD001 ). In some instances, an immune checkpoint inhibitor, for example, a PD-L1 axis binding antagonist and/or CTLA4 antagonist, may be administered in conjunction with ridaforolimus (also known as AP-23573, MK-8669. or deforolimus). In some instances, an immune checkpoint inhibitor, for example, a PD-L1 axis binding antagonist and/or CTLA4 antagonist, may be administered in conjunction with OSI-027. In some instances, an immune checkpoint inhibitor, for example, a PD-L1 axis binding antagonist and/or CTLA4 antagonist, may be administered in conjunction with AZD8055. In some instances, an immune checkpoint inhibitor, for example, a PD-L1 axis binding antagonist and/or CTLA4 antagonist, may be administered in conjunction with INK1 28. In some instances, an immune checkpoint inhibitor, for example, a PD-L1 axis binding antagonist and/or CTLA4 antagonist, may be administered in conjunction with a dual PI3K/mTOR inhibitor. In some instances, an immune checkpoint inhibitor, for example, a PD-L1 axis binding antagonist and/or CTLA4 antagonist, may be administered in conjunction with XL765 In some instances, an immune checkpoint inhibitor, for example, a PD-L1 axis binding antagonist and/or CTLA4 antagonist, may be administered in conjunction with GDC-0980. In some instances, an immune checkpoint inhibitor, for example, a PD-L1 axis binding antagonist and/or CTLA4 antagonist, may be administered in conjunction with BEZ235 (also known as NVP-BEZ235). In some instances, an immune checkpoint inhibitor, for example, a PD-L1 axis binding antagonist and/or CTLA4 antagonist, may be administered in conjunction with BGT226. In some instances, an immune checkpoint inhibitor, for example, a PD-L1 axis binding antagonist and/or CTLA4 antagonist, may be administered in conjunction with GSK2126458. In some instances, an immune checkpoint inhibitor, for example, a PD-L1 axis binding antagonist and/or CTLA4 antagonist, may be administered in conjunction with PF-04691502. In some instances, an immune checkpoint inhibitor, for example, a PD-L1 axis binding antagonist and/or CTLA4 antagonist, may be administered in conjunction with PF-05212384 (also known as PKI-587).

While PD-L1 axis binding antagonists and CTLA4 antagonists are called out *supra* as exemplary cancer immunotherapies, this is not intended to be limiting; any cancer immunotherapy of the present disclosure may be administered in conjunction with any of the other treatments described herein, or otherwise known in the art (subject to medical judgement).

### Antibody Preparation

An antibody according to any of the above embodiments (*e.g*., a checkpoint inhibitor of the present disclosure) may incorporate any of the features, singly or in combination, as described below:

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20^{®}) in PBS. When the plates have dried, 150 µl/well of scintillant (MICROSCINT-20^{™}; Packard) is added, and the plates are counted on a TOPCOUNT^{™} gamma counter (Packard) for ten minutes. Concentrations of each Fab that give less than or equal to 20% of maximal binding are chosen for use in competitive binding assays.

According to another embodiment, Kd is measured using a BIACORE^{®} surface plasmon resonance assay. For example, an assay using a BIACORE^{®}-2000 or a BIACORE ^{®}-3000 (BIAcore, Inc., Piscataway, NJ) is performed at 25°C with immobilized antigen CM5 chips at ~10 response units (RU). In one embodiment, carboxymethylated dextran biosensor chips (CM5, BIACORE, Inc.) are activated with *N*-ethyl-*N*'- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (~0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{™}) surfactant (PBST) at 25°C at a flow rate of approximately 25 µl/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (Kd) is calculated as the ratio k_{off}/k_{on.} See, e.g., Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25oC of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO^{™} spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem, 271:22611-22618 (1996)).

### 4. Human Antibodies

In certain embodiments, an antibody provided herein is a human antibody Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE^{™} technology; U.S. Patent No. 5,770,429 describing HUMAB^{®} technology; U.S. Patent No. 7,041,870 describing K-M MOUSE^{®} technology, and U.S. Patent Application Publication No. US 2007/0061900, describing VELOCIMOUSE^{®} technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/01 17126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for an immune checkpoint protein of the present disclosure and the other is for any other antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); crosslinking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to an immune checkpoint protein as well as another, different antigen (see, US 2008/0069820, for example).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table A under the heading of "conservative substitutions." More substantial changes are provided in Table A under the heading of "other exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE A**

| **Original Residue** | **Other Exemplary Substitutions** | **Conservative Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Cln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gin;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244: 1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain embodiments, an antibody of the present disclosure is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US2003/0157108; US2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody of the present disclosure, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the present disclosure contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat 'l Acad. Sci. USA 83:7059-7063 (1986)) and Hellstrom, I et al., Proc. Nat 'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

Therapeutic formulations of the immune checkpoint inhibitors, e.g., PD-L1 axis binding antagonists (e.g , an anti-PD-L1 antibody (e.g , MPDL3280A)) and antagonists directed against a co-inhibitory molecule (e g., a CTLA-4 antagonist (e g., an anti-CTLA-4 antibody), a TIM-3 antagonist (e.g., an anti-TIM-3 antibody), or a LAG-3 antagonist (e.g., an anti-LAG-3 antibody)) used in accordance with the present invention are prepared for storage by mixing the antagonist having the desired degree of purity with optional pharmaceutically acceptable carriers, excipients, or stabilizers in the form of lyophilized formulations or aqueous solutions. For general information concerning formulations, see, e.g, Gilman et al. (eds.) The Pharmacological Bases of Therapeutics, 8th Ed., Pergamon Press, 1990; A. Gennaro (ed.), Remington's Pharmaceutical Sciences, 18th Edition, Mack Publishing Co., Pennsylvania, 1990, Avis et al. (eds.) Pharmaceutical Dosage Forms: Parenteral Medications Dekker, New York, 1993; Lieberman et al. (eds.) Pharmaceutical Dosage Forms. Tablets Dekker, New York. 1990; Lieberman et al. (eds.), Pharmaceutical Dosage Forms. Disperse Systems Dekker, New York, 1990; and Walters (ed.) Dermatological and Transdermal Formulations (Drugs and the Pharmaceutical Sciences), Vol 1 19, Marcel Dekker, 2002.

Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride, benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™}, or polyethylene glycol (PEG).

The formulation herein may also contain more than one active compound, for example, those with complementary activities that do not adversely affect each other The type and effective amounts of such medicaments depend, for example, on the amount and type of antagonist present in the formulation, and clinical parameters of the subjects.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions Such techniques are disclosed in Remington's Pharmaceutical Sciences 16th edition, Osol, A Ed. (1980)

Sustained-release preparations may be prepared. Suitable examples of sustained-release preparations include semi-permeable matrices of solid hydrophobic polymers containing the antagonist, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,91 9), copolymers of L-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

It is to be understood that any of the above articles of manufacture may include an immunoconjugate described herein in place of or in addition to an immune checkpoint inhibitor.

### IV. Articles of Manufacture or Kits

Provided herein are kits including one or more reagents for detecting an *EWSR1-WT1* fusion gene encoding a neoantigen, as described herein, from a sample from the individual. In some embodiments, the reagents include one or more oligonucleotides, *e.g.*, for hybridization with DNA, RNA, or cDNA encoding any of the fusion genes or neoantigens of the present disclosure. In some embodiments, the one or more oligonucleotides hybridize with at least a portion of a DNA or RNA sequence encoding the amino acid sequence of any one of SEQ ID Nos:1-11.

Optionally, the kit may further include instructions to use the kit to select a medicament (e.g., an immune checkpoint inhibitor, for example, a PD-L1 axis binding antagonist (e.g , anti-PD-L1 antibody, e.g., MPDL3280A)), an antagonist directed against a co-inhibitory molecule (e.g., a CTLA-4 antagonist (e.g , an anti-CTLA-4 antibody), a TIM-3 antagonist (e g.. an anti-TIM-3 antibody), or a LAG-3 antagonist (e.g., an anti-LAG-3 antibody)), or any combination thereof, for treating a cancer if the presence of an *LWSR1-WT1* fusion gene encoding a neoantigen is detected.

Provided herein are also articles of manufacture including, packaged together, an immune checkpoint inhibitor, for example, a PD-L1 axis binding antagonist (e g., an anti- PD-L1 antibody), an antagonist directed against a co-inhibitory molecule (e.g., a CTLA-4 antagonist (e g., an anti-CTLA-4 antibody), a TIM-3 antagonist (e.g , an anti-TIM-3 antibody), or a LAG-3 antagonist (e g., an anti-LAG-3 antibody)), or any combination thereof, in a pharmaceutically acceptable carrier and a package insert indicating that the immune checkpoint inhibitor is for treating a patient with a cancer as described herein based at least in part on the presence of a fusion gene or neoantigen of the present disclosure. Treatment methods include any of the treatment methods disclosed herein. The invention also concerns a method for manufacturing an article of manufacture comprising combining in a package a pharmaceutical composition comprising an immune checkpoint inhibitor, for example, a PD-L1 axis binding antagonist (e.g., an anti-PD-L1 antibody), an antagonist directed against a co-inhibitory molecule (e.g., a CTLA-4 antagonist (e.g., an anti-CTLA-4 antibody), a TIM-3 antagonist (e.g., an anti-TIM-3 antibody), or a LAG-3 antagonist (e.g.. an anti-LAG-3 antibody)), or any combination thereof,, and a package insert indicating that the pharmaceutical composition is for treating a patient with a disorder of the present disclosure based on the presence of a fusion gene or neoantigen detected in a sample from the individual.

The article of manufacture may include, for example, a container and a label or package insert on or associated with the container. Suitable containers include, for example, bottles, vials, syringes, and the like. The container may be formed from a variety of materials such as glass or plastic. The container holds or contains a composition comprising the cancer medicament as the active agent and may have a sterile access port (e.g , the container may be an intravenous solution bag or a vial having a stopper pierceable by a hypodermic injection needle).

The article of manufacture may further include a second container comprising a pharmaceutically-acceptable diluent buffer, such as bacteriostatic water for injection (BWFI), phosphate-buffered saline, Ringer's solution, and/or dextrose solution. The article of manufacture may further include other materials desirable from a commercial and user standpoint, including other buffers, diluents, filters, needles, and syringes.

The article of manufacture of the present invention also includes information, for example in the form of a package insert, indicating that the composition is used for treating cancer, as described herein. The insert or label may take any form, such as paper or on electronic media such as a magnetically recorded medium (e.g., floppy disk), a CD-ROM, a Universal Serial Bus (USB) flash drive, and the like. The label or insert may also include other information concerning the pharmaceutical compositions and dosage forms in the kit or article of manufacture.

The specification is considered to be sufficient to enable one skilled in the art to practice the invention. Various modifications of the invention in addition to those shown and described herein will become apparent to those skilled in the art from the foregoing description and fall within the scope of the appended claims. All publications, patents, and patent applications cited herein are hereby incorporated by reference in their entirety for all purposes.

### EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

### Example 1: Comprehensive genomic profiling (CGP) of DSRCT identifies predicted neoantigenic gene fusions

DSRCT is a rare and aggressive class of sarcomas characterized by an *FWSR1-WT1* gene fusion. Treatment of patients (pts) with DSRCT is limited to chemo-radiotherapy and surgical resection due, in part, to a paucity of actionable genomic alterations (GAs). Despite this aggressive therapy, these pts have a 5-year survival rate of 15-25% Recently, Yang et al. (Nat. Med. 25:767-775 (2019)) have highlighted that gene fusions can be a source of immunogenic neoantigens and identified an exceptional responder to immune checkpoint inhibition (ICI), despite having a low tumor mutational burden (TMB).

This Example describes the use of CGP to analyze the predicted neoantigenicity of the *FWSR1-WT1* fusion in DSRCT in order to elucidate the potential of ICI as a therapeutic option for patients with this disease.

### Materials and Methods

DSRCT was defined by central path review and the presence of the pathognomonic *EWSR1-WT1* fusion. Genomic DNA (≥50 ng) and RNA (≥3.5 ng/µL) were extracted from 40 µm of formalin-fixed paraffin-embedded (FFPE) sections. 109 DSRCTs were evaluated by hybrid capture-based CGP of 465 genes on DNA with additional fusion detection by RNA-seq of 265 genes. 5 samples did not have definitive *EWSR1-WT1* breakpoints and were filtered out, leaving a total of 104 *EWSR1-WT1* positive DSRCT for subsequent analyses.

Hybrid capture-based sequencing was performed using adaptor ligation-based libraries (Frampton, G.M. et al. (2013) Nat. Biotech. 31:1023-1031). Mean coverage depth was >600X. Base substitutions, insertions and deletions (short variants; SV), rearrangements (RE), and copy number (CN) changes were assessed.

Tumor mutational burden (TMB) was calculated from 1.2 Mb sequenced DNA, and microsatellite instability (MSI) was measured from 114 loci. Ancestry was inferred from over 50,000 SNPs that were previously characterized in the 1000 Genomes Project. HLA typing was performed using the OptiType HLA genotyping algorithm (Szolek, A. et al. (2014) Bioinformatics 30:3310-3316), and neoantigens were predicted using the NetMHCpan v4.0 (IEDB) MHC-I binding prediction tool as previously described (Hartmaier, R.J. et al. (2017) Genome Med. 9:16). Each HLA-A, HLA-B, and HLA-C allele was assessed for loss-of-heterozygosity (LOH) using the framework described in Sun, J.X. et al. (2018) PLoS Comput. Biol. 14:el005965.

### Results

In this cohort, DSRCT pts were young (median age: 25; range 6-55; FIG. 1A) and primarily male (79.8% of DSRCT pts vs. 46% of other sarcoma pts; OR:4.6, p<3e-12; FIG. 1B). Using a SNP-based ancestry calling algorithm, pts of African (AFR) ancestry were overrepresented in DSRCT (24% of DSRCT pts vs. 9.7% of other sarcoma pts; OR:3.0, p<2e-5; **FIG. 1C**).

As shown in FIG. 2A, DSCRT never harbored a high TMB (median 1.61 mut/Mb; range 0-9.7) and were never MSI. Outside of the *EWSR1-WT1* fusion, molecular characterization of DSRCT identified an average of 1.21 pathogenic GAs. A histogram of number of known or likely pathogenic GAs per sample is shown in **FIG. 2B** (including the pathognomonic *EWSR1-WT1* rearrangement). Frequency of known or likely pathogenic variant types is shown in **FIG. 2C**.

86.5% of *EWR1-WT1* fusions were formed through breakpoints in intron 7 of *EWSR1* and intron 7 of *VT1* (**FIG. 3A**). In the most common fusion product, exons 1-7 of *EWSR1* (5'end) were joined with exons 8-10 of *WT1* (3' end), resulting in a novel peptide sequence at the joining point (**FIG. 3B**). This peptide sequence was predicted to generate a neoantigen that bound strongly (<50 nM binding affinity) to two HLA types in 11.5% of all DSRCT pts (**FIG. 3C**). Outside of this, recurrent breakpoints were also found in intron 8 (4.8%), intron 9 (2.8%), and exon 7 (3.8%) of *EWSR1* (**FIG. 3A**). DSRCT never presented with pathogenic alterations in assessable genes that are critical for MHC class I presentation (*CIITA, B2M*)*.* Only 1 of the 9 assessable DSRCT had LOH in a strongly bound HLA type.

In summary, these data indicate that DSRCT patients tend to be young (median age:25) and predominantly male (79.8%). Ancestry calling from CGP corroborated previous reports of high (>20%) African-descended population. Examination of the DSRCT driver *EWSR1-WT1* gene fusions unveiled 11.5% of patients with a strong predicted fusion-associated neoantigen that may benefit from ICI. Given the highly recurrent breakpoint (86.5%), which results in a consistent and novel fusion peptide sequence, these data indicate that CGP can identify pts with high peptide-MHC binding affinity that may benefit from the rational use of immunotherapies such as immune checkpoint inhibitors and/or cancer vaccine approaches.

### Example 2: Scaling up identification of fusion neoantigens in sarcomas

The methods described in Example 1 were applied to other sarcomas in addition to DSRCT. These analyses were limited to samples in which DNA support existed for gene rearrangements.

From these samples, precise gene fusions were mapped. Mapped gene alignments were generated from sorted .bam files, and rearrangements were pulled with single base resolution. Peptide sequences were then inferred, and total score and binding affinity of neoantigens were predicted using the NetMHCpan v4.0 (IEDB) MHC-I binding prediction tool as previously described (Hartmaier, R.J. et al. (2017) Genome Med. 9:16).

As shown in **FIGS. 4A-4C**, 1238 samples from a variety of sarcomas were found to have at least one known or likely rearrangement with DNA support, and 1139 samples had at least one precision breakpoint called. When limited to variants without an intergenic partner and with a coding rearrangement/fusion, 988 rearrangements remained. These results were further limited to variants with a predicted fusion CDS that is divisible by 3, leaving 576 rearrangements remaining. Neoantigen predictions were made in ~439 samples.

As shown in **FIGS. 5A-5C**, among assessed samples, 106 had a predicted fusion neoantigen. Top sarcomas with a neoantigenic gene fusion (by count) were: soft tissue sarcomas (n=57; 53.8%), Ewing sarcomas (n=24; 22.6%), bone sarcoma (n=8; 7.5%), and leoimyosarcoma (n=8; 7.5%).

*EWSR1* rearrangements were found to account for the majority of predicted fusion neoantigens (**FIG. 6A**). 55.9% of rearrangements with predicted fusion neoantigens were found to be *EWSR1* rearrangements, including 29.03% *EWSR1-WT1* gene fusions and 23.66% *EWSRI-FLI1* gene fusions. Other rearrangement partners included *ERG, FEV, NR4A3, ATF1, CREB1, CREM, CREB3L1, CREB3L2, PATZ1, NFATC2, KLF15, C11orf93, ZNF444, PBX1, DDIT3,* and *TFCP2* (**FIG. 6B**).
Aspects of the invention are disclosed in the following numbered clauses:
1. A method of detecting a fusion gene encoding a neoantigen, the method comprising detecting presence of a Ewing sarcoma breakpoint region 1 - Wilms tumor protein (*EWSR1-WT1*) fusion gene encoding a neoantigen in a sample from an individual.
2. A method of identifying an individual having cancer who may benefit from a treatment comprising a cancer immunotherapy, the method comprising detecting presence of a Ewing sarcoma breakpoint region 1 - Wilms tumor protein (*EWSR1-WT1*) fusion gene encoding a neoantigen in a sample from the individual, wherein the presence of an *EWSR1-WT1* fusion gene encoding a neoantigen in the sample identifies the individual as one who may benefit from a treatment comprising a cancer immunotherapy.
3. A method of selecting a therapy for an individual having cancer, the method comprising detecting presence of a Ewing sarcoma breakpoint region 1 - Wilms tumor protein (*EWSR1-WT1*) fusion gene encoding a neoantigen in a sample from the individual, wherein the presence of an *EWSR1-WT1* fusion gene encoding a neoantigen in the sample identifies the individual as one who may benefit from a treatment comprising a cancer immunotherapy.
4. A method of identifying one or more treatment options for an individual having cancer, the method comprising:
   (a) detecting presence of a Ewing sarcoma breakpoint region 1 - Wilms tumor protein (*EWSRI-WT1*) fusion gene encoding a neoantigen in a sample from the individual; and
   (b) generating a report comprising one or more treatment options identified for the individual based at least in part on the presence of the *EWSR1-WT1* fusion gene encoding a neoantigen in the sample, wherein the one or more treatment options comprise a treatment comprising a cancer immunotherapy.
5. A method of identifying one or more treatment options for an individual having cancer, the method comprising:
   (a) acquiring knowledge of a Ewing sarcoma breakpoint region 1 - Wilms tumor protein (*HWSR1-WT1*) fusion gene encoding a neoantigen in a sample from the individual; and
   (b) generating a report comprising one or more treatment options identified for the individual based at least in part on said knowledge, wherein the one or more treatment options comprise a treatment comprising a cancer immunotherapy.
6. The method of clause 4 or clause 5, wherein the report further comprises a score that associates the one or more treatment options with a predicted outcome and/or response.
7. A method of selecting treatment for an individual having cancer, comprising acquiring knowledge of a Ewing sarcoma breakpoint region 1 - Wilms tumor protein (*EWSR1-WT1*) fusion gene encoding a neoantigen in a sample from an individual having cancer, wherein responsive to the acquisition of said knowledge: (i) the individual is classified as a candidate to receive a treatment comprising a cancer immunotherapy; and/or (ii) the individual is identified as likely to respond to a treatment that comprises a cancer immunotherapy.
8. A method of treating or delaying progression of cancer, comprising administering to an individual an effective amount of a treatment comprising a cancer immunotherapy, wherein the cancer comprises a Ewing sarcoma breakpoint region 1 - Wilms tumor protein (*EWSR1-WT1*) fusion gene encoding a neoantigen.
9. A method of treating or delaying progression of cancer, comprising, responsive to knowledge of a Ewing sarcoma breakpoint region 1 - Wilms tumor protein (*EWSR1-WT1*) fusion gene encoding a neoantigen in a sample from an individual, administering to the individual an effective amount of a treatment comprising a cancer immunotherapy.
10. The method of clause 8 or clause 9, wherein an *EWSR1-WT1* fusion gene encoding a neoantigen has been detected in a sample from the cancer of the individual prior to administration of the treatment.
11. The method of clause 8 or clause 9, further comprising, prior to administration of the treatment, detecting an *EWSR1-WT1* fusion gene encoding a neoantigen in a sample from the cancer of the individual.
12. A method of treating or delaying progression of cancer, comprising:
   (a) detecting a Ewing sarcoma breakpoint region 1 - Wilms tumor protein (*EWSR1-WT1*) fusion gene encoding a neoantigen in a sample from an individual; and
   (b) administering to the individual an effective amount of a treatment comprising a cancer immunotherapy.
13. A method of treating or delaying progression of cancer, comprising:
   (a) acquiring knowledge of a Ewing sarcoma breakpoint region 1 - Wilms tumor protein (*EWSR1-WT1*) fusion gene encoding a neoantigen in a sample from an individual; and
   (b) responsive to said knowledge, administering to the individual an effective amount of a treatment comprising a cancer immunotherapy.
14. The method of any one of clauses 1-4 and 10-12, further comprising, prior to detecting the presence of an *EWSR1-WT1* fusion gene encoding a neoantigen in the sample, obtaining the sample from the individual.
15. The method of any one of clauses 1-7, 9-12, and 14, wherein the sample is a blood, bone marrow, tumor, or tissue sample.
16. The method of any one of clauses 1-7, 9-12, and 14, wherein the sample is from amniotic fluid, blood, plasma, serum, semen, lymphatic fluid, cerebral spinal fluid, ocular fluid, urine, saliva, stool, mucus, sweat, blood, skin, hair, hair follicles, saliva, oral mucous, vaginal mucus, sweat, tears, epithelial tissues, urine, semen, seminal fluid, seminal plasma, prostatic fluid, Cowper's fluid, excreta, biopsy, ascites, cerebrospinal fluid, or lymph.
17. The method of clause 15 or clause 16, wherein the sample is a biopsy or formalin-fixed paraffin-embedded (FFPE) sample.
18. The method of any one of clauses 1-7 and 9-17, wherein the presence of an *EWSR1-WT1* fusion gene is detected in DNA or RNA from the sample.
19. The method of clause 18, wherein the presence of an *EWSR1-WT1* fusion gene is detected in cell-free DNA (cfDNA).
20. The method of clause 18 or clause 19, wherein the presence of an *EWSR1-WT1* fusion gene is detected in DNA by next-generation sequencing (NGS).
21. The method of clause 18 or clause 19, wherein the presence of an *EWSR1-WT1* fusion gene is detected in DNA by polymerase chain reaction (PCR), Sanger sequencing, or fluorescence *in situ* hybridization (FISH).
22. The method of clause 18, wherein the presence of an *EWSR1-WT1* fusion gene is detected in RNA by RNA-sequencing (RNA-seq).
23. The method of clause 18, wherein the presence of an *EWSR1-WT1* fusion gene is detected in RNA by polymerase chain reaction (PCR), Sanger sequencing, or fluorescence *in situ* hybridization (FISH).
24. The method of any one of clauses 1-23, wherein the neoantigen binds, or is predicted to bind, a major histocompatibility complex (MHC) class I molecule of the individual.
25. The method of clause 24, wherein the neoantigen binds, or is predicted to bind, an.MHC class I molecule of the individual with a dissociation constant (K_{D}) of 50 nM or less.
26. The method of clause 24 or clause 25, further comprising: genotyping one or more human leukocyte antigen (HLA)-A, HLA-B, and HLA-C alleles from the sample from the individual.
27. The method of any one of clauses 1-26, wherein the cancer does not comprise a loss-of-heterozygosity at an HLA-A, HLA-B, or HLA-C allele that encodes an MHC class I molecule to which the neoantigen binds, or is predicted to bind.
28. The method of any one of clauses 1-26, wherein the cancer comprises a loss-of-heterozygosity at one or more, but not all, of HLA-A, HLA-B, or HLA-C allele(s) that encode an MHC class I molecule to which the neoantigen binds, or is predicted to bind.
29. The method of any one of clauses 1-28, wherein the cancer does not comprise a loss- . of- function mutation in a *CIITA* or *B2M* gene.
30. The method of any one of clauses 1-7 and 9-29, further comprising genotyping a *CIITA* and/or *B2M* gene from the sample from the individual.
31. The method of any one of clauses 1-7 and 9-30, further comprising determining a tumor mutational burden (TMB) from the sample from the
32. The method of any one of clauses 1-31, wherein the cancer is not characterized by a high tumor mutational burden (TMB).
33. The method of any one of clauses 1-31, wherein the cancer is characterized by a high tumor mutational burden (TMB).
34. The method of any one of clauses 1-33, wherein the cancer is not characterized by microsatellite instability (MSI).
35. The method of any one of clauses 1-33, wherein the cancer is characterized by microsatellite instability (MST).
36. The method of any one of clauses 1-7 and 9-35, further comprising determining microsatellite instability (MSI) from the sample from the individual.
37. The method of any one of clauses 1-36, wherein the cancer is a desmoplastic small round cell tumor (DSRCT).
38. The method of clause 37, wherein the DSRCT is characterized by a low TMB.
39. The method of clause 38, wherein the DSRCT has a TMB of about 20 mutations/Mb or less.
40. The method of clause 39, wherein the DSRCT has a TMB of about 10 mutations/Mb or less.
41. The method of any one of clauses 37-40, wherein the DSRCT is not characterized by microsatellite instability (MSI).
42. The method of any one of clauses 1-36, wherein the cancer is a sarcoma.
43. The method of clause 42, wherein the sarcoma is selected from the group consisting of synovial sarcomas, ewing sarcomas, clear cell sarcomas, solitary fibrous tumors, myxoid liposarcomas, mesenchymal chondrosarcomas, uterine endometrial stromal sarcomas, undifferentiated round cell/ewing-like sarcomas, alveolar soft part sarcomas, alveolar rhabdomyosarcomas, inflammatory myofibroblastic tumors, extraskeletal myxoid chondrosarcomas, epithelioid hemangioendotheliomas, low grade fibromyxoid sarcomas, sclerosing epithelioid fibrosarcomas, dermatofibrosarcoma protuberans, and ossifying fibromyxoid tumors.
44. The method of any one of clauses 1-36, wherein the cancer is a carcinoma.
45. The method of clause 44, wherein the cancer is a NUT midline carcinoma.
46. The method of any one of clauses 1-36, wherein the cancer is selected from the group consisting of lung cancer, kidney cancer, a bladder cancer, breast cancer, colorectal cancer. ovarian cancer, pancreatic cancer, gastric carcinoma, esophageal cancer, mesothelioma, melanoma, head and neck cancer, thyroid cancer, , prostate cancer, glioblastoma, cervical cancer, thymic carcinoma, leukemia, lymphoma, myeloma, mycoses fungoides, merkel cell cancer, hematologic malignancy, cancer of hematological tissues. B cell cancer, bronchus cancer, stomach cancer, brain or central nervous system cancer, peripheral nervous system cancer, uterine or endometrial cancer, cancer of the oral cavity or pharynx, liver cancer, testicular cancer, biliary tract cancer, small bowel or appendix cancer, salivary gland cancer, adrenal gland cancer, adenocarcinoma, inflammatory myofibroblastic tumor, gastrointestinal stromal tumor (GIST), colon cancer, myelodysplastic syndrome (MDS), myeloproliferative disorder (MPD), polycythemia Vera, chordoma, synovioma, Ewing's tumor, squamous cell carcinoma, basal cell carcinoma, adenocarcinoma, sweat gland carcinoma, sebaceous gland carcinoma, papillary carcinoma, papillary adenocarcinoma, medullary carcinoma, bronchogenic carcinoma, renal cell carcinoma, hepatoma, bile duct carcinoma, choriocarcinoma, seminoma, embryonal carcinoma. Wilms' tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, follicular lymphoma, diffuse large B-cell lymphoma, mantle cell lymphoma, hepatocellular carcinoma, thyroid cancer, small cell cancer, essential thrombocythemia, agnogenic myeloid metaplasia, hypereosinophilic syndrome, systemic mastocytosis, familiar hypereosinophilia, neuroendocrine cancer, and carcinoid tumor.
47. The method of any one of clauses 1-46, wherein the *EWSR1-WT1* fusion gene comprises a coding sequence of *EWSRI* at the 5' end and a coding sequence of *WT1* at the 3' end.
48. The method of any one of clauses 1-46, wherein the *EWSR1-WT1* fusion gene is formed via a breakpoint in intron 7, intron 8, intron 9, or exon 7 of *EWSRI.*
49. The method of any one of clauses 1-46, wherein the *EWSR1-WT1* fusion gene is formed via breakpoints in intron 7 of *EWSR1* and intron 7 of *WT1.*
50. The method of clause 49, wherein the *EWSRI-WT1* fusion gene encodes exons 1-7 of *EWSR1* and exons 8-10 of *WT1.*
51. The method of clause 49, wherein the *EWSR1-WT1* fusion gene encodes exons 1-7 of *EWSR1* and exons 7-9 of *WT1.*
52. The method of any one of clauses 1-51, wherein the neoantigen comprises at least 5 contiguous amino acids from the sequence SSYGQQSEK (SEQ ID NO:1).
53. The method of clause 52, wherein the neoantigen comprises 6, 7, 8, or 9 contiguous amino acids from the sequence SSYGQQSEK (SEQ ID NO:1).
54. The method of clause 52, wherein the neoantigen comprises 7, 8, or 9 contiguous amino acids from the sequence SSYGQQSEK (SEQ ID NO:1).
55. The method of clause 52, wherein the neoantigen comprises 8 or 9 contiguous amino acids from the sequence SSYGQQSEK (SEQ ID NO:1).
56. The method of clause 52, wherein the neoantigen comprises the sequence SSYGQQSEK (SEQ ID NO:1).
57. The method of any one of clauses 1-51, wherein the neoantigen comprises at least 5 contiguous amino acids from the sequence YGQQSEKPY (SEQ ID NO:2).
58. The method of clause 57, wherein the neoantigen comprises 6, 7, 8, or 9 contiguous amino acids from the sequence YGQQSEKPY (SEQ ID NO:2).
59. The method of clause 57, wherein the neoantigen comprises 7, 8, or 9 contiguous amino acids from the sequence YGQQSEKPY (SEQ ID NO:2).
60. The method of clause 57, wherein the neoantigen comprises 8 or 9 contiguous amino acids from the sequence YGQQSEKPY (SEQ ID NO:2).
61. The method of clause 57, wherein the neoantigen comprises the sequence YGQQSEKPY (SEQ ID NO:2).
62. The method of any one of clauses 1-61, wherein the *EWSR1-WT1* fusion gene encodes the sequence SSSYGQQSEKPYQCDF (SEQ ID NO:3).
63. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGEKPFSCRWPSCQKKFARSDELVRHHNM
HQRNMTKLQLAL (SEQ ID NO:4).
64. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
 
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:5).
65. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGEKPFSCRWPSCQKKFARSDELVRH
HNMHQRNMTKLQLAL (SEQ ID NO:6).
66. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGEKPFSCRWPSCQKKFARSDEL
VRHHNMHQRNMTKLQLAL (SEQ ID NO:7).
67. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
 
HTGVKPFQCKTCQRKFSRSDHLKTHTRTHTGEKPFSCRWPSCQKKFARSDELVRHHNM
HQRNMTKLQLAL (SEQ ID NO:8).
68. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTSYSSTQPTSYDQSSYSQQNTYGQPSSYGQQSSYGQQS
SYGQQPPTSYPPQTGSYSQAPSQYSQQSSSYGQQSEKPYQCDFKDCERRFSRSDQLKRH
QRRHTGVKPFQCKTCQRKFSRSDHLKTHTRTHTGKTSEKPFSCRWPSCQKKFARSDELV
RHHNMHQRNMTKLQLAL (SEQ ID NO:9).
69. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTVEGTSTGYTTPTAPQAYSQPVQGYGTGAYDTTTATVTTTQ
ASYAAQSAYGTQPAYPAYGQQPAATAPTRPQDGNKPTETSQPQSSTGGYNQPSLGYGQ
SNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSEKPYQCDFKDCERRFSRSDQL
KRHQRRHTGVKPFQCKTCQRKFSRSDHLKTHTRTHTGKTSEKPFSCRWPSCQKKFARS
DELVRHHNMHQRNMTKLQLAL (SEQ ID NO:10).
70. The method of any one of clauses 1-61, wherein the *EWSR1-WT1* fusion gene encodes a sequence that is at least 80% identical to the sequence 
MASTDYSTYSQAAAQQGYSAYTAQPTQGYAQTTQAYGQQSYGTYGQPTDVSYTQAQT
TATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYP
QVPGSYPMQPVTAPPSYPPTSYSSTQPTSYDQSSYSQQNTYGQPSSYGQQSSYGQQSSYG
QQPPTSYPPQTGSYSQAPSQYSQQSSSYGQQSEKPYQCDFKDCERRFSRSDQLKRHQRR
HTGVKPFQCKTCQRKFSRSDHLKTHTRTHTGKTSEKPFSCRWPSCQKKFARSDELVRHH
NMHQRNMTKLQLAL (SEQ ID NO:11).
71. The method of any one of clauses 1-70, further comprising detecting one or more additional mutations in the cancer or sample.
72. The method of clause 71, wherein the one or more additional mutations are in one or more genes other than *EWSR1* and *WT1.*
73. The method of clause 71 or clause 72, wherein the one or more additional mutations are in one or more genes selected from the group consisting of ABL1, BRAF, CDKN1A, EPHA3, FGFR4, IKZF1, MCL1, NKX2-1, PMS2, RNF43, TET2, ACVRIB, BRCA1, CDKN1B, EPHB1, FH, INPP4B, MDM2, NOTCH1, POLD1, ROS1, TGFBR2, AKT1, BRCA2, CDKN2A, EPHB4, FLCN, IRF2, MDM4, NOTCH2, POLE, RPTOR, TIPARP, AKT2, BRD4, CDKN2B, ERBB2, FLT1, IRF4, MED12, NOTCH3, PPARG, SDHA, TNFAIP3, AKT3, BRIPI, CDKN2C, ERBB3, FLT3, IRS2, MEF2B, NPM1, PPP2R1A, SDHB, TNFRSF14, ALK, BTG1, CEBPA, ERBB4, FOXL2, JAK1, MEN1, NRAS, PPP2R2A, SDHC, TP53, ALOX12B, BTG2, CHEK1, ERCC4, FUBP1, JAK2, MERTK. NT5C2, PRDM1, SDHD, TSC1, A MER1, BTK, CHEK2, ERG. GABRA6, JAK3, MET, NTRK1, PRKAR1A, SETD2. TSC2, APC, C11orf30, CIC, ERRFI1, GATA3, JUN, MITF, NTRK2, PRKCI, SF3B1, TYRO3, AR, CALR, CREBBP, ESR1, GATA4, KDM5A. MKNKI, NTRK3, PTCH1, SGK1, U2AF1, ARAF, CARD11, CRKL, EZH2, GATA6, KDM5C, MLH1, P2RY8, PTEN, SMAD2, VEGFA, ARFRP1, CASP8, CSF1R, FAM46C, GID4, (C17orf39), KDM6A, MPL, PALB2, PTPN11, SMAD4, VHL, ARID1A, CBFB, CSF3R, FANCA, GNA11, KDR, MRE 11A, PARK2, PTPRO, SMARCA4, WHSC1, ASXL1, CBL, CTCF, FANCC, GNA13, KEAP1, MSH2, PARP1, QKI, SMARCB1, WHSC1L1, ATM, CCNDI, CTNNA1, FANCG, GNAQ, KEL, MSH3, PARP2, RAC1, SMO, WT1, ATR, CCND2, CTNNB1, FANCL, GNAS, KIT, MSH6, PARP3, RAD21, SNCAIP, XPO1, ATRX, CCND3, CUL3, FAS, GRM3, KLHL6, MST1R, PAX5, RAD51, SOCS1, XRCC2, AURKA, CCNE1, CUL4A, FBXW7, GSK3B, KMT2A, (MLL), MTAP, PBRM1, RAD51B, SOX2, ZNF217, AURKB, CD22, CXCR4, FGF10, H3F3A, KMT2D, (MLL2), MTOR, PDCD1, RAD51C, SOX9, ZNF703, AXIN1, CD274, CYP17A1, FGF12, HDAC1, KRAS, MUTYH, PDCD1LG2, RAD51D, SPEN, AXL, CD70, DAXX, FGF14, HGF, LTK, MYC, PDGFRA, RAD52, SPOP, BAP1, CD79A, DDR1, FGF19, HNF1A, LYN, MYCL, PDGFRB, RAD54L, SRC, BARD1, CD79B, DDR2, FGF23, HRAS, MAF, MYCN, PDK1, RAF1, STAG2, BCL2, CDC73, DIS3, FGF3, HSD3B1, MAP2K1, MYD88, PIK3C2B, RARA, STAT3, BCL2L1, CDH1, DNMT3A, FGF4, ID3, MAP2K2, NBN, PIK3C2G, RB1, STK11, BCL2L2, CDK12, DOT1L, FGF6, IDH1, MAP2K4, NF1, PIK3CA, RBM10, SUFU, BCL6, CDK4, EED, FGFR1, IDH2, MAP3K1, NF2, PIK3CB, REL, SYK, BCOR, CDK6, EGFR, FGFR2, IGF1R, MAP3K13, NFE2L2, PIK3R1, RET, TBX3, BCORL1, CDK8, EP300, FGFR3, IKBKE, MAPK1, NFKBIA, PIM1, RICTOR, TEK, BCR, CD74, ETV4, ETV5, ETV6, EWSR1, EZR, MYB, NUTM1, RSPO2, SDC4, SLC34A2, TERC, TERT, and TMPRSS2.
74. The method of any one of clauses 1-73, wherein the cancer immunotherapy comprises one or more of: a checkpoint inhibitor, cancer vaccine, cell-based therapy, T cell receptor (TCR)-based therapy, adjuvant immunotherapy, cytokine immunotherapy, and oncolytic virus therapy.
75. The method of clause 74, wherein the cancer immunotherapy comprises small molecule, nucleic acid, polypeptide, carbohydrate, toxin, cell-based, or binding agent therapeutic agent.
76. The method of clause 74, wherein the cancer immunotherapy comprises a checkpoint inhibitor.
77. The method of clause 76, wherein the checkpoint inhibitor targets PD-L1, PD-1, CTLA-4, CEACAM, LAIR1, CD160, 2B4, CD80, CD86, CD276, VTCN1, HVEM, KIR, A2AR, MHC class I, MHC class II, GALS, adenosine, TGFR, OX40, CD137, CD40, IDO, CSF1R, TIM-3, BTLA, VISTA, LAG-3, TIGIT, IDO, MICA/B, or arginase.
78. The method of clause 77, wherein the checkpoint inhibitor is an agent that inhibits PD-1.
79. The method of clause 78, wherein the agent that inhibits PD-1 is a small molecule, a nucleic acid, a polypeptide, carbohydrate, a lipid, a metal, or a toxin.
80. The method of clause 79, wherein the agent that inhibits PD-1 is a PD-1 binding antagonist.
81. The method of clause 80, wherein the PD-1 binding antagonist is an antibody, antibody-drug conjugate, antibody fragment, or immunoadhesin.
82. The method of clause 80, wherein the PD-1 binding antagonist is selected from the group consisting of pembrolinimab, nivolumab, cemiplimab, spartalizumab, genolimzumab, SHR1210, JS001, BGB-108, BGB-A317, IBI308, GLS-010, BMS-936558, BCD-100, REGN2810, MGA-012, BI 754091, STI-A1110, INCSHR-1210, PF-06801591, TSR-042, AM0001, JNJ-63723283, and ENUM 244C8.
83. The method of clause 77, wherein the checkpoint inhibitor is an agent that inhibits PD-L1 and/or PD-L2.
84. The method of clause 83, wherein the agent that inhibits PD-L1 and/or PD-L2 is a small molecule, a nucleic acid, a polypeptide, carbohydrate, a lipid, a metal, or a toxin.
85. The method of clause 84, wherein the agent that inhibits PD-L1 is a PD-L1 binding antagonist.
86. The method of clause 85, wherein the PD-L1 binding antagonist is an antibody, antibody- drug conjugate, antibody fragment, or immunoadhesin.
87. The method of clause 85, wherein the PD-L1 binding antagonist is selected from the group consisting of atezolizumab, avelumab, durvalumab, KN035, CS1001, MDX-1 105, LY3300054, STI-A1014, FAZ053, and CX-072.
88. The method of clause 77, wherein the checkpoint inhibitor is an agent that inhibits CTLA4.
89. The method of clause 88, wherein the agent that inhibits CTLA4 is a small molecule, a nucleic acid, a polypeptide, carbohydrate, a lipid, a metal, or a toxin.
90. The method of clause 89, wherein the agent that inhibits CTLA4 is an antibody, antibody- drug conjugate, antibody fragment, or immunoadhesin.
91. The method of clause 89, wherein the agent that inhibits CTLA4 is selected from the group consisting of ipilimumab, APL-509, AGEN1884, and CS1002.
92. The method of any one of clauses 1-91, further comprising administering an additional anti-cancer therapy to the individual.
93. The method of clause 92, wherein the additional anti-cancer therapy comprises one or more of surgery, radiotherapy, chemotherapy, anti-angiogenic therapy, anti-DNA repair therapy, and anti-inflammatory therapy.
94. The method of clause 93, wherein the additional anti-cancer therapy comprises a chemotherapeutic agent.
95. The method of clause 94, wherein the chemotherapeutic agent is a platinum-based chemotherapeutic agent.
96. The method of any one of clauses 1-91, further comprising administering an additional cancer immunotherapy to the individual.
97. The method of clause 1, wherein the *EWSR1-WT1* fusion gene encoding the neoantigen is detected using one or more oligonucleotides.
98. The method of clause 97, wherein the *EWSR1-WT1* fusion gene encoding the neoantigen is detected by PCR amplification of a DNA or RNA sequence encoding the amino acid sequence of any one of SEQ ID Nos:1-11.
99. The method of clause 97, wherein the *EWSR1-WT1* fusion gene encoding the neoantigen is detected by *in situ* hybridization of a DNA or RNA oligonucleotide with a sequence encoding the amino acid sequence of any one of SEQ ID Nos:1-11.
100. The method of any one of clauses 97-99, further comprising administering a cancer immunotherapy to the individual.
101. A kit comprising one or more oligonucleotides, wherein the one or more oligonucleotides hybridize with at least a portion of a DNA or RNA sequence encoding the amino acid sequence of any one of SEQ ID Nos:1-11.
102. A cancer immunotherapy for use in a method of treating or delaying progression of cancer, wherein the method comprises administering the cancer immunotherapy to an individual, and wherein a Ewing sarcoma breakpoint region 1 - Wilms tumor protein (*EWSR1-WT1*) fusion gene encoding a neoantigen has been detected in a sample obtained from the individual.
103. A cancer immunotherapy for use in the manufacture of a medicament for treating or delaying progression of cancer, wherein the medicament is to be administered to an individual, wherein a Ewing sarcoma breakpoint region 1 - Wilms tumor protein (*EWSR1-WT1*) fusion gene encoding a neoantigen has been detected in a sample obtained from the individual.
104. An isolated polypeptide comprising an amino acid sequence that is at least 80% identical to a sequence selected from the group consisting of SEQ ID Nos:1-11.
105. An isolated polypeptide comprising an amino acid sequence selected from the group consisting of SEQ ID Nos:1-11.
106. An isolated polynucleotide encoding the isolated polypeptide of clause 104 or clause 105.
107. A vector comprising the polynucleotide of clause 106.
108. A composition comprising the isolated polypeptide of clause 104 or clause 105, the isolated polynucleotide of clause 106, or the vector of clause 107.
109. A method of detecting a fusion gene encoding a neoantigen, the method comprising detecting presence of a Ewing sarcoma breakpoint region 1 (*EWSR1*) fusion gene encoding a neoantigen in a sample from an individual.
110. A method of identifying an individual having cancer who may benefit from a treatment comprising a cancer immunotherapy, the method comprising detecting presence of a Ewing sarcoma breakpoint region 1 (*EWSR1*) fusion gene encoding a neoantigen in a sample from the individual, wherein the presence of an *EWSR1* fusion gene encoding a neoantigen in the sample identifies the individual as one who may benefit from a treatment comprising a cancer immunotherapy.
111. A method of selecting a therapy for an individual having cancer, the method comprising detecting presence of a Ewing sarcoma breakpoint region 1 (*EWSR1*) fusion gene encoding a neoantigen in a sample from the individual, wherein the presence of an *EWSR1* fusion gene encoding a neoantigen in the sample identifies the individual as one who may benefit from a treatment comprising a cancer immunotherapy.
112. A method of identifying one or more treatment options for an individual having cancer, the method comprising:
   (a) detecting presence of a Ewing sarcoma breakpoint region 1 (*EWSR1*) fusion gene encoding a neoantigen in a sample from the individual; and
   (b) generating a report comprising one or more treatment options identified for the individual based at least in part on the presence of the *EWSR1* fusion gene encoding a neoantigen in the sample, wherein the one or more treatment options comprise a treatment comprising a cancer immunotherapy.
113. A method of identifying one or more treatment options for an individual having cancer, the method comprising:
   (a) acquiring knowledge of a Ewing sarcoma breakpoint region 1 (*EWSR1*) fusion gene encoding a neoantigen in a sample from the individual; and
   (b) generating a report comprising one or more treatment options identified for the individual based at least in part on said knowledge, wherein the one or more treatment options comprise a treatment comprising a cancer immunotherapy.
114. The method of clause 112 or clause 113, wherein the report further comprises a score that associates the one or more treatment options with a predicted outcome and/or response.
115. A method of selecting treatment for an individual having cancer, comprising acquiring knowledge of a Ewing sarcoma breakpoint region 1 (*EWSR1*) fusion gene encoding a neoantigen in a sample from an individual having cancer, wherein responsive to the acquisition of said knowledge: (i) the individual is classified as a candidate to receive a treatment comprising a cancer immunotherapy; and/or (ii) the individual is identified as likely to respond to a treatment that comprises a cancer immunotherapy.
116. A method of treating or delaying progression of cancer, comprising administering to an individual an effective amount of a treatment comprising a cancer immunotherapy, wherein the cancer comprises a Ewing sarcoma breakpoint region 1 (*EWSR1*) fusion gene encoding a neoantigen.
117. A method of treating or delaying progression of cancer, comprising, responsive to knowledge of a Ewing sarcoma breakpoint region 1 (*EWSR1*) fusion gene encoding a neoantigen in a sample from an individual, administering to the individual an effective amount of a treatment comprising a cancer immunotherapy.
118. The method of clause 116 or clause 117, wherein an *EWSR1* fusion gene encoding a neoantigen has been detected in a sample from the cancer of the individual prior to administration of the treatment.
119. The method of clause 116 or clause 117, further comprising, prior to administration of the treatment, detecting an *EWSR1* fusion gene encoding a neoantigen in a sample from the cancer of the individual.
120. A method of treating or delaying progression of cancer, comprising:
   (a) detecting a Ewing sarcoma breakpoint region 1 (*EWSR1*) fusion gene encoding a neoantigen in a sample from an individual; and
   (b) administering to the individual an effective amount of a treatment comprising a cancer immunotherapy.
121. A method of treating or delaying progression of cancer, comprising:
   (a) acquiring knowledge of a Ewing sarcoma breakpoint region 1 (*EWSR1*) fusion gene encoding a neoantigen in a sample from an individual; and
   (b) responsive to said knowledge, administering to the individual an effective amount of a treatment comprising a cancer immunotherapy.
122. The method of any one of clauses 109-121, wherein the *EWSR1* fusion gene is a fusion gene between *EWSR1* and one of: *FLII*, *ERG, FEV, NR4A3, ATFI, CREB1, CREM, CREB3L1, CREB3L2, PATZ1, NFATC2, KLF15, C11orf93, ZNF444, PBX1, DDIT3,* and *TFCP2.*
123. The method of any one of clauses 109-112, 118-120, and 122, further comprising, prior to detecting the presence of an *EWSR1* fusion gene encoding a neoantigen in the sample, obtaining the sample from the individual.
124. The method of any one of clauses 109-115, 117-120, 122, and 123, wherein the sample is a blood, bone marrow, tumor, or tissue sample.
125. The method of any one of clauses 109-115, 117-120, 122, and 123, wherein the sample is from amniotic fluid, blood, plasma, serum, semen, lymphatic fluid, cerebral spinal fluid, ocular fluid, urine, saliva, stool, mucus, sweat, blood, skin, hair, hair follicles, saliva, oral mucous, vaginal mucus, sweat, tears, epithelial tissues, urine, semen, seminal fluid, seminal plasma, prostatic fluid, Cowper's fluid, excreta, biopsy, ascites, cerebrospinal fluid, or lymph.
126. The method of clause 124 or clause 125, wherein the sample is a biopsy or formalin-fixed paraffin-embedded (FFPE) sample.
127. The method of any one of clauses 109-115 and 117-126, wherein the presence of an *EWSR1* fusion gene is detected in DNA or RNA from the sample.
128. The method of clause 127, wherein the presence of an *EWSR1* fusion gene is detected in cell-free DNA (cfDNA).
129. The method of clause 127 or clause 128, wherein the presence of an *EWSR1-WT1* fusion gene is detected in DNA by next-generation sequencing (NGS).
130. The method of clause 127 or clause 128, wherein the presence of an *EWSR1* fusion gene is detected in DNA by polymerase chain reaction (PCR), Sanger sequencing, or fluorescence *in situ* hybridization (FISH).
131. The method of clause 127, wherein the presence of an *EWSR1* fusion gene is detected in RNA by RNA-sequencing (RNA-seq).
132. The method of clause 127, wherein the presence of an *EWSR1* fusion gene is detected in RNA by polymerase chain reaction (PCR), Sanger sequencing, or fluorescence *in situ* hybridization (FISH).
133. The method of any one of clauses 109-132, wherein the neoantigen binds, or is predicted to bind, a major histocompatibility complex (MHC) class I molecule of the individual.
134. The method of clause 133, wherein the neoantigen binds, or is predicted to bind, an MHC class I molecule of the individual with a dissociation constant (K_{D}) of 50 nM or less.
135. The method of clause 133 or clause 134, further comprising: genotyping one or more human leukocyte antigen (HLA)-A, HLA-B, and HLA-C alleles from the sample from the individual.
136. The method of any one of clauses 109-135, wherein the cancer does not comprise a loss-of-heterozygosity at an HLA-A, HLA-B, or HLA-C allele that encodes an MHC class I molecule to which the neoantigen binds, or is predicted to bind.
137. The method of any one of clauses 109-135, wherein the cancer comprises a loss-of-heterozygosity at one or more, but not all, of HLA-A, HLA-B, or HLA-C allele(s) that encode an MHC class I molecule to which the neoantigen binds, or is predicted to bind.
138. The method of any one of clauses 109-137, wherein the cancer is Ewing sarcoma, extraskeletal myxoid chondrosarcoma, clear cell sarcoma, low-grade fibromyxoid sarcoma/sclerosing epithelioid fibrosarcoma, undifferentiated round cell sarcoma/Ewing-like, sarcoma not otherwise specified (NOS), rare sarcoma, myxoid liposarcoma, or rhabdomyosarcoma NOS.
139. The method of any one of clauses 109-138, wherein the cancer immunotherapy comprises one or more of: a checkpoint inhibitor, cancer vaccine, cell-based therapy, T cell receptor (TCR)-based therapy, adjuvant immunotherapy, cytokine immunotherapy, and oncolytic virus therapy.
140. The method of clause 139, wherein the cancer immunotherapy comprises small molecule, nucleic acid, polypeptide, carbohydrate, toxin, cell-based, or binding agent therapeutic agent.
141. The method of clause 139, wherein the cancer immunotherapy comprises a checkpoint inhibitor.
142. The method of clause 141, wherein the checkpoint inhibitor targets PD-L1, PD-1, CTLA-4, CEACAM, LAIR1, CD160, 2B4, CD80, CD86, CD276, VTCN1, HVEM, KIR, A2AR, MHC class I, MHC class II, GALS, adenosine, TGFR, OX40, CD137, CD40, IDO, CSF1R, TIM-3, BTLA, VISTA, LAG-3, TIGIT, IDO, MICA/B, or arginase.
143. The method of clause 142, wherein the checkpoint inhibitor is an agent that inhibits PD-1.
144. The method of clause 143, wherein the agent that inhibits PD-1 is a small molecule, a nucleic acid, a polypeptide, carbohydrate, a lipid, a metal, or a toxin.
145. The method of clause 144, wherein the agent that inhibits PD-1 is a PD-1 binding antagonist.
146. The method of clause 145, wherein the PD-1 binding antagonist is an antibody, antibody-drug conjugate, antibody fragment, or immunoadhesin.
147. The method of clause 145, wherein the PD-1 binding antagonist is selected from the group consisting of pembrolizumab, nivolumab, cemiplimab, spartalizumab, genolimzumab, SHR1210, JS001, BGB-108, BGB-A317, IBI308, GLS-010, BMS-936558, BCD-100, REGN2810, MGA- 012, Bl 754091, STI-A1110, INCSHR-1210, PF-06801591, TSR-042, AM0001, JNJ-63723283, and ENUM 244C8.
148. The method of clause 142, wherein the checkpoint inhibitor is an agent that inhibits PD-L 1 and/or PD-L2.
149. The method of clause 148, wherein the agent that inhibits PD-L1 and/or PD-L2 is a small molecule, a nucleic acid, a polypeptide, carbohydrate, a lipid, a metal, or a toxin.
150. The method of clause 148, wherein the agent that inhibits PD-L1 is a PD-L1 binding antagonist.
151. The method of clause 150, wherein the PD-L1 binding antagonist is an antibody, antibody-drug conjugate, antibody fragment, or immunoadhesin.
152. The method of clause 150, wherein the PD-L1 binding antagonist is selected from the group consisting of atezolizumab, avelumab, durvalumab, KN035, CS1001, MDX-1 105, LY3300054, STI-A1014, FAZ053, and CX-072.
153. The method of clause 142, wherein the checkpoint inhibitor is an agent that inhibits CTLA4.
154. The method of clause 153, wherein the agent that inhibits CTLA4 is a small molecule, a nucleic acid, a polypeptide, carbohydrate, a lipid, a metal, or a toxin.
155. The method of clause 153, wherein the agent that inhibits CTLA4 is an antibody, antibody-drug conjugate, antibody fragment, or immunoadhesin.
156. The method of clause 153, wherein the agent that inhibits CTLA4 is selected from the group consisting of ipilimumab, APL-509, AGEN1884, and CS1002.
157. The method of any one of clauses 109-156, further comprising administering an additional anti-cancer therapy to the individual.
158. The method of clause 157, wherein the additional anti-cancer therapy comprises one or more of surgery, radiotherapy, chemotherapy, anti-angiogenic therapy, anti-DNA repair therapy, and anti-inflammatory therapy.
159. The method of clause 158, wherein the additional anti-cancer therapy comprises a chemotherapeutic agent.
160. The method of clause 159, wherein the chemotherapeutic agent is a platinum-based chemotherapeutic agent.
161. The method of any one of clauses 109-138, further comprising administering an additional cancer immunotherapy to the individual.
162. A cancer immunotherapy for use in a method of treating or delaying progression of cancer, wherein the method comprises administering the cancer immunotherapy to an individual, and wherein a Ewing sarcoma breakpoint region 1 (*EWSR1*) fusion gene encoding a neoantigen has been detected in a sample obtained from the individual.
163. A cancer immunotherapy for use in the manufacture of a medicament for treating or delaying progression of cancer, wherein the medicament is to be administered to an individual, wherein a Ewing sarcoma breakpoint region 1 (*EWSR1*) fusion gene encoding a neoantigen has been detected in a sample obtained from the individual.

## Claims

1. A method of identifying one or more treatment options for an individual having cancer, the method comprising:
a. acquiring knowledge of a Ewing sarcoma breakpoint region 1 - Wilms tumor protein (*EWSR1-WT1*) fusion gene encoding a neoantigen in a sample from the individual; and
b. generating a report comprising one or more treatment options identified for the individual based at least in part on said knowledge, wherein the one or more treatment options comprise a treatment comprising a cancer immunotherapy;
wherein the neoantigen binds, or is predicted to bind, a major histocompatibility complex (MHC) class I molecule of the individual.

2. The method according to claim 1, wherein the *EWSR1* fusion gene is a fusion gene between *EWSR1* and *WT1*, wherein the *EWSR1* fusion gene is formed via a breakpoint in intron 7, intron 8, intron 9, or exon 7 of *EWSR1.*

3. The method according to claim 1 or claim 2, wherein the *EWSR1-WT1* fusion gene is formed via breakpoints in intron 7 of *EWSR1* and intron 7 of *WT1.*

4. The method according to any one of claims 1 to 3, wherein the neoantigen comprises at least 5 contiguous amino acids from the sequence SSYGQQSEK (SEQ ID NO: 1).

5. The method according to any one of claims 1 to 4, wherein the sample is a blood, bone marrow, tumor, or tissue sample.

6. The method according to any one of claims 1 to 5, wherein the presence of an *EWSR1-WT1* fusion gene is detected in DNA or RNA from the sample.

7. The method according to claim 6, wherein the presence of an *EWSR1-WT1* fusion gene is detected in cell-free DNA (cfDNA).

8. The method according to claim 6 or claim 7, wherein the presence of an *EWSRI-WT1* fusion gene is detected in DNA by next-generation sequencing (NGS).

9. The method according to claim 6, wherein the presence of an *EWSRI-WT1* fusion gene is detected in RNA by RNA-sequencing (RNA-seq).

10. The method according to any one of claims 1 to 9, wherein the neoantigen binds, or is predicted to bind, an MHC class I molecule of the individual with a dissociation constant (KD) of 50 nM or less.

11. The method according to any one of claims 1 to 10, further comprising: genotyping one or more human leukocyte antigen (HLA)-A, HLA-B, and HLA-C alleles from the sample from the individual.

12. The method according to any one of claims 1 to 11, wherein the cancer does not comprise a loss-of-heterozygosity at an HLA-A, HLA-B, or HLA-C allele that encodes an MHC class I molecule to which the neoantigen binds, or is predicted to bind.

13. The method according to any one of claims 1 to 12, wherein the cancer comprises a loss-of-heterozygosity at one or more, but not all, of HLA-A, HLA-B, or HLA-C allele(s) that encode an MHC class I molecule to which the neoantigen binds, or is predicted to bind.

14. The method according to any one of claims 1 to 13, wherein the cancer immunotherapy comprises a checkpoint inhibitor.
